# EUROPÄISCHE PATENTSCHRIFT

(11) **EP 3 484 877 B1**
(45) Veröffentlichungstag und Bekanntmachung des Hinweises auf die Patenterteilung: **01.07.2020**
(21) Anmeldenummer: 17751252.2
(22) Anmeldetag: 10.07.2017
(51) Int. Cl.: C07D 403/14, A01N 43/647, A01N 43/707

(54) **BICYCLISCHE VERBINDUNGEN ALS SCHÄDLINGSBEKÄMPFUNGSMITTEL**
BICYCLIC COMPOUNDS AS PEST CONTROLLERS
COMPOSES BICYCLIQUES COMME PESTICIDES

(30) Priorität: 12.07.2016 EP 16179030
(43) Veröffentlichungstag der Anmeldung: 22.05.2019
(73) Patentinhaber: Bayer CropScience Aktiengesellschaft, 40789 Monheim am Rhein (DE)
(72) Erfinder: JESCHKE, Peter, 51467 Bergisch Gladbach (DE); CEREZO-GALVEZ, Silvia, 40764 Langenfeld (DE); FÜSSLEIN, Martin, 40225 Düsseldorf (DE); ILG, Kerstin, 50670 Köln (DE); PORTZ, Daniela, 52391 Vettweiß (DE); LÖSEL, Peter, 51371 Leverkusen (DE); BIERER, Donald, 42781 Haan (DE); WEBSTER, Robert Alan, 69005 Lyon (FR)
(74) Vertreter: BIP Patents
(86) Internationale Anmeldenummer: PCT/EP2017/067202
(87) Internationale Veröffentlichungsnummer: WO 2018/011111

(56) Entgegenhaltungen:
- WO-A1-2016/087363
- WO-A1-2016/087368
- WO-A1-2016/087421
- WO-A1-2016/087422

## Beschreibung

Die vorliegende Anmeldung betrifft neue bicyclische Verbindungen, Mittel enthaltend diese Verbindungen, ihre Verwendung zur Bekämpfung von tierischen Schädlingen sowie Verfahren und Zwischenprodukte zu Ihrer Herstellung.

Es sind bereits bicyclische Verbindungen bekannt geworden, die insektizide Eigenschaften besitzen (WO 2015/038503 A1, WO 2016/087363 A1, WO 2016/087368 A1, WO 2016/087421 A1, WO 2016/087422 A1).

Moderne Pflanzenschutzmittel müssen vielen Anforderungen genügen, beispielsweise in Bezug auf Höhe, Dauer und Breite ihrer Wirkung und möglichen Verwendung. Es spielen Fragen der Toxizität, der Kombinierbarkeit mit anderen Wirkstoffen oder Formulierhilfsmitteln eine Rolle sowie die Frage des Aufwands, der für die Synthese eines Wirkstoffs betrieben werden muss. Ferner können Resistenzen auftreten. Schon aus all diesen Gründen kann die Suche nach neuen Pflanzenschutzmitteln nicht als abgeschlossen betrachtet werden und es besteht ständig Bedarf an neuen Verbindungen mit gegenüber den bekannten Verbindungen zumindest in Bezug auf einzelne Aspekte verbesserten Eigenschaften.

Aufgabe der vorliegenden Erfindung war es, Verbindungen bereitzustellen, durch die das Spektrum der Schädlingsbekämpfungsmittel unter verschiedenen Aspekten verbreitert wird.

Gelöst wird die Aufgabe, sowie weitere nicht explizit genannte Aufgaben, die aus den hierin diskutierten Zusammenhängen ableitbar oder erschließbar sind, durch Verbindungen der Formel (I) 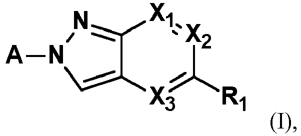 in welcher
- A: für einen A-Rest aus der Reihe (A-b) bis (A-f) 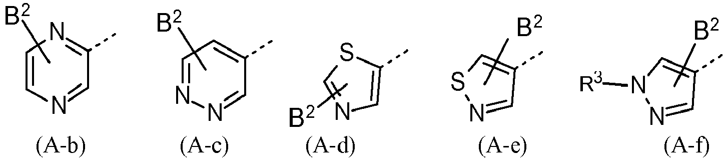 steht, worin die gestrichelte Linie die Bindung zum Kohlenstoffatom des Bicyclus der Formel (I) bedeutet und
- B²: für einen Rest aus der Reihe Wasserstoff, Halogen, Cyano, Nitro, Alkyl, Halogenalkyl, Alkoxy, Halogenalkoxy und jeweils gegebenenfalls substituiertes Cycloalkyl und Cycloalkenyl steht,
- X₁: für Stickstoff (N) oder CH steht,
- X₂: für Stickstoff oder CH steht,
- X₃: für Stickstoff oder CH steht, wobei entweder keine oder nur eine der Variablen X₁, X₂ und X₃ für Stickstoff steht,
- R₁: für einen B-Rest aus der Reihe 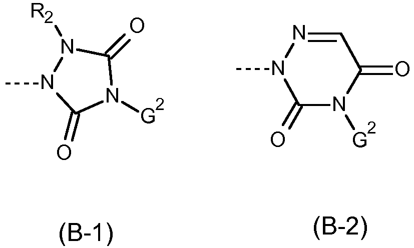 steht, worin die gestrichelte Linie die Bindung zum Kohlenstoffatom des Bicyclus der Formel (I) bedeutet,
- R₂: für einen Rest aus der Reihe Wasserstoff, Alkyl, Halogenalkyl, Alkenyl, Alkinyl, Alkoxyalkyl, Alkyl-S-alkyl, Alkyl-S(O)-alkyl, Alkyl-S(O)₂-alkyl, Cycloalkyl, Halogencycloalkyl und Cycloalkenyl steht,
- G²: für einen Rest aus der Reihe Wasserstoff, Alkyl, Halogenalkyl, Alkenyl, Alkinyl, Alkoxyalkyl, Alkyl-S-alkyl, Alkyl-S(O)-alkyl, Alkyl-S(O)₂-alkyl, Cycloalkyl, Halogencycloalkyl, Cycloalkenyl, Halogencycloalkenyl, Cycloalkylalkyl, Cycloalkenylalkyl, Halogencycloalkenylalkyl und jeweils gegebenenfalls substituiertes Aryl, Arylalkyl, Pyridyl und Pyridylalkyl steht, und Verbindungen der Formel (I), in welcher
- A: für den Rest (A-a) 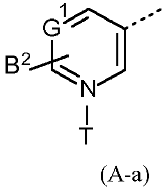 steht, worin die gestrichelte Linie die Bindung zum Kohlenstoffatom des Bicyclus der Formel (I) bedeutet und
- G¹: für N oder C-B¹ steht,
- B¹: für einen Rest aus der Reihe Wasserstoff, Halogen, Cyano, Nitro, Alkyl, Halogenalkyl, Alkoxy, Halogenalkoxy und jeweils gegebenenfalls substituiertes Cycloalkyl und Cycloalkenyl steht,
- B²: für einen Rest aus der Reihe Wasserstoff, Halogen, Cyano, Nitro, Alkyl, Halogenalkyl, Alkoxy, Halogenalkoxy und jeweils gegebenenfalls substituiertes Cycloalkyl und Cycloalkenyl steht,
- T: für Sauerstoff oder ein Elektronenpaar steht,
- X₁: für Stickstoff oder CH steht,
- X₂: für Stickstoff oder CH steht,
- X₃: für Stickstoff oder CH steht, wobei entweder keine oder nur eine der Variablen X₁, X₂ und X₃ für Stickstoff steht,
- R₁: für einen der folgenden B-Reste 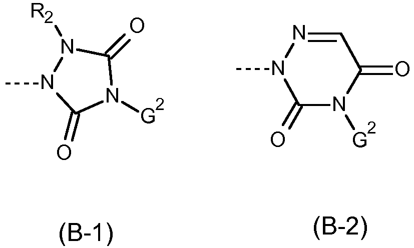 steht, worin die gestrichelte Linie die Bindung zum Kohlenstoffatom des Bicyclus der Formel (I) bedeutet, worin
- R₂: für einen Rest aus der Reihe Wasserstoff, Alkyl, Halogenalkyl, Alkenyl, Alkinyl, Alkoxyalkyl, Alkyl-S-alkyl, Alkyl-S(O)-alkyl, Alkyl-S(O)₂-alkyl, Cycloalkyl, Halogencycloalkyl und Cycloalkenyl steht,
- G²: für einen Rest aus der Reihe Wasserstoff, Alkyl, Halogenalkyl, Alkenyl, Alkinyl, Alkoxyalkyl, Alkyl-S-alkyl, Alkyl-S(O)-alkyl, Alkyl-S(O)₂-alkyl, Cycloalkyl, Halogencycloalkyl, Cycloalkenyl, Halogencycloalkenyl, Cycloalkylalkyl, Cycloalkenylalkyl, Halogencycloalkenylalkyl und jeweils gegebenenfalls substituiertes Aryl, Arylalkyl, Pyridyl und Pyridylalkyl steht.

Weiterhin wurde gefunden, dass die Verbindungen der Formel (I) eine gute Wirksamkeit als Schädlingsbekämpfungsmittel, beispielsweise gegen Arthropoden und insbesondere Insekten, besitzen und darüber hinaus in der Regel insbesondere gegenüber Kulturpflanzen sehr gut pflanzenverträglich sind und/oder über günstige toxikologische und/oder günstige umweltrelevante Eigenschaften verfügen.

Vorzugsbereich (1): Bevorzugt sind Verbindungen der Formel (I), in welchen
- A: für einen der folgenden A-Reste 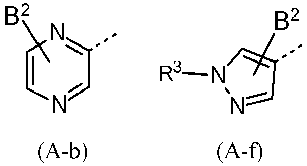 steht, worin die gestrichelte Linie die Bindung zum Kohlenstoffatom des Bicyclus der Formel (I) bedeutet, und
- B²: für einen Rest aus der Reihe Wasserstoff, Halogen, C₁-C₆-Alkyl und C₁-C₄-Halogenalkyl steht,
- X₁: für CH steht,
- X₂: für CH steht,
- X₃: für CH steht,
- R₁: für einen der folgenden B-Reste 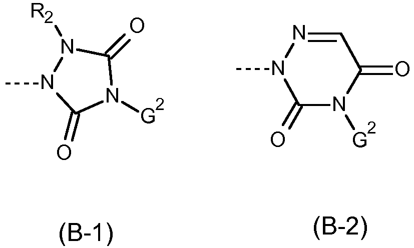 steht, worin die gestrichelte Linie die Bindung zum Kohlenstoffatom des Bicyclus der Formel (I) bedeutet, worin
- R₂: für Wasserstoff oder C₁-C₆-Alkyl steht,
- G²: für einen Rest aus der Reihe Wasserstoff, C₁-C₆-Alkyl, Halogen-C₁-C₆-alkyl, C₃-C₆-Cycloalkyl, Halogen-C₃-C₆-cycloalkyl, C₃-C₆-Cycloalkenyl, Halogen-C₃-C₆-cycloalkenyl, Phenyl oder Benzyl (wobei Phenyl und Benzyl selbst wiederum substituiert sein können durch Halogen, Cyano, Nitro, C₁-C₆-Alkyl, Halogen-C₁-C₆-alkyl und C₃-C₆-Cycloalkyl), Pyridyl oder Pyridylmethyl (wobei Pyridyl und Pyridylmethyl selbst wiederum substituiert sein können durch Halogen, Cyano, Nitro, C₁-C₆-Alkyl, Halogen-C₁-C₆-alkyl und C₃-C₆-Cycloalkyl), steht, und
Verbindungen der Formel (I), in welchen
- A: für den folgenden A-Rest 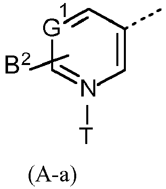 steht, worin die gestrichelte Linie die Bindung zum Kohlenstoffatom des Bicyclus der Formel (I) bedeutet, und
- G¹: für N oder C-B¹ steht,
- B¹: für einen Rest aus der Reihe Wasserstoff, Halogen, C₁-C₆-Alkyl und C₁-C₄-Halogenalkyl steht,
- B²: für einen Rest aus der Reihe Wasserstoff, Halogen, C₁-C₆-Alkyl und C₁-C₄-Halogenalkyl steht,
- T: für Sauerstoff oder ein Elektronenpaar steht,
- X₁: für CH steht,
- X₂: für CH steht,
- X₃: für CH steht,
- R₁: für einen der folgenden B-Reste 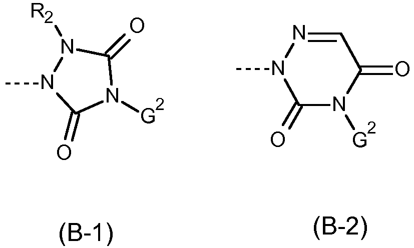 steht, worin die gestrichelte Linie die Bindung zum Kohlenstoffatom des Bicyclus der Formel (I) bedeutet, worin
- R₂: für Wasserstoff oder C₁-C₆-Alkyl steht,
- G²: für einen Rest aus der Reihe Wasserstoff, C₁-C₆-Alkyl, Halogen-C₁-C₆-alkyl, C₃-C₆-Cycloalkyl, Halogen-C₃-C₆-cycloalkyl, C₃-C₆-Cycloalkenyl, Halogen-C₃-C₆-cycloalkenyl, Phenyl oder Benzyl (wobei Phenyl und Benzyl selbst wiederum substituiert sein können durch Halogen, Cyano, Nitro, C₁-C₆-Alkyl, Halogen-C₁-C₆-alkyl und C₃-C₆-Cycloalkyl), Pyridyl oder Pyridylmethyl (wobei Pyridyl und Pyridylmethyl selbst wiederum substituiert sein können durch Halogen, Cyano, Nitro, C₁-C₆-Alkyl, Halogen-C₁-C₆-alkyl und C₃-C₆-Cycloalkyl), steht.

Vorzugsbereich (2): Besonders bevorzugt sind Verbindungen der Formel (I), worin
- A: für einen A-Rest aus der Reihe (A-b) und (A-f) 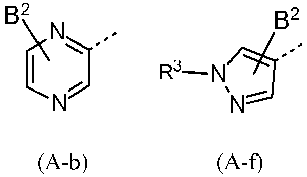 steht, worin die gestrichelte Linie die Bindung zum Kohlenstoffatom des Bicyclus der Formel (I) bedeutet,
- B²: für einen Rest aus der Reihe Wasserstoff, Halogen, C₁-C₆-Alkyl und C₁-C₄-Halogenalkyl steht,
- X₁: für CH steht,
- X₂: für CH steht,
- X₃: für CH steht,
- R₁: für einen B-Rest aus der Reihe 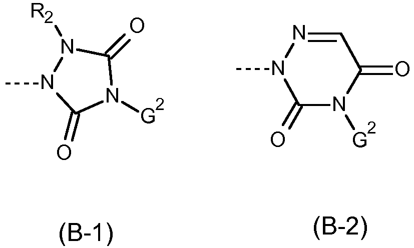 steht, worin die gestrichelte Linie die Bindung zum Kohlenstoffatom des Bicyclus der Formel (I) bedeutet, worin
- R₂: für Wasserstoff oder C₁-C₆-Alkyl steht,
- G²: für einen Rest aus der Reihe Wasserstoff, C₁-C₆-Alkyl, Halogen-C₁-C₆-alkyl, C₃-C₆-Cycloalkyl, Halogen-C₃-C₆-cycloalkyl, C₃-C₆-Cycloalkenyl und Halogen-C₃-C₆-cycloalkenyl steht, und Verbindungen der Formel (I), worin
- A: für den A-Rest 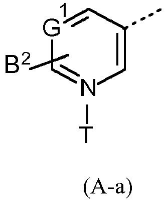 steht, worin die gestrichelte Linie die Bindung zum Kohlenstoffatom des Bicyclus der Formel (I) bedeutet,
- G¹: für N oder C-B¹ steht,
- B¹: für einen Rest aus der Reihe Wasserstoff, Halogen, C₁-C₆-Alkyl und C₁-C₄-Halogenalkyl steht,
- B²: für einen Rest aus der Reihe Wasserstoff, Halogen, C₁-C₆-Alkyl und C₁-C₄-Halogenalkyl steht,
- T: für Sauerstoff oder ein Elektronenpaar steht,
- X₁: für CH steht,
- X₂: für CH steht,
- X₃: für CH steht,
- R₁: für einen B-Rest aus der Reihe 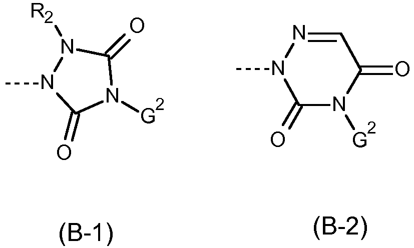 steht, worin die gestrichelte Linie die Bindung zum Kohlenstoffatom des Bicyclus der Formel (I) bedeutet, worin
- R₂: für Wasserstoff oder C₁-C₆-Alkyl steht,
- G²: für einen Rest aus der Reihe Wasserstoff, C₁-C₆-Alkyl, Halogen-C₁-C₆-alkyl, C₃-C₆-Cycloalkyl, Halogen-C₃-C₆-cycloalkyl, C₃-C₆-Cycloalkenyl und Halogen-C₃-C₆-cycloalkenyl steht.

Vorzugsbereich (3): Ganz besonders bevorzugt sind Verbindungen der Formel (I), in welchen
- A: für einen der folgenden A-Reste 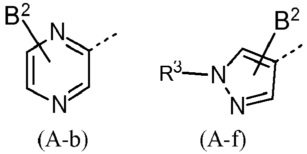 steht, worin die gestrichelte Linie die Bindung zum Kohlenstoffatom des Bicyclus der Formel (I) bedeutet,
- B²: für Wasserstoff steht,
- X₁: für CH steht,
- X₂: für CH steht,
- X₃: für CH steht,
- R₁: für einen B-Rest aus der Reihe 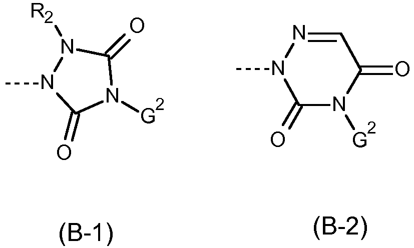 steht, worin die gestrichelte Linie die Bindung zum Kohlenstoffatom des Bicyclus der Formel (I) bedeutet, worin
- R₂: für Wasserstoff oder C₁-C₆-Alkyl steht,
- G²: für einen Rest aus der Reihe Wasserstoff, C₁-C₆-Alkyl, Halogen-C₁-C₆-alkyl und C₃-C₆-Cycloalkyl steht, und Verbindungen der Formel (I), in welchen
- A: für den A-Rest 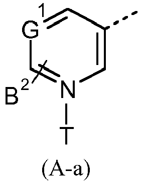 steht, worin die gestrichelte Linie die Bindung zum Kohlenstoffatom des Bicyclus der Formel (I) bedeutet,
- G¹: für N oder C-B¹ steht,
- B¹: für einen Rest aus der Reihe Wasserstoff und Fluor steht,
- B²: für Wasserstoff steht,
- T: für Sauerstoff oder ein Elektronenpaar steht,
- X₁: für CH steht,
- X₂: für CH steht,
- X₃: für CH steht,
- R₁: für einen B-Rest aus der Reihe 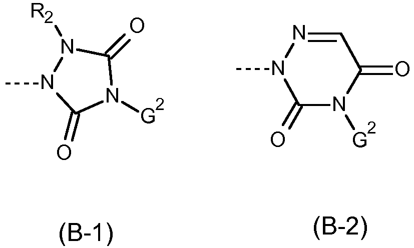 steht, worin die gestrichelte Linie die Bindung zum Kohlenstoffatom des Bicyclus der Formel (I) bedeutet, worin
- R₂: für Wasserstoff oder C₁-C₆-Alkyl steht,
- G²: für einen Rest aus der Reihe Wasserstoff, C₁-C₆-Alkyl, Halogen-C₁-C₆-alkyl und C₃-C₆-Cycloalkyl steht.

Vorzugsbereich (4): Eine hevorgehobene Gruppe von Verbindungen der Formel (I) sind solche, in welchen
- A: für 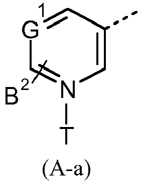 steht, worin die gestrichelte Linie die Bindung zum Kohlenstoffatom des Bicyclus der Formel (I) bedeutet,
- G¹: für C-B¹ steht,
- B¹: für Wasserstoff oder Fluor steht,
- B²: für Wasserstoff steht,
- T: für ein Elektronenpaar steht,
- X₁: für CH steht,
- X₂: für CH steht,
- X₃: für CH steht,
- R₁: für den Rest (B-1) 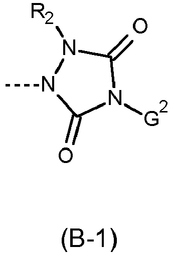 steht, worin die gestrichelte Linie die Bindung zum Kohlenstoffatom des Bicyclus der Formel (I) bedeutet, worin
- R₂: für Wasserstoff oder C₁-C₆-Alkyl steht und
- G²: für einen Rest aus der Reihe Wasserstoff, C₁-C₆-Alkyl, Halogen-C₁-C₆-alkyl und C₃-C₆-Cycloalkyl steht.

Vorzugsbereich (5): Eine weitere hervorgehobene Gruppe von Verbindungen der Formel (I) sind solche, in welchen
- A: für den A-Rest 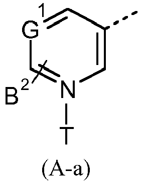 steht, worin die gestrichelte Linie die Bindung zum Kohlenstoffatom des Bicyclus der Formel (I) bedeutet,
- G¹: für N oder C-B¹ steht,
- B¹: für Wasserstoff oder Fluor steht,
- B²: für Wasserstoff steht,
- T: für ein Elektronenpaar steht,
- X₁: für CH steht,
- X₂: für CH steht,
- X₃: für CH steht,
- R₁: für den Rest (B-2) 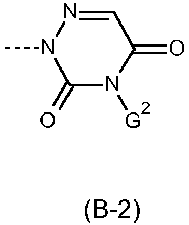 steht, worin die gestrichelte Linie die Bindung zum Kohlenstoffatom des Bicyclus der Formel (I) bedeutet, worin
- G²: für einen Rest aus der Reihe Wasserstoff, C₁-C₆-Alkyl, Halogen-C₁-C₆-alkyl und C₃-C₆-Cycloalkyl steht.

Vorzugsbereich (1a): Bevorzugt sind auch Verbindungen der Formel (I), in welchen
- A: für einen der folgenden A-Reste 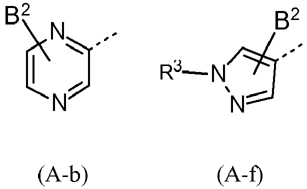 steht, worin die gestrichelte Linie die Bindung zum Kohlenstoffatom des Bicyclus der Formel (I) bedeutet, und
- B²: für einen Rest aus der Reihe Wasserstoff, Halogen, C₁-C₆-Alkyl und C₁-C₄-Halogenalkyl steht,
- X₁: für CH steht,
- X₂: für CH steht,
- X₃: für CH steht,
- R₁: für einen der folgenden B-Reste 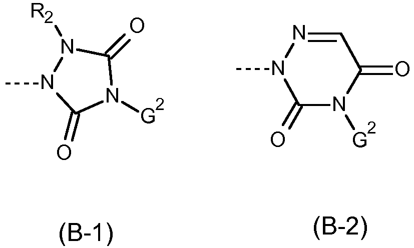 steht, worin die gestrichelte Linie die Bindung zum Kohlenstoffatom des Bicyclus der Formel (I) bedeutet, worin
- R₂: für Wasserstoff oder C₁-C₆-Alkyl steht,
- G²: für einen Rest aus der Reihe Wasserstoff, C₁-C₆-Alkyl, Halogen-C₁-C₆-alkyl, C₃-C₆-Cycloalkyl, Halogen-C₃-C₆-cycloalkyl, C₃-C₆-Cycloalkenyl, Halogen-C₃-C₆-cycloalkenyl, Phenyl oder Benzyl (wobei Phenyl und Benzyl selbst wiederum substituiert sein können durch Halogen, Cyano, Nitro, C₁-C₆-Alkyl, Halogen-C₁-C₆-alkyl und C₃-C₆-Cycloalkyl), Pyridyl oder Pyridylmethyl (wobei Pyridyl und Pyridylmethyl selbst wiederum substituiert sein können durch Halogen, Cyano, Nitro, C₁-C₆-Alkyl, Halogen-C₁-C₆-alkyl und C₃-C₆-Cycloalkyl), steht, und

Verbindungen der Formel (I), in welchen
- A: für den folgenden A-Rest 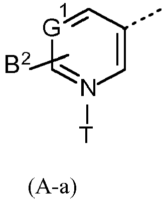 steht, worin die gestrichelte Linie die Bindung zum Kohlenstoffatom des Bicyclus der Formel (I) bedeutet, und
- G¹: für N oder C-B¹ steht,
- B¹: für einen Rest aus der Reihe Wasserstoff, Halogen, C₁-C₆-Alkyl und C₁-C₄-Halogenalkyl steht,
- B²: für einen Rest aus der Reihe Wasserstoff, Halogen, C₁-C₆-Alkyl und C₁-C₄-Halogenalkyl steht,
- T: für Sauerstoff oder ein Elektronenpaar steht,
- X₁: für N oder CH steht,
- X₂: für CH steht,
- X₃: für CH steht,
- R₁: für einen der folgenden B-Reste 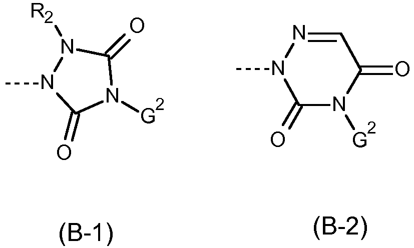 steht, worin die gestrichelte Linie die Bindung zum Kohlenstoffatom des Bicyclus der Formel (I) bedeutet, worin
- R₂: für Wasserstoff, C₁-C₆-Alkyl oder Halogen-C₁-C₆-alkyl steht, und
- G²: für einen Rest aus der Reihe Wasserstoff, C₁-C₆-Alkyl, Halogen-C₁-C₆-alkyl, C₁-C₆-Alkoxy-C₁-C₆-alkyl, C₁-C₆-Alkyl-S-C₁-C₆-alkyl, C₁-C₆-Alkyl-S(O)-C₁-C₆-alkyl, C₁-C₆-Alkyl-S(O₂)-C₁-C₆-alkyl, C₃-C₆-Cycloalkyl, C₃-C₆-Cycloalkyl-C₁-C₆-alkyl, Halogen-C₃-C₆-cycloalkyl, C₃-C₆-Cycloalkenyl, Halogen-C₃-C₆-cycloalkenyl, Phenyl oder Benzyl (wobei Phenyl und Benzyl selbst wiederum substituiert sein können durch Halogen, Cyano, Nitro, C₁-C₆-Alkyl, Halogen-C₁-C₆-alkyl und C₃-C₆-Cycloalkyl), Pyridyl oder Pyridylmethyl (wobei Pyridyl und Pyridylmethyl selbst wiederum substituiert sein können durch Halogen, Cyano, Nitro, C₁-C₆-Alkyl, Halogen-C₁-C₆-alkyl und C₃-C₆-Cycloalkyl), steht.

Vorzugsbereich (2a): Besonders bevorzugt sind auch Verbindungen der Formel (I), worin
- A: für einen A-Rest aus der Reihe (A-b) und (A-f) 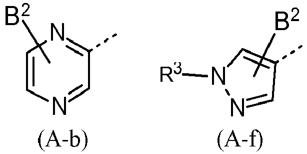 steht, worin die gestrichelte Linie die Bindung zum Kohlenstoffatom des Bicyclus der Formel (I) bedeutet,
- B²: für einen Rest aus der Reihe Wasserstoff, Halogen, C₁-C₆-Alkyl und C₁-C₄-Halogenalkyl steht,
- X₁: für CH steht,
- X₂: für CH steht,
- X₃: für CH steht,
- R₁: für einen B-Rest aus der Reihe 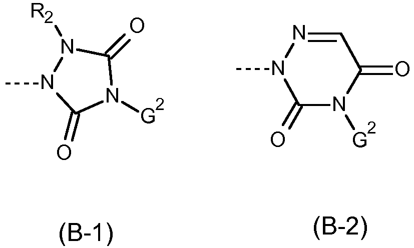 steht, worin die gestrichelte Linie die Bindung zum Kohlenstoffatom des Bicyclus der Formel (I) bedeutet, worin
- R₂: für Wasserstoff oder C₁-C₆-Alkyl steht,
- G²: für einen Rest aus der Reihe Wasserstoff, C₁-C₆-Alkyl, Halogen-C₁-C₆-alkyl, C₃-C₆-Cycloalkyl, Halogen-C₃-C₆-cycloalkyl, C₃-C₆-Cycloalkenyl und Halogen-C₃-C₆-cycloalkenyl steht, und

Verbindungen der Formel (I), worin
- A: für den A-Rest 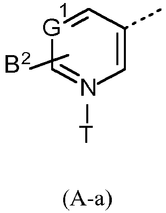 steht, worin die gestrichelte Linie die Bindung zum Kohlenstoffatom des Bicyclus der Formel (I) bedeutet,
- G¹: für N oder C-B¹ steht,
- B¹: für einen Rest aus der Reihe Wasserstoff, Halogen, C₁-C₆-Alkyl und C₁-C₄-Halogenalkyl steht,
- B²: für einen Rest aus der Reihe Wasserstoff, Halogen, C₁-C₆-Alkyl und C₁-C₄-Halogenalkyl steht,
- T: für Sauerstoff oder ein Elektronenpaar steht,
- X₁: für N oder CH steht,
- X₂: für CH steht,
- X₃: für CH steht,
- R₁: für einen B-Rest aus der Reihe 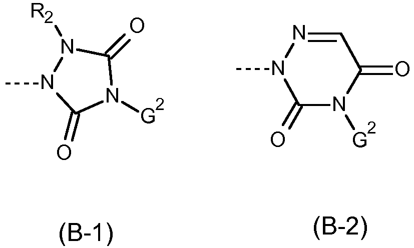 steht, worin die gestrichelte Linie die Bindung zum Kohlenstoffatom des Bicyclus der Formel (I) bedeutet, worin
- R₂: für Wasserstoff, C₁-C₆-Alkyl oder Halogen-C₁-C₆-alkyl steht,
- G²: für einen Rest aus der Reihe Wasserstoff, C₁-C₆-Alkyl, Halogen-C₁-C₆-alkyl, C₁-C₆-Alkoxy-C₁-C₆-alkyl, C₁-C₆-Alkyl-S-C₁-C₆-alkyl, C₁-C₆-Alkyl-S(O)-C₁-C₆-alkyl, C₁-C₆-Alkyl-S(O₂)-C₁-C₆-alkyl, C₃-C₆-Cycloalkyl, C₃-C₆-Cycloalkyl-C₁-C₆-alkyl, Phenyl oder Benzyl (wobei Phenyl und Benzyl selbst wiederum substituiert sein können durch Halogen, Cyano, Nitro, C₁-C₆-Alkyl, Halogen-C₁-C₆-alkyl und C₃-C₆-Cycloalkyl), Pyridylmethyl, welches durch Halogen substituiert sein kann, Halogen-C₃-C₆-cycloalkyl, C₃-C₆-Cycloalkenyl und Halogen-C₃-C₆-cycloalkenyl steht.

Vorzugsbereich (3a): Ganz besonders bevorzugt sind auch Verbindungen der Formel (I), in welchen
- A: für einen der folgenden A-Reste 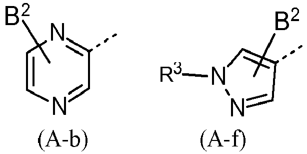 steht, worin die gestrichelte Linie die Bindung zum Kohlenstoffatom des Bicyclus der Formel (I) bedeutet,
- B²: für Wasserstoff steht,
- X₁: für CH steht,
- X₂: für CH steht,
- X₃: für CH steht,
- R₁: für einen B-Rest aus der Reihe 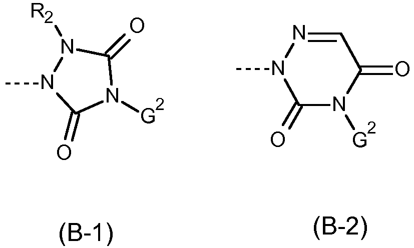 steht, worin die gestrichelte Linie die Bindung zum Kohlenstoffatom des Bicyclus der Formel (I) bedeutet, worin
- R₂: für Wasserstoff oder C₁-C₆-Alkyl steht,
- G²: für einen Rest aus der Reihe Wasserstoff, C₁-C₆-Alkyl, Halogen-C₁-C₆-alkyl und C₃-C₆-Cycloalkyl steht, und

Verbindungen der Formel (I), in welchen
- A: für den A-Rest 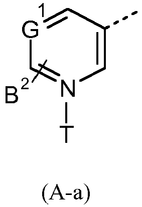 steht, worin die gestrichelte Linie die Bindung zum Kohlenstoffatom des Bicyclus der Formel (I) bedeutet,
- G¹: für N oder C-B¹ steht,
- B¹: für einen Rest aus der Reihe Wasserstoff und Fluor steht,
- B²: für Wasserstoff steht,
- T: für Sauerstoff oder ein Elektronenpaar steht,
- X₁: für N oder CH steht,
- X₂: für CH steht,
- X₃: für CH steht,
- R₁: für einen B-Rest aus der Reihe 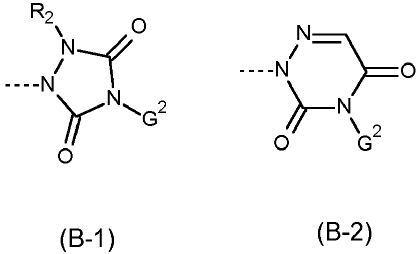 steht, worin die gestrichelte Linie die Bindung zum Kohlenstoffatom des Bicyclus der Formel (I) bedeutet, worin
- R₂: für Wasserstoff, C₁-C₆-Alkyl oder Halogen-C₁-C₆-alkyl steht,
- G²: für einen Rest aus der Reihe C₁-C₄-Alkyl, Halogen-C₁-C₄-alkyl, C₁-C₄-Alkoxy-C₁-C₄-alkyl, C₁-C₄-Alkyl-S-C₁-C₄-alkyl, C₁-C₄-Alkyl-S(O)-C₁-C₄-alkyl, C₁-C₄-Alkyl-S(O₂)-C₁-C₄-alkyl,C₃-C₆-Cycloalkyl, C₃-C₆-Cycloalkyl-C₁-C₄-alkyl, Phenyl oder Benzyl (wobei Phenyl und Benzyl selbst wiederum substituiert sein können durch Halogen und Cyano), Pyridylmethyl, welches durch Halogen substituiert sein kann, steht.

Vorzugsbereich (4a): Eine hevorgehobene Gruppe von Verbindungen der Formel (I) sind solche, in welchen
- A: für 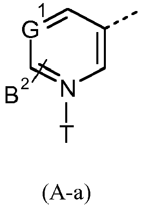 steht, worin die gestrichelte Linie die Bindung zum Kohlenstoffatom des Bicyclus der Formel (I) bedeutet,
- G¹: für C-B¹ steht,
- B¹: für Wasserstoff steht,
- B²: für Wasserstoff steht,
- T: für ein Elektronenpaar steht,
- X₁: für N oder CH steht,
- X₂: für CH steht,
- X₃: für CH steht,
- R₁: für den Rest (B-1) 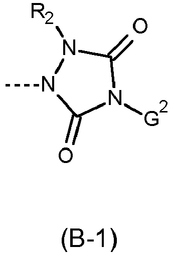 steht, worin die gestrichelte Linie die Bindung zum Kohlenstoffatom des Bicyclus der Formel (I) bedeutet, worin
- R₂: für Wasserstoff, C₁-C₄-Alkyl oder Halogen-C₁-C₄-alkyl steht und
- G²: für einen Rest aus der Reihe C₁-C₄-Alkyl, Halogen-C₁-C₄-alkyl, C₁-C₄-Alkoxy-C₁-C₄-alkyl, C₁-C₄-Alkyl-S-C₁-C₄-alkyl, C₁-C₄-Alkyl-S(O)-C₁-C₄-alkyl, C₃-C₆-Cycloalkyl, C₃-C₆-Cycloalkyl-C₁-C₄-alkyl, Phenyl, Benzyl und Pyridylmethyl (wobei Phenyl, Benzyl und Pyridylmethyl selbst wiederum durch Halogen substituiert sein können) steht.

Vorzugsbereich (5a): Eine weitere hervorgehobene Gruppe von Verbindungen der Formel (I) sind solche, in welchen
- A: für den A-Rest 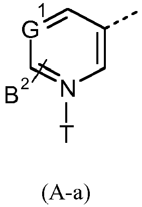 steht, worin die gestrichelte Linie die Bindung zum Kohlenstoffatom des Bicyclus der Formel (I) bedeutet,
- G¹: für C-B¹ steht,
- B¹: für Wasserstoff steht,
- B²: für Wasserstoff steht,
- T: für ein Elektronenpaar steht,
- X₁: für N oder CH steht,
- X₂: für CH steht,
- X₃: für CH steht,
- R₁: für den Rest (B-2) 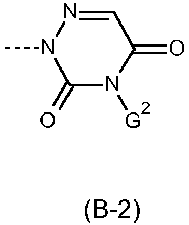
steht, worin die gestrichelte Linie die Bindung zum Kohlenstoffatom des Bicyclus der Formel (I) bedeutet, worin
- G²: für einen Rest aus der Reihe C₁-C₄-Alkyl, Halogen-C₁-C₄-alkyl, C₃-C₆-Cycloalkyl-C₁-C₄-alkyl, Phenyl oder Benzyl (wobei Phenyl und Benzyl selbst wiederum durch Halogen substituiert sein können) steht.

In den oben aufgeführten Definitionen, ist, sofern nichts anderes angegeben ist, Halogen ausgewählt aus der Reihe Fluor, Chlor, Brom und Iod, bevorzugt wiederum aus der Reihe Fluor, Chlor und Brom.

Durch Halogen substituierte Reste, z.B. Halogenalkyl (= Haloalkyl), sind einfach oder mehrfach bis zur maximal möglichen Substituentenzahl halogeniert. Bei mehrfacher Halogenierung können die Halogenatome gleich oder verschieden sein. Halogen steht dabei für Fluor, Chlor, Brom oder Iod, insbesondere für Fluor, Chlor oder Brom.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können, wenn nichts anderes erwähnt ist, einfach oder mehrfach substituiert sein, wobei bei Mehrfachsubstitutionen die Substituenten gleich oder verschieden sein können.

Wenn T im Rest A der Formel (A-a) 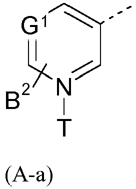 für ein Elektronenpaar steht, liegt der Rest als Pyridinderivat der Formel 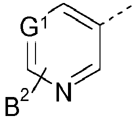 vor.

Wenn T im Rest A der Formel (A-a) 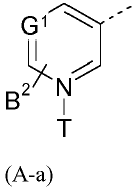 für Sauerstoff steht, liegt der Rest als Pyridin-*N*-Oxid-derivat der Formel 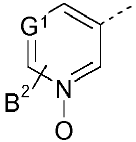 vor. Auf die Darstellung der Formalladungen (+ am Stickstoff und - am Sauerstoff) wurde hier verzichtet.

Die oben aufgeführten allgemeinen oder in Vorzugsbereichen aufgeführten Restedefinitionen bzw. Erläuterungen gelten für die Endprodukte und für die Ausgangsprodukte und Zwischenprodukte entsprechend. Diese Restedefinitionen können untereinander, also auch zwischen den jeweiligen Vorzugsbereichen, beliebig kombiniert werden.

Erfindungsgemäß bevorzugt sind Verbindungen der Formel (I), in welchen eine Kombination der vorstehend als bevorzugt aufgeführten Bedeutungen vorliegt (Vorzugsbereich (1)).

Erfindungsgemäß besonders bevorzugt sind Verbindungen der Formel (I), in welchen eine Kombination der vorstehend als besonders bevorzugt aufgeführten Bedeutungen vorliegt (Vorzugsbereich (2)).

Erfindungsgemäß ganz besonders bevorzugt sind Verbindungen der Formel (I), in welchen eine Kombination der vorstehend als ganz besonders bevorzugt aufgeführten Bedeutungen vorliegt (Vorzugsbereich (3)).

Erfindungsgemäß insbesonders bevorzugt sind Verbindungen der Formel (I), in welchen eine Kombination der vorstehend als hervorgehoben aufgeführten Bedeutungen vorliegt (Vorzugsbereich (4)).

Erfindungsgemäß insbesonders bevorzugt sind auch Verbindungen der Formel (I), in welchen eine Kombination der vorstehend als hervorgehoben aufgeführten Bedeutungen vorliegt (Vorzugsbereich (5)).

Erfindungsgemäß bevorzugt sind auch Verbindungen der Formel (I), in welchen eine Kombination der vorstehend als bevorzugt aufgeführten Bedeutungen vorliegt (Vorzugsbereich (1a)).

Erfindungsgemäß besonders bevorzugt sind auch Verbindungen der Formel (I), in welchen eine Kombination der vorstehend als besonders bevorzugt aufgeführten Bedeutungen vorliegt (Vorzugsbereich (2a)).

Erfindungsgemäß ganz besonders bevorzugt sind auchVerbindungen der Formel (I), in welchen eine Kombination der vorstehend als ganz besonders bevorzugt aufgeführten Bedeutungen vorliegt (Vorzugsbereich (3a)).

Erfindungsgemäß insbesonders bevorzugt sind auch Verbindungen der Formel (I), in welchen eine Kombination der vorstehend als hervorgehoben aufgeführten Bedeutungen vorliegt (Vorzugsbereich (4a)).

Erfindungsgemäß insbesonders bevorzugt sind auch Verbindungen der Formel (I), in welchen eine Kombination der vorstehend als hervorgehoben aufgeführten Bedeutungen vorliegt (Vorzugsbereich (5a)).

Eine bevorzugte Ausführungsform der Erfindung betrifft Verbindungen der Formel (I), in welchen A für den Rest der Formel (A-a) 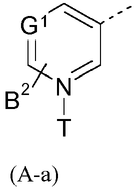 steht.

Eine weitere bevorzugte Ausführungsform der Erfindung betrifft Verbindungen der Formel (I), in welchen A für Pyridin-3-yl steht.

Eine weitere bevorzugte Ausführungsform der Erfindung betrifft Verbindungen der Formel (I), in welchen A für 5-Fluor-pyridin-3-yl steht.

Eine weitere bevorzugte Ausführungsform der Erfindung betrifft Verbindungen der Formel (I), in welchen A für Pyrimidin-5-yl steht.

Eine weitere bevorzugte Ausführungsform der Erfindung betrifft Verbindungen der Formel (I), in welchen A für Pyridazin-4-yl steht.

Die oben aufgeführten allgemeinen oder in Vorzugsbereichen aufgeführten Restedefinitionen bzw. Erläuterungen gelten für die Endprodukte (auch für die Verbindungen der später aufgeführten Formeln (I-A) bis (I-P)) und für die Ausgangsprodukte und Zwischenprodukte entsprechend. Diese Restedefinitionen können untereinander, also auch zwischen den jeweiligen Vorzugsbereichen, beliebig kombiniert werden.

In einer bevorzugten Ausführungsform betrifft die Erfindung Verbindungen der Formel (I-A)

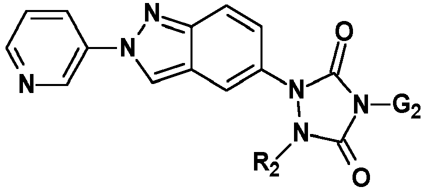

In einer weiteren bevorzugten Ausführungsform betrifft die Erfindung Verbindungen der Formel (I-B)

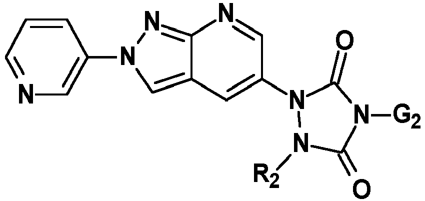

In einer weiteren bevorzugten Ausführungsform betrifft die Erfindung Verbindungen der Formel (I-C)

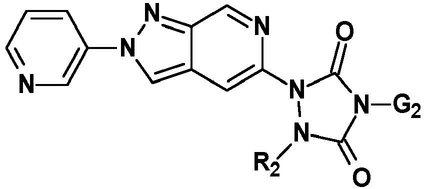

In einer weiteren bevorzugten Ausführungsform betrifft die Erfindung Verbindungen der Formel (I-D)

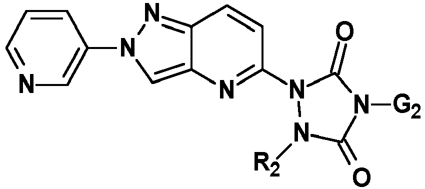

In einer weiteren bevorzugten Ausführungsform betrifft die Erfindung Verbindungen der Formel (I-E)

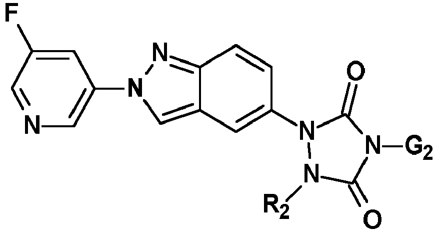

In einer weiteren bevorzugten Ausführungsform betrifft die Erfindung Verbindungen der Formel (I-F)

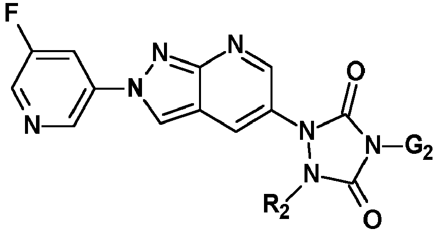

In einer weiteren bevorzugten Ausführungsform betrifft die Erfindung Verbindungen der Formel (I-G)

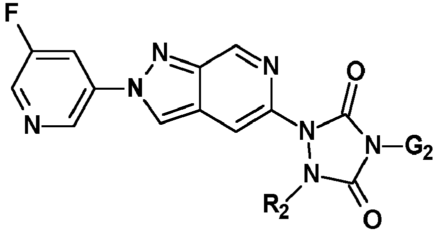

In einer weiteren bevorzugten Ausführungsform betrifft die Erfindung Verbindungen der Formel (I-H)

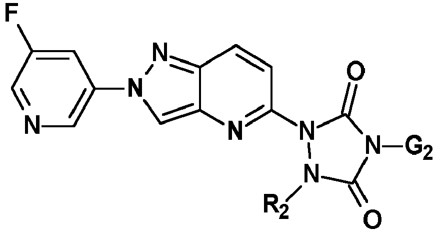

In einer weiteren bevorzugten Ausführungsform betrifft die Erfindung Verbindungen der Formel (I-I)

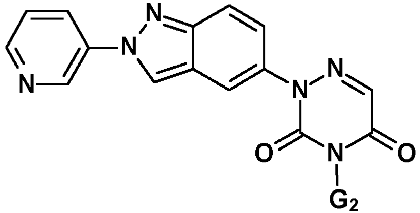

In einer weiteren bevorzugten Ausführungsform betrifft die Erfindung Verbindungen der Formel (I-J)

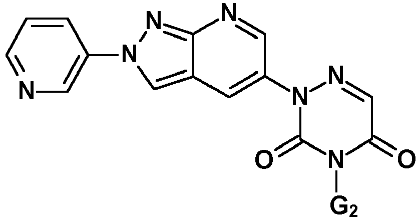

In einer weiteren bevorzugten Ausführungsform betrifft die Erfindung Verbindungen der Formel (I-K)

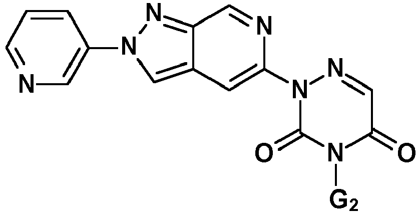

In einer weiteren bevorzugten Ausführungsform betrifft die Erfindung Verbindungen der Formel (I-L)

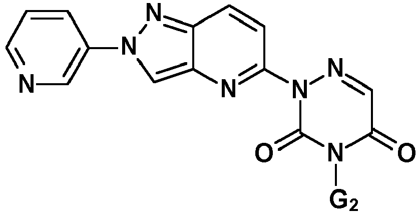

In einer weiteren bevorzugten Ausführungsform betrifft die Erfindung Verbindungen der Formel (I-M)

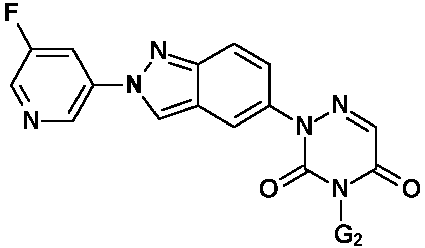

In einer weiteren bevorzugten Ausführungsform betrifft die Erfindung Verbindungen der Formel (I-N)

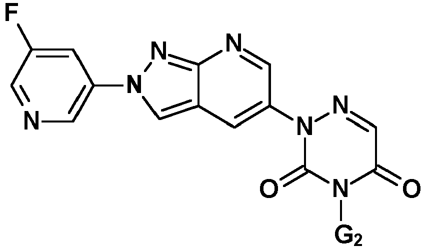

In einer weiteren bevorzugten Ausführungsform betrifft die Erfindung Verbindungen der Formel (I-O)

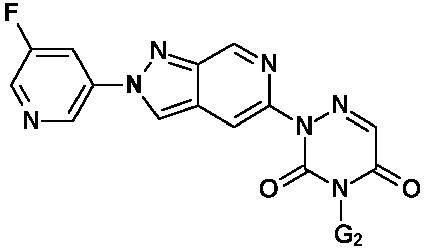

In einer weiteren bevorzugten Ausführungsform betrifft die Erfindung Verbindungen der Formel (I-P)

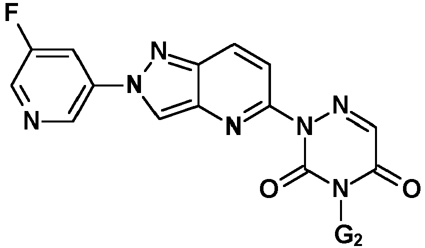

In den Formeln (I-A) bis (I-P) haben die Variablen die weiter oben, auch in den Vorzugsbereichen, genannten Bedeutungen.

Die Verbindungen der Formel (I) und deren Säureadditionssalze und Metallsalzkomplexe besitzen gute Wirksamkeit, insbesondere zur Bekämpfung von tierischen Schädlingen, zu denen Arthropoden und insbesondere Insekten zählen.

Als geeignete Salze der Verbindungen der Formel (I) können übliche nicht toxische Salze, d. h. Salze mit entsprechenden Basen und Salze mit zugesetzten Säuren genannt werden. Vorzugsweise sind Salze mit anorganischen Basen, wie Alkalimetallsalze, beispielsweise Natrium-, Kalium- oder Cäsiumsalze, Erdalkalimetallsalze, beispielsweise Calzium- oder Magnesiumsalze, Ammoniumsalze, Salze mit organischen Basen sowie mit anorganischen Aminen, beispielsweise Triethylammonium-, Dicyclohexylammonium-, N,N'-Dibenzylethylendiammonium-, Pyridinium-, Picolinium- oder Ethanolammoniumsalze, Salze mit anorganischen Säuren, beispielsweise Hydrochloride, Hydrobromide, Dihydrosulfate, Trihydrosulfate, oder Phosphate, Salze mit organischen Carbonsäuren oder organischen Sulfosäure, beispielsweise Formiate, Acetate, Trifluoracetate, Maleate, Tartrate, Methansulfonate, Benzolsulfonate oder para-Toluolsulfonate, Salze mit basischen Aminosäuren, beispielsweise Arginate, Aspartate oder Glutamate und Ähnliches zu nennen.

Die Verbindungen der Formel (I) können gegebenenfalls auch in Abhängigkeit von der Art der Substituenten als Stereoisomere, d.h. als geometrische und/oder als optische Isomere oder Isomerengemische vorliegen in unterschiedlichen Zusammensetzungen vorliegen. Sowohl die reinen Stereoisomeren als auch beliebige Gemische dieser Isomeren sind Gegenstand dieser Erfindung, auch wenn hier im Allgemeinen nur von Verbindungen der Formel (I) die Rede ist.

Die Erfindung betrifft daher sowohl die reinen Enantiomeren und Diastereomeren, als auch deren Gemische zur Bekämpfung von tierischen Schädlingen, zu denen Arthropoden und insbesondere Insekten zählen.

Vorzugsweise werden jedoch die optisch aktiven, stereoisomeren Formen der Verbindungen der Formel (I) und deren Salze erfindungsgemäß verwendet.

Im Folgenden werden die Begriffe "Synthesebeispiel" und "Anwendungsbeispiel" gleichbedeutend verwendet, sofern nichts anderes angegeben ist oder sich aus dem Zusammenhang nichts anderes ergibt.

Die Vorstufen (A-4) für die Verbindungen der Formel (I), in denen der Heterocyclus A für gegebenenfalls mit einem Rest B² substituiertes Pyrimidin-5-yl (A-a; G¹ = N), Pyridin-3-yl (A-a; G¹ = C-B¹), Pyrazin-2-yl (A-b), Pyridazin-3-yl (A-c), Thiazol-5-yl (A-d), Isothiazol-4-yl (A-e) und Pyrazol-4-yl (A-f) steht, X₁ für CH steht, X₂ und X₃ jeweils unabhängig von einander für Stickstoff oder CH stehen, können beispielsweise gemäss Reaktionsschema I in zwei Schritten hergestellt werden.

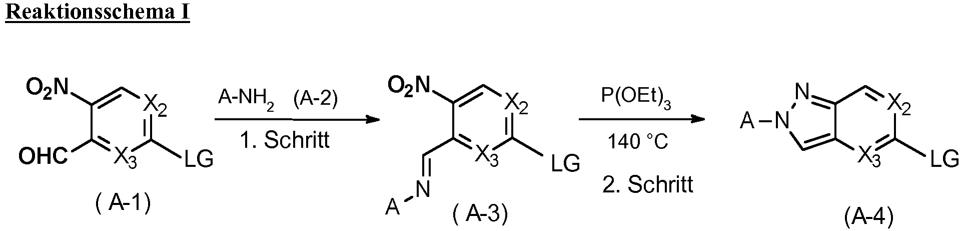

Im Reaktionsschema I haben A, X₂ und X₃ die weiter oben genannten Bedeutungen und LG steht für eine nucleofuge Abgangsgruppe (*"Leaving Group*"), bevorzugt für Halogen.

Beispielsweise können substituierte 2-Nitro-benzaldehyde der Formel (A-1; z. B. X₂, X₃ = CH) mit den entsprechenden 3-aminosubstituierten Heterocyclen der Formel (A-2) in Gegenwart von sauren Reaktionshilfsmitteln in einem ersten Reaktionsschritt zu Verbindungen der Formel (A-3; z. B. X₂, X₃ = CH) umgesetzt werden, die dann in einem zweiten Reaktionsschritt in Gegenwart eines geeigneten Phosphor(III)-reagenz, beispielsweise Triethylphosphit, reduktiv unter Bildung der Verbindungen (A-4; z. B. X₂, X₃ = CH) cyclisiert werden.

Wird beispielsweise zur Herstellung der Vorstufen (A-4; z. B. X₂, X₃ = CH) als Verbindung der Formel (A-1) 5-Brom-2-nitro-benzaldehyd (LG = Br) und als Verbindung der Formel (A-2) 3-Pyridinamin (A-a = Pyridin-3-yl) eingesetzt, so entsteht zunächst das *N*-[(5-Brom-2-nitro-phenyl)methylen]-3-pyridinamin der Formel (A-3) (A-a = Pyridin-3-yl, X₂, X₃ = CH LG = Br). Nachfolgende Reduktion und Cyclisierung führt dann zum 5-Brom-2-(pyridin-3-yl)-2*H*-indazol (A-4, A-a = Pyridin-3-yl, X₂, X₃ = CH, LG = Br) (vgl. Ausgangsprodukt für die Herstellungsbeispiele 1 und 2).

Die Verbindungen der Formel (A-1) sind teilweise bekannt und kommerziell erhältlich bzw. können nach im Prinzip bekannten Herstellungsverfahren (für X₂, X₃ = CH, LG = Br; 5-Brom-2-nitrobenzaldehyd (WO 2014/121416 A1)) erhalten werden.

Die Verbindungen der Formel (A-2) sind teilweise bekannt und kommerziell erhältlich bzw. können nach im Prinzip bekannten Herstellungsverfahren erhalten werden, vgl. beispielsweise für A = 5-Fluor-pyridin-3-yl (A-a; B² = H, G¹ = C-F; T = Elektronenpaar) (WO 2011/ 123751 A2); Pyrazin-2-yl (A-b; B² = H) (WO 2012/ 151567 A1); Pyridazin-4-yl (A-c; B² = H) (WO 2011/038572 A1); Thiazol-5-yl (A-d; B² = H) (JP 4600 6049 B4); Isothiazol-4-yl (A-e; B² = H) (US 2,839,529) oder 1-Methyl-1*H*-pyrazol-4-yl (A-f; B² = H, R³ = CH₃).

Die Vorstufen (A-4) können nach im Prinzip bekannten Synthesemethoden erhalten werden, vgl. beispielsweise für A = Pyridin-3-yl (A-a; B² = H, G¹ = CH; T = Elektronenpaar; R₁, R₂ = H) (S. Ostrowski, A. M. Wolniewicz, Chem. Het. Compd. (New York) (Transl. Khim. Geterotsikl. Soedin.) 2000, 36(6), 705-713) oder für A = Pyrimid-2-yl (A-b; B², R₁, R₂ = H) (A. L. El-Ansary et al., Egypt. J. Chem. 1991, 33(2), 129-145).

Beispielhaft wird eine Auswahl an erfindungsgemäß zu verwendenden Vorstufen (A-4) für die Verbindungen der Formel (I) genannt:
- 5-Brom-2-(3-pyridinyl)-2*H*-indazol (A-a = Pyridin-3-yl; X₁, X₂, X₃ = CH; LG = Br) (vgl. WO 2016/071499 A1 und WO 2016/087421 A1), und
- 5-Brom-2-(3-pyridinyl)-2*H*-pyrazolo[3,4-*b*]pyridin, (A-a = Pyridin-3-yl; X₁ = N, X₂, X₃ = CH; LG = Br) (vgl. WO 2016087368 A1).

Schliesslich lassen sich die *ortho*-Imino-nitrobenzene Vorstufen (A-3) gemäss dem zweiten Reaktionsschritt mittels einer reduktiven Cyclisierung, beispielsweise nach der Candogan Indazolsynthese in Gegenwart von Triethylphosphit, erhalten (vgl. J. I. G. Candogan et al., J. Chem. Soc. 1965, 4831).

Alternativ können auch abgewandelte Reaktionsbedingungen der reduktiven Cyclisierung nach Candogan *et al.* genutzt werden oder entsprechende alternative Reaktionsbedingungen Verwendung finden, wie beispielsweise die Übergangsmetall-katalysierte reduktive Cyclisierung von Iminonitroaromaten und die thermische Übergangsmetall-katalysierte Cyclisierung von 2-Azido-iminen (vgl. N. E. Genung et al., Org. Lett. 2014, 16, 3114-3117 und darin zitierte Literatur).

Alternativ können die 2-halogensubstituierten 3-Pyridincarboxaldehyde der Formel (A-5; X₁= N) in einem ersten Reaktionsschritt mit Natriumazid in die entsprechenden 2-Azido-3-pyridincarboxaldehyde der Formel (A-6; X₁= N) überführt werden, die dann mit 3-aminosubstituierten Heterocyclen der Formel (A-2) in einem zweiten Reaktionsschritt zu Verbindungen der Formel (A-7; X₁= N), sogenannten *"Schiff Basen"*, reagieren und in einem dritten Reaktionsschritt unter Rückflußtemperatur in einem geeigneten Lösungsmittel unter Bildung der Vorstufen (A-8; X₁= N) cyclisieren (vgl. beispielsweise für 5-Brom-2-(3-pyridyl)pyrazolo[3,4-*b*]pyridin (A-a; B² = H; X₁= N, X₂, X₃ = CH; G¹ = C-H; T = Elektronenpaar; Reaktionsschema II).

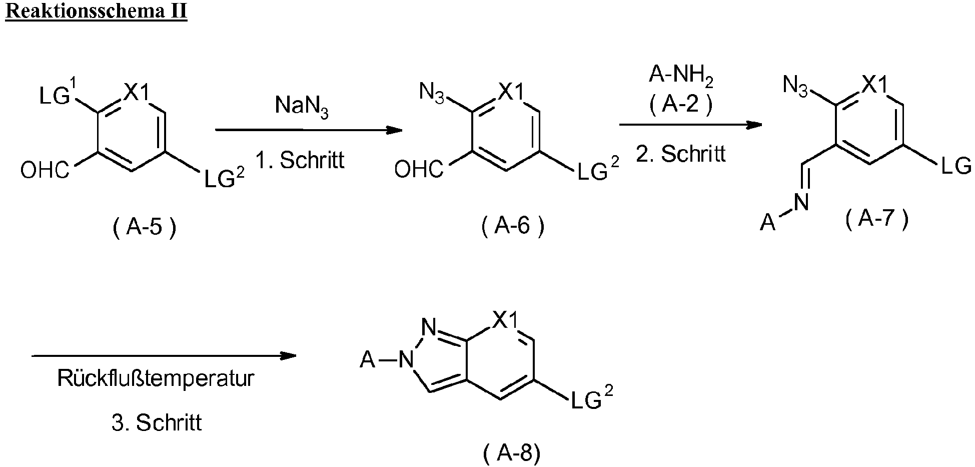

Im Reaktionsschema II haben A und X₁ die weiter oben genannte Bedeutung und LG¹ sowie LG² stehen für geeignete nucleofuge Abgangsgruppen, bevorzugt für Halogen.

Verbindungen der Formel (I), in denen A, X₁, X₂, X₃ und R₁ die weiter oben genannte Bedeutung haben, können gemäß Reaktionsschema III aus den entsprechenden Vorstufen (A-4) und (A-8) erhalten werden.

Beispielsweise können die Verbindungen der Formel (I), in denen A, G², R₂, X₁, X₂ und X₃ die weiter oben genannte Bedeutung haben und R₁ für einen Rest (B-1) und (B-2) steht, gemäß Reaktionsschema III erhalten werden (vgl. auch Herstellungsbeispiele 1 bis 29).

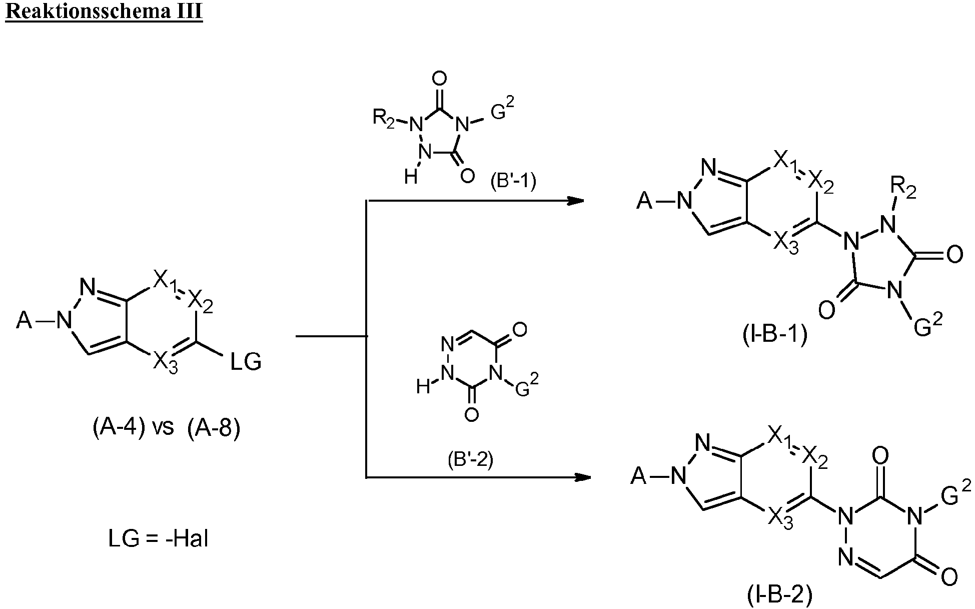

Im Reaktionsschema III besitzen die Vorstufen (A-4) und (A-8) der Verbindungen (I), in denen A, X₁, X₂ und X₃ die weiter oben genannte Bedeutung haben, eine gegebenenfalls *in-situ* erzeugte nucleofuge Abgangsgruppe LG bzw. ein Halogen wie beispielsweise Brom.

Beispielsweise können gegebenenfalls 1,4-substituierte 1,2,4-Triazolidin-3,5-dione der Formel (B'-1) oder 4-substituierte 1,2,4-Triazine-3,5(2*H*,4*H*)-dione der Formel (B'-2) mit den entsprechenden Vorstufen (A-4) und (A-8) zu Verbindungen der Formel (I) umgesetzt werden.

Die Verbindungen der Formel (B'-1) sind teilweise bekannt und kommerziell erhältlich bzw. können nach im Prinzip bekannten Herstellungsverfahren erhalten werden (vgl. auch Beispiele (B'-1)-1 bis (B'-1)-10 im Experimentellen Teil).

Die Verbindungen der Formel (B'-1), in denen R₂ und G² die weiter oben genannte Bedeutung haben, können beispielsweise aus entsprechenden N-substituierten Hydrazincarbonsäureestern der Formel (A-9) und Isocyanaten der Formel (A-10), gemäß Reaktionsschema IV erhalten werden (vgl. Beispiel in WO 99/07687 mit R = Ethyl, R₂ = Methyl; G² = 3-Brom-benzyl).

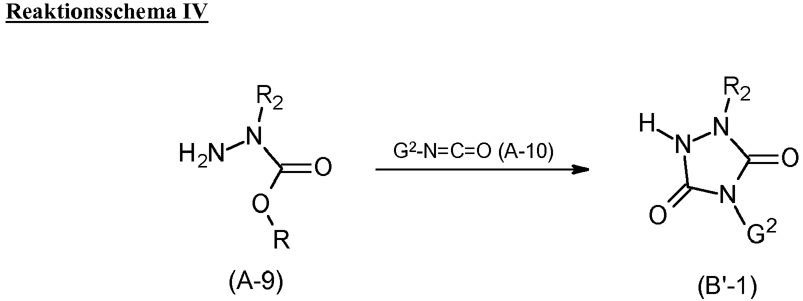

Beispielhaft wird eine Auswahl an erfindungsgemäß zu verwendenden 1,2,4-Triazolidin-3,5-dionen der Formel (B'-1) genannt, die beschrieben oder kommerziell erhältlich sind:
- 1,4-Dimethyl-1,2,4-triazolidin-3,5-dion (CAS-Nr. 34771-26-1; Apichemical (Shanghai)),
- 1-Methyl-4-phenyl-1,2,4-triazolidin-3,5-dion (CAS-Nr. 28538-67-2; Apichemical (Shanghai)),
- 1-Methyl-4-(1-methylethyl)-1,2,4-triazolidin-3,5-dion (CAS-Nr. 1641986-25-5; vgl. WO 2014/202505 A1),
- 4-Ethyl-1-methyl-1,2,4-triazolidin-3,5-dion (CAS-Nr. 1641986-30-2; vgl. WO 2014/ 202505 A1),
- 1-Methyl-4-(2-propen-1-yl)-1,2,4-triazolidin-3,5-dion (CAS-Nr. 801213-17-2; vgl. WO 99/ 07687 A1),
- 4-(1,1-Dimethylethyl)-1-methyl-1,2,4-triazolidin-3,5-dione (CAS-Nr. 1641986-16-4; vgl. WO 2014/ 202505 A1),
- 4-Butyl-1-methyl-1,2,4-triazolidin-3,5-dion (CAS-Nr. 10270-05-0; vgl. W. S. Jr. Wadsworth, W. D. Emmons, J. Org. Chem. 1967, 32(5), 1279-85),
- 1-Methyl-4-phenyl-1,2,4-triazolidin-3,5-dion (CAS-Nr. 28538-67-2 , Apichemical (Shanghai)),
- 4-[(3-bromophenyl)methyl]-1-methyl-1,2,4-triazolidin-3,5-dion, (CAS-Nr. 220464-61-9; vgl. WO 99/ 07687 A1).

Die Verbindungen der Formel (B'-2), in denen G² die weiter oben genannte Bedeutung haben, können beispielsweise aus dem kommerziell erhältlichen 1,2,4-Triazine-3,5(2*H*,4*H*)-dion der Formel (A-11) und Verbindungen Formel (A-12), in denen LG für eine gegebenenfalls *in-situ* erzeugte nucleofuge Abgangsgruppe LG steht (z. B. Halogen), gemäß Reaktionsschema V erhalten werden (vgl. J. S. D. Kumar et al., J. Med. Chem. 2006, 49, 125-134; vgl. auch Beispiele (B'-2)-1 und (B'-2)-2 im Experimentellen Teil).

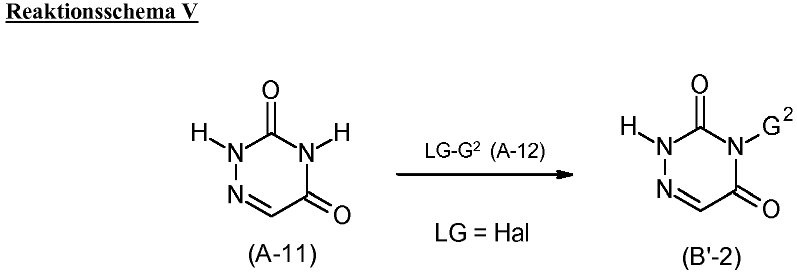

Beispielhaft wird eine Auswahl an erfindungsgemäß zu verwendenden 1,2,4-Triazolidin-3,5(2*H*,4*H*)-dionen der Formel (B'-2) genannt, die beschrieben oder kommerziell erhältlich sind:
- 4-Methyl-1,2,4-triazin-3,5(2*H*,4*H*)-dion (CAS-Nr. 1627-30-1; AK Scientific)
- 4-Ethyl-1,2,4-triazin-3,5(2*H*,4*H*)-dion (CAS-Nr. 1627-31-2; Apichemical (Shanghai)),
- 4-(2-Propen-1-yl)-1,2,4-triazin-3,5(2*H*,4*H*)-dion (CAS-Nr. 79944-17-5; Apichemical (Shanghai)),
- 4-(2-Propyn-1-yl)-1,2,4-triazin-3,5(2*H*,4*H*)-dione (CAS-Nr. 79944-18-6 ; vgl. EP 33163 A2),
- 4-Butyl-1,2,4-triazin-3,5(2*H*,4*H*)-dion (CAS-Nr. 61958-55-2 ; Apichemical (Shanghai))
- 4-(2-fluoroethyl)-1,2,4-triazin-3,5(2*H*,4*H*)-dion (CAS-Nr. 1351411-61-4 ; FCH Group Reagents for Synthesis),
- 4-(Phenylmethyl)-1,2,4-Triazin-3,5(2*H*,4*H*)-dion (CAS-Nr. 5579-47-5 ; Shanghai Chemhere)
- 4-[(4-chlorophenyl)methyl]-1,2,4-triazin-3,5(2*H*,4*H*)-dion (CAS-Nr. 1207441-70-0; Hong Kong Chemhere).

Die Verbindungen der Formel (I) können analog zu literaturbekannten Methoden, durch Umsetzung von Halogeniden der Formel (A-4) (z. B. LG = Br) oder (A-8) (z. B. LG = Br) mit heterocyclischen Verbindungen der Formel (B'-1) oder (B'-2) hergestellt werden.

Bevorzugt findet die Umsetzung durch Übergangsmetall-Katalyse oder -Vermittlung statt. Beispielhafte Reaktionsbedingungen für die Umsetzung von Arylbromiden mit Triazolinonen sind in der Literatur zahlreich bekannt, zum Beispiel in WO 2013/062027 A1 und WO 2015/035059 A1.

Bevorzugt werden Kupfer oder Kupfersalze, zum Beispiel Kupfer(I)-iodid, Kupfer(I)-oxid oder Kupfer(I)-triflat, als Katalysator verwendet, häufig in Gegenwart eines Liganden, zum Beispiel Diamin-Liganden wie *N,N'*-Dimethylethylendiamin oder *trans-N,N'*-Dimethylcyclohexan-1,2-diamin (vgl. beispielsweise WO 2013/062027 A1). Alternativ können 1,3-Diketone, wie z.B. 2,4-Pentandion, 2,2,6,6-Tetramethyl-3,5-heptandion oder Dibenzoylmethan, Aminosäuren, wie z.B. L-Prolin oder Glycin (vgl. WO 2015/035059 A1) oder andere Verbindungen wie 8-Hydroxychinolin oder Bipyridin Verwendung finden. In der Regel wird die Umsetzung unter Anwesenheit einer Base, häufig Carbonat- oder Phosphat-Basen, wie zum Beispiel Kaliumcarbonat, Natriumcarbonat, Cäsiumcarbonat oder Kaliumphosphat, in geeigneten Lösungsmitteln wie zum Beispiel Dioxan, Toluol, Dimethylsulfoxid oder *N,N-*Dimethylformamid durchgeführt. Desweiteren können Additive wie z. B. Kaliumiodid, Cäsiumfluorid oder andere Salze Einsatz finden.

Alternativ lassen sich derartige Umsetzungen unter Palladiumkatalyse in Analogie zu literaturbekannten Methoden durchführen, etwa durch Einsatz von Palladium-basierten Katalysatoren wie zum Beispiel Palladiumacetat, Tetrakis(triphenylphosphin)palladium, Bis-(triphenylphosphin)-palladium(II)-chlorid, Tris-(dibenzylidenaceton)-dipalladium(0) in Gegenwart von Liganden, zum Beispiel 2,2'-Bis-(diphenylphosphino)-1,1'-binaphthyl (BINAP), 9,9-Dimethyl-4,5-bis-(diphenylphosphino)-xanthen, 1,1'-Bis-(diphenylphosphino)-ferrocen, und Basen wie zum Beispiel Kaliumcarbonat, Natriumcarbonat, Cäsiumcarbonat oder Kaliumphosphat, in geeigneten Lösungsmitteln wie zum Beispiel Dioxan, Toluol, Dimethylsulfoxid oder *N,N*-Dimethylformamid.

Beispielhafte Reaktionsbedingungen finden sich in der Literatur, zum Beispiel in WO 2005/085226 und WO 2006/067139 A1. Der Einsatz von Palladium(0)-Quellen wie z.B. Tris-(dibenzylidenaceton)-dipalladium(0), in Kombination mit Liganden wie 5-(Di-*tert*-butylphosphino)-1,3,5-triphenyl-1-*H-*[1,4]bipyrazol für die Kupplung zwischen Arylhalogeniden und Amiden, findet bevorzugt in Lösungsmittel wie Ether, z.B. Dimethoxyethan, und Alkoholen, wie tert-Butylalkohol oder *tert-*Amylalkohol, und ist beispielsweise in US 20070244178 A1, T. Ikawa, et al. J. Amer. Chem. Soc. 2007, 129, 13001-13007, und A. J. Rosenberg, et al. Org. Lett. 2012, 14, 1764-1767, beschrieben.

Verbindungen der Formel (I) lassen sich auch analog zu literaturbekannten Methoden, durch Umsetzung von Boronsäuren der Formel (A-4 oder A-8, in denen LG für B(OH)₂ steht) mit heterocyclischen Verbindungen der Formel (B'-1) oder (B'-2), herstellen.

In der Regel finden die Reaktionen unter Katalyse oder Vermittlung durch Kupfer(II)-salze wie zum Beispiel Kupfer(II)-acetat, Kupfer(II)triflat oder auch durch Kupfer(I)-salze wie zum Beispiel Kupfer(I)-chlorid, Kupfer(I)-acetat unter Luft- oder Sauerstoffatmosphäre, häufig unter wasserentziehenden Bedingungen (zum Beispiel mit Molekularsieb) statt.

Als Basen werden zum Beispiel Triethylamin, *N*-Ethyldiisopropylamin, Pyridin, 2,6-Lutidin, *N*-Methylmorpholin oder 1,8-Diazabicycloundec-7-en in geeigneten Lösungmitteln wie zum Beispiel Dichlormethan, Dichlorethan, Methanol, *N,N-*Dimethylformamid, Tetrahydrofuran, Dioxan, Acetonitril, Essigester oder Toluol verwendet. In der Literatur werden zahlreiche Beispiele beschrieben, unter anderem in Q. Tan Darlene, et al. Proc. Natl. Acad. Sci. USA 2011, 108 (17), 6781-6786, in WO 2005/85226 oder in WO 2008/62905 A2. Zusammenfassende Übersichten finden sich zum Beispiel in J. X. Qiao, P. Y. S. Lam Synthesis 2011, 6, 829-856 oder in R. Sanjeeva, T.-S. Wu Tetrahedron 2012, 68, 7735-7754

Anstelle der Boronsäure können auch andere Borverbindungen wie etwa Kaliumtrifluorborate, Boronsäureester etc. sowie andere Organometallverbindungen wie etwa Stannane, Silane oder Bismuthane verwendet werden.

Die Enantiomere können auch aus dem Racemat gewonnen werden, beispielsweise durch präparative Trennung mittels einer chiralen HPLC.

### Isomere

Die Verbindungen der Formel (I) können in Abhängigkeit von der Art der Substituenten als geometrische und/oder als optisch aktive Isomere oder entsprechende Isomerengemische in unterschiedlicher Zusammensetzung vorliegen. Diese Stereoisomere sind beispielsweise Enantiomere, Diastereomere, Atropisomere oder geometrische Isomere. Die Erfindung umfasst somit reine Stereoisomere als auch beliebige Gemische dieser Isomere.

### Verfahren und Verwendungen

Die Erfindung betrifft auch Verfahren zur Bekämpfung von tierischen Schädlingen, bei dem man Verbindungen der Formel (I) auf tierische Schädlinge und/oder ihren Lebensraum einwirken lässt. Bevorzugt wird die Bekämpfung der tierischen Schädlinge in der Land- und Forstwirtschaft und im Materialschutz durchgeführt. Hierunter vorzugsweise ausgeschlossen sind Verfahren zur chirurgischen oder therapeutischen Behandlung des menschlichen oder tierischen Körpers und Diagnostizierverfahren, die am menschlichen oder tierischen Körper vorgenommen werden.

Die Erfindung betrifft ferner die Verwendung der Verbindungen der Formel (I) als Schädlingsbekämpfungsmittel, insbesondere Pflanzenschutzmittel.

Im Rahmen der vorliegenden Anmeldung umfasst der Begriff Schädlingsbekämpfungsmittel auch den Begriff Pflanzenschutzmittel.

Die Verbindungen der Formel (I) eignen sich bei guter Pflanzenverträglichkeit, günstiger Warmblütertoxizität und guter Umweltverträglichkeit zum Schutz von Pflanzen und Pflanzenorganen vor biotischen und abiotischen Stressfaktoren, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Aquakulturen, in Forsten, in Gärten und Freizeiteinrichtungen, im Vorrats- und Materialschutz sowie auf dem Hygienesektor vorkommen. Sie können vorzugsweise als Schädlingsbekämpfungsmittel eingesetzt werden. Sie sind gegen normal sensible und resistente Arten sowie gegen alle oder einzelne Entwicklungsstadien wirksam. Zu den oben erwähnten Schädlingen gehören:
Schädlinge aus dem Stamm der Arthropoda, insbesondere aus der Klasse der Arachnida z.B. Acarus spp., z.B. Acarus siro, Aceria kuko, Aceria sheldoni, Aculops spp., Aculus spp., z.B. Aculus fockeui, Aculus schlechtendali, Amblyomma spp., Amphitetranychus viennensis, Argas spp., Boophilus spp., Brevipalpus spp., z.B. Brevipalpus phoenicis, Bryobia graminum, Bryobia praetiosa, Centruroides spp., Chorioptes spp., Dermanyssus gallinae, Dermatophagoides pteronyssinus, Dermatophagoides farinae, Dermacentor spp., Eotetranychus spp., z.B. Eotetranychus hicoriae, Epitrimerus pyri, Eutetranychus spp., z.B. Eutetranychus banksi, Eriophyes spp., z.B. Eriophyes pyri, Glycyphagus domesticus, Halotydeus destructor, Hemitarsonemus spp., z.B. Hemitarsonemus latus (=Polyphagotarsonemus latus), Hyalomma spp., Ixodes spp., Latrodectus spp., Loxosceles spp., Neutrombicula autumnalis, Nuphersa spp., Oligonychus spp., z.B. Oligonychus coniferarum, Oligonychus ilicis, Oligonychus indicus, Oligonychus mangiferus, Oligonychus pratensis, Oligonychus punicae, Oligonychus yothersi, Ornithodorus spp., Ornithonyssus spp., Panonychus spp., z.B. Panonychus citri (=Metatetranychus citri), Panonychus ulmi (=Metatetranychus ulmi), Phyllocoptruta oleivora, Platytetranychus multidigituli, Polyphagotarsonemus latus, Psoroptes spp., Rhipicephalus spp., Rhizoglyphus spp., Sarcoptes spp., Scorpio maurus, Steneotarsonemus spp., Steneotarsonemus spinki, Tarsonemus spp., z.B. Tarsonemus confusus, Tarsonemus pallidus, Tetranychus spp., z.B. Tetranychus canadensis, Tetranychus cinnabarinus, Tetranychus turkestani, Tetranychus urticae, Trombicula alfreddugesi, Vaejovis spp., Vasates lycopersici;
aus der Klasse der Chilopoda z.B. Geophilus spp., Scutigera spp.;
aus der Ordnung oder der Klasse der Collembola z.B. Onychiurus armatus; Sminthurus viridis;
aus der Klasse der Diplopoda z.B. Blaniulus guttulatus;
aus der Klasse der Insecta, z.B. aus der Ordnung der Blattodea z.B. Blatta orientalis, Blattella asahinai, Blattella germanica, Leucophaea maderae, Panchlora spp., Parcoblatta spp., Periplaneta spp., z.B. Periplaneta americana, Periplaneta australasiae, Supella longipalpa;
aus der Ordnung der Coleoptera z.B. Acalymma vittatum, Acanthoscelides obtectus, Adoretus spp., Agelastica alni, Agriotes spp., z.B. Agriotes linneatus, Agriotes mancus, Alphitobius diaperinus, Amphimallon solstitialis, Anobium punctatum, Anoplophora spp., Anthonomus spp., z.B. Anthonomus grandis, Anthrenus spp., Apion spp., Apogonia spp., Atomaria spp., z.B. Atomaria linearis, Attagenus spp., Baris caerulescens, Bruchidius obtectus, Bruchus spp., z.B. Bruchus pisorum, Bruchus rufimanus, Cassida spp., Cerotoma trifurcata, Ceutorrhynchus spp., z.B. Ceutorrhynchus assimilis, Ceutorrhynchus quadridens, Ceutorrhynchus rapae, Chaetocnema spp., z.B. Chaetocnema confinis, Chaetocnema denticulata, Chaetocnema ectypa, Cleonus mendicus, Conoderus spp., Cosmopolites spp., z.B. Cosmopolites sordidus, Costelytra zealandica, Ctenicera spp., Curculio spp., z.B. Curculio caryae, Curculio caryatrypes,Curculio obtusus, Curculio sayi, Cryptolestes ferrugineus, Cryptolestes pusillus, Cryptorhynchus lapathi, Cryptorhynchus mangiferae, Cylindrocopturus spp., Cylindrocopturus adspersus, Cylindrocopturus furnissi, Dermestes spp., Diabrotica spp., z.B. Diabrotica balteata, Diabrotica barberi, Diabrotica undecimpunctata howardi, Diabrotica undecimpunctata undecimpunctata, Diabrotica virgifera virgifera, Diabrotica virgifera zeae, Dichocrocis spp., Dicladispa armigera, Diloboderus spp., Epilachna spp., z.B. Epilachna borealis, Epilachna varivestis, Epitrix spp., z.B. Epitrix cucumeris, Epitrix fuscula, Epitrix hirtipennis, Epitrix subcrinita, Epitrix tuberis, Faustinus spp., Gibbium psylloides, Gnathocerus cornutus, Hellula undalis, Heteronychus arator, Heteronyx spp., Hylamorpha elegans, Hylotrupes bajulus, Hypera postica, Hypomeces squamosus, Hypothenemus spp., z.B. Hypothenemus hampei, Hypothenemus obscurus, Hypothenemus pubescens, Lachnosterna consanguinea, Lasioderma serricorne, Latheticus oryzae, Lathridius spp., Lema spp., Leptinotarsa decemlineata, Leucoptera spp., z.B. Leucoptera coffeella, Lissorhoptrus oryzophilus, Lixus spp., Luperomorpha xanthodera, Luperodes spp., Lyctus spp., Megascelis spp., Melanotus spp., z.B. Melanotus longulus oregonensis, Meligethes aeneus, Melolontha spp., z.B. Melolontha melolontha, Migdolus spp., Monochamus spp., Naupactus xanthographus, Necrobia spp., Niptus hololeucus, Oryctes rhinoceros, Oryzaephilus surinamensis, Oryzaphagus oryzae, Otiorhynchus spp., z.B. Otiorhynchus cribricollis, Otiorhynchus ligustici, Otiorhynchus ovatus, Otiorhynchus rugosostriarus, Otiorhynchus sulcatus, Oxycetonia jucunda, Phaedon cochleariae, Phyllophaga spp., Phyllophaga helleri, Phyllotreta spp., z.B. Phyllotreta armoraciae, Phyllotreta pusilla, Phyllotreta ramosa, Phyllotreta striolata, Popillia japonica, Premnotrypes spp., Prostephanus truncatus, Psylliodes spp., z.B. Psylliodes affinis, Psylliodes chrysocephala, Psylliodes punctulata, Ptinus spp., Rhizobius ventralis, Rhizopertha dominica, Sitophilus spp., z.B. Sitophilus granarius, Sitophilus linearis, Sitophilus oryzae, Sitophilus zeamais, Sphenophorus spp., Stegobium paniceum, Sternechus spp., z.B. Sternechus paludatus, Symphyletes spp., Tanymecus spp., z.B. Tanymecus dilaticollis, Tanymecus indicus, Tanymecus palliatus, Tenebrio molitor, Tenebrioides mauretanicus, Tribolium spp., z.B. Tribolium audax, Tribolium castaneum, Tribolium confusum, Trogoderma spp., Tychius spp., Xylotrechus spp., Zabrus spp., z.B. Zabrus tenebrioides;
aus der Ordnung der Diptera z.B. Aedes spp., z.B. Aedes aegypti, Aedes albopictus, Aedes sticticus, Aedes vexans, Agromyza spp., z.B. Agromyza frontella, Agromyza parvicornis, Anastrepha spp., Anopheles spp., z.B. Anopheles quadrimaculatus, Anopheles gambiae, Asphondylia spp., Bactrocera spp., z.B. Bactrocera cucurbitae, Bactrocera dorsalis, Bactrocera oleae, Bibio hortulanus, Calliphora erythrocephala, Calliphora vicina, Ceratitis capitata, Chironomus spp., Chrysomya spp., Chrysops spp., Chrysozona pluvialis, Cochliomya spp., Contarinia spp., z.B. Contarinia johnsoni, Contarinia nasturtii, Contarinia pyrivora, Contarinia schulzi, Contarinia sorghicola, Contarinia tritici,Cordylobia anthropophaga, Cricotopus sylvestris, Culex spp., z.B. Culex pipiens, Culex quinquefasciatus, Culicoides spp., Culiseta spp., Cuterebra spp., Dacus oleae, Dasineura spp., z.B. Dasineura brassicae, Delia spp., z.B. Delia antiqua, Delia coarctata, Delia florilega, Delia platura, Delia radicum, Dermatobia hominis, Drosophila spp., z.B. Drosphila melanogaster, Drosophila suzukii, Echinocnemus spp., Fannia spp., Gasterophilus spp., Glossina spp., Haematopota spp., Hydrellia spp., Hydrellia griseola, Hylemya spp., Hippobosca spp., Hypoderma spp., Liriomyza spp., z.B. Liriomyza brassicae, Liriomyza huidobrensis, Liriomyza sativae, Lucilia spp., z.B. Lucilia cuprina, Lutzomyia spp., Mansonia spp., Musca spp., z.B. Musca domestica, Musca domestica vicina, Oestrus spp., Oscinella frit, Paratanytarsus spp., Paralauterborniella subcincta, Pegomya spp., z.B. Pegomya betae, Pegomya hyoscyami, Pegomya rubivora, Phlebotomus spp., Phorbia spp., Phormia spp., Piophila casei, Prodiplosis spp., Psila rosae, Rhagoletis spp., z.B. Rhagoletis cingulata, Rhagoletis completa, Rhagoletis fausta, Rhagoletis indifferens, Rhagoletis mendax, Rhagoletis pomonella, Sarcophaga spp., Simulium spp., z.B. Simulium meridionale, Stomoxys spp., Tabanus spp., Tetanops spp., Tipula spp., z.B. Tipula paludosa, Tipula simplex;
aus der Ordnung der Hemiptera z.B. Acizzia acaciaebaileyanae, Acizzia dodonaeae, Acizzia uncatoides, Acrida turrita, Acyrthosipon spp., z.B. Acyrthosiphon pisum, Acrogonia spp., Aeneolamia spp., Agonoscena spp., Aleyrodes proletella, Aleurolobus barodensis, Aleurothrixus floccosus, Allocaridara malayensis, Amrasca spp., z.B. Amrasca bigutulla, Amrasca devastans, Anuraphis cardui, Aonidiella spp., z.B. Aonidiella aurantii, Aonidiella citrina, Aonidiella inornata, Aphanostigma piri, Aphis spp., z.B. Aphis citricola, Aphis craccivora, Aphis fabae, Aphis forbesi, Aphis glycines, Aphis gossypii, Aphis hederae, Aphis illinoisensis, Aphis middletoni, Aphis nasturtii, Aphis nerii, Aphis pomi, Aphis spiraecola, Aphis viburniphila, Arboridia apicalis, Arytainilla spp., Aspidiella spp., Aspidiotus spp., z.B. Aspidiotus nerii, Atanus spp., Aulacorthum solani, Bemisia tabaci, Blastopsylla occidentalis, Boreioglycaspis melaleucae, Brachycaudus helichrysi, Brachycolus spp., Brevicoryne brassicae, Cacopsylla spp., z.B. Cacopsylla pyricola, Calligypona marginata, Carneocephala fulgida, Ceratovacuna lanigera, Cercopidae, Ceroplastes spp., Chaetosiphon fragaefolii, Chionaspis tegalensis, Chlorita onukii, Chondracris rosea, Chromaphis juglandicola, Chrysomphalus ficus, Cicadulina mbila, Coccomytilus halli, Coccus spp., z.B. Coccus hesperidum, Coccus longulus, Coccus pseudomagnoliarum, Coccus viridis, Cryptomyzus ribis, Cryptoneossa spp., Ctenarytaina spp., Dalbulus spp., Dialeurodes citri, Diaphorina citri, Diaspis spp., Drosicha spp., Dysaphis spp., z.B. Dysaphis apiifolia, Dysaphis plantaginea, Dysaphis tulipae, Dysmicoccus spp., Empoasca spp., z.B. Empoasca abrupta, Empoasca fabae, Empoasca maligna, Empoasca solana, Empoasca stevensi, Eriosoma spp., z.B. Eriosoma americanum, Eriosoma lanigerum, Eriosoma pyricola, Erythroneura spp., Eucalyptolyma spp., Euphyllura spp., Euscelis bilobatus, Ferrisia spp., Geococcus coffeae, Glycaspis spp., Heteropsylla cubana, Heteropsylla spinulosa, Homalodisca coagulata, Hyalopterus arundinis, Hyalopterus pruni, Icerya spp., z.B. Icerya purchasi, Idiocerus spp., Idioscopus spp., Laodelphax striatellus, Lecanium spp., z.B. Lecanium corni (=Parthenolecanium corni), Lepidosaphes spp., z.B. Lepidosaphes ulmi, Lipaphis erysimi, Lycorma delicatula, Macrosiphum spp., z.B. Macrosiphum euphorbiae, Macrosiphum lilii, Macrosiphum rosae, Macrosteles facifrons, Mahanarva spp., Melanaphis sacchari, Metcalfiella spp., Metcalfa pruinosa, Metopolophium dirhodum, Monellia costalis, Monelliopsis pecanis, Myzus spp., z.B. Myzus ascalonicus, Myzus cerasi, Myzus ligustri, Myzus ornatus, Myzus persicae,. Myzus nicotianae, Nasonovia ribisnigri, Nephotettix spp., z.B. Nephotettix cincticeps,, Nephotettix nigropictus, Nilaparvata lugens, Oncometopia spp., Orthezia praelonga, Oxya chinensis, Pachypsylla spp., Parabemisia myricae, Paratrioza spp., z.B. Paratrioza cockerelli, Parlatoria spp., Pemphigus spp., z.B. Pemphigus bursarius, Pemphigus populivenae, Peregrinus maidis, Phenacoccus spp., z.B. Phenacoccus madeirensis, Phloeomyzus passerinii, Phorodon humuli, Phylloxera spp., z.B. Phylloxera devastatrix, Phylloxera notabilis, Pinnaspis aspidistrae, Planococcus spp., z.B. Planococcus citri, Prosopidopsylla flava, Protopulvinaria pyriformis, Pseudaulacaspis pentagona, Pseudococcus spp., z.B. Pseudococcus calceolariae, Pseudococcus comstocki, Pseudococcus longispinus, Pseudococcus maritimus, Pseudococcus viburni, Psyllopsis spp., Psylla spp., z.B. Psylla buxi, Psylla mali, Psylla pyri, Pteromalus spp., Pyrilla spp., Quadraspidiotus spp., z.B. Quadraspidiotus juglansregiae, Quadraspidiotus ostreaeformis, Quadraspidiotus perniciosus, Quesada gigas, Rastrococcus spp., Rhopalosiphum spp., z.B. Rhopalosiphum maidis, Rhopalosiphum oxyacanthae, Rhopalosiphum padi, Rhopalosiphum rufiabdominale, Saissetia spp., z.B. Saissetia coffeae, Saissetia miranda, Saissetia neglecta, Saissetia oleae, Scaphoideus titanus, Schizaphis graminum, Selenaspidus articulatus, Sitobion avenae, Sogata spp., Sogatella furcifera, Sogatodes spp., Stictocephala festina, Siphoninus phillyreae, Tenalaphara malayensis,Tetragonocephela spp., Tinocallis caryaefoliae, Tomaspis spp., Toxoptera spp., z.B. Toxoptera aurantii, Toxoptera citricidus, Trialeurodes vaporariorum, Trioza spp., z.B. Trioza diospyri, Typhlocyba spp., Unaspis spp., Viteus vitifolii, Zygina spp.;
aus der Unterordnung der Heteroptera z.B. Anasa tristis, Antestiopsis spp., Boisea spp., Blissus spp., Calocoris spp., Campylomma livida, Cavelerius spp., Cimex spp., z.B. Cimex adjunctus, Cimex hemipterus, Cimex lectularius, Cimex pilosellus, Collaria spp., Creontiades dilutus, Dasynus piperis, Dichelops furcatus, Diconocoris hewetti, Dysdercus spp., Euschistus spp., z.B. Euschistus heros, Euschistus servus, Euschistus tristigmus, Euschistus variolarius, Eurygaster spp., Halyomorpha halys, Heliopeltis spp., Horcias nobilellus, Leptocorisa spp., Leptocorisa varicornis, Leptoglossus occidentalis, Leptoglossus phyllopus, Lygocoris spp., z.B. Lygocoris pabulinus, Lygus spp., z.B. Lygus elisus, Lygus hesperus, Lygus lineolaris, Macropes excavatus, Monalonion atratum, Nezara spp., z.B. Nezara viridula, Oebalus spp., Piesma quadrata, Piezodorus spp., z.B. Piezodorus guildinii, Psallus spp., Pseudacysta persea, Rhodnius spp., Sahlbergella singularis, Scaptocoris castanea, Scotinophora spp., Stephanitis nashi, Tibraca spp., Triatoma spp.;
aus der Ordnung der Hymenoptera z.B. Acromyrmex spp., Athalia spp., z.B. Athalia rosae, Atta spp., Diprion spp., z.B. Diprion similis, Hoplocampa spp., z.B. Hoplocampa cookei, Hoplocampa testudinea, Lasius spp., Linepithema humile, Monomorium pharaonis, Sirex spp., Solenopsis invicta, Tapinoma spp., Urocerus spp., Vespa spp., z.B. Vespa crabro, Xeris spp.;
aus der Ordnung der Isopoda z.B. Armadillidium vulgare, Oniscus asellus, Porcellio scaber;
aus der Ordnung der Isoptera z.B. Coptotermes spp., z.B. Coptotermes formosanus, Comitermes cumulans, Cryptotermes spp., Incisitermes spp., Microtermes obesi, Odontotermes spp., Reticulitermes spp., z.B. Reticulitermes flavipes, Reticulitermes hesperus;
aus der Ordnung der Lepidoptera z.B. Achroia grisella, Acronicta major, Adoxophyes spp., z.B. Adoxophyes orana, Aedia leucomelas, Agrotis spp., z.B. Agrotis segetum, Agrotis ipsilon, Alabama spp., z.B. Alabama argillacea, Amyelois transitella, Anarsia spp., Anticarsia spp., z.B. Anticarsia gemmatalis, Argyroploce spp., Barathra brassicae, Borbo cinnara, Bucculatrix thurberiella, Bupalus piniarius, Busseola spp., Cacoecia spp., Caloptilia theivora, Capua reticulana, Carpocapsa pomonella, Carposina niponensis, Cheimatobia brumata, Chilo spp., z.B. Chilo plejadellus, Chilo suppressalis, Choristoneura spp., Clysia ambiguella, Cnaphalocerus spp., Cnaphalocrocis medinalis, Cnephasia spp., Conopomorpha spp., Conotrachelus spp., Copitarsia spp., Cydia spp., z.B. Cydia nigricana, Cydia pomonella, Dalaca noctuides, Diaphania spp., Diatraea saccharalis, Earias spp., Ecdytolopha aurantium, Elasmopalpus lignosellus, Eldana saccharina, Ephestia spp., z.B. Ephestia elutella, Ephestia kuehniella, Epinotia spp., Epiphyas postvittana, Etiella spp., Eulia spp., Eupoecilia ambiguella, Euproctis spp., z.B. Euproctis chrysorrhoea, Euxoa spp., Feltia spp., Galleria mellonella, Gracillaria spp., Grapholitha spp., z.B. Grapholita molesta, Grapholita prunivora, Hedylepta spp., Helicoverpa spp., z.B. Helicoverpa armigera, Helicoverpa zea, Heliothis spp., z.B. Heliothis virescens Hofmannophila pseudospretella, Homoeosoma spp., Homona spp., Hyponomeuta padella, Kakivoria flavofasciata, Laphygma spp., Leucinodes orbonalis, Leucoptera spp., z.B. Leucoptera coffeella, Lithocolletis spp., z.B. Lithocolletis blancardella, Lithophane antennata, Lobesia spp., z.B. Lobesia botrana, Loxagrotis albicosta, Lymantria spp., z.b. Lymantria dispar, Lyonetia spp., z.B. Lyonetia clerkella, Malacosoma neustria, Maruca testulalis, Mamestra brassicae, Melanitis leda, Mocis spp., Monopis obviella, Mythimna separata, Nemapogon cloacellus, Nymphula spp., Oiketicus spp., Oria spp., Orthaga spp., Ostrinia spp., z.B. Ostrinia nubilalis, Oulema melanopus, Oulema oryzae, Panolis flammea, Parnara spp., Pectinophora spp., z.B. Pectinophora gossypiella, Perileucoptera spp., Phthorimaea spp., z.B. Phthorimaea operculella, Phyllocnistis citrella, Phyllonorycter spp., z.B. Phyllonorycter blancardella, Phyllonorycter crataegella, Pieris spp., z.B. Pieris rapae, Platynota stultana, Plodia interpunctella, Plusia spp., Plutella xylostella (=Plutella maculipennis), Prays spp., Prodenia spp., Protoparce spp., Pseudaletia spp., z.B. Pseudaletia unipuncta, Pseudoplusia includens, Pyrausta nubilalis, Rachiplusia nu, Schoenobius spp., z.B. Schoenobius bipunctifer, Scirpophaga spp., z.B. Scirpophaga innotata, Scotia segetum, Sesamia spp., z.B. Sesamia inferens, Sparganothis spp., Spodoptera spp., z.b. Spodoptera eradiana, Spodoptera exigua, Spodoptera frugiperda, Spodoptera praefica, Stathmopoda spp., Stomopteryx subsecivella, Synanthedon spp., Tecia solanivora, Thermesia gemmatalis, Tinea cloacella, Tinea pellionella, Tineola bisselliella, Tortrix spp., Trichophaga tapetzella, Trichoplusia spp., z.B. Trichoplusia ni, Tryporyza incertulas, Tuta absoluta, Virachola spp.;
aus der Ordnung der Orthoptera oder Saltatoria z.B. Acheta domesticus, Dichroplus spp., Gryllotalpa spp., z.B. Gryllotalpa gryllotalpa, Hieroglyphus spp., Locusta spp., z.B. Locusta migratoria, Melanoplus spp., z.B. Melanoplus devastator, Paratlanticus ussuriensis, Schistocerca gregaria;
aus der Ordnung der Phthiraptera z.B. Damalinia spp., Haematopinus spp., Linognathus spp., Pediculus spp., Phylloxera vastatrix, Phthirus pubis, Trichodectes spp.;
aus der Ordnung der Psocoptera z.B. Lepinotus spp., Liposcelis spp.;
aus der Ordnung der Siphonaptera z.B. Ceratophyllus spp., Ctenocephalides spp., z.B. Ctenocephalides canis, Ctenocephalides felis, Pulex irritans, Tunga penetrans, Xenopsylla cheopis;
aus der Ordnung der Thysanoptera z.B. Anaphothrips obscurus, Baliothrips biformis, Drepanothrips reuteri, Enneothrips flavens, Frankliniella spp., z.B. Frankliniella fusca, Frankliniella occidentalis, Frankliniella schultzei, Frankliniella tritici, Frankliniella vaccinii, Frankliniella williamsi, Heliothrips spp., Hercinothrips femoralis, Rhipiphorothrips cruentatus, Scirtothrips spp., Taeniothrips cardamomi, Thrips spp., z.B. Thrips palmi, Thrips tabaci;
aus der Ordnung der Zygentoma (= Thysanura), z. B. Ctenolepisma spp., Lepisma saccharina, Lepismodes inquilinus, Thermobia domestica;
aus der Klasse der Symphyla z.B. Scutigerella spp., z.B. Scutigerella immaculata;
Schädlinge aus dem Stamm der Mollusca, insbesondere aus der Klasse der Bivalvia, z.B. Dreissena spp.;
sowie aus der Klasse der Gastropoda z.B. Arion spp., z.B. Arion ater rufus, Biomphalaria spp., Bulinus spp., Deroceras spp., z.B. Deroceras laeve, Galba spp., Lymnaea spp., Oncomelania spp., Pomacea spp., Succinea spp.;
Tier- und Humanparasiten aus den Stämmen der Platyhelminthes und Nematoda, z.B. Aelurostrongylus spp., Amidostomum spp., Ancylostoma spp, Angiostrongylus spp., Anisakis spp., Anoplocephala spp., Ascaris spp., Ascaridia spp., Baylisascaris spp., Brugia spp., Bunostomum spp., Capillaria spp., Chabertia spp., Clonorchis spp., Cooperia spp., Crenosoma spp., Cyathostoma spp., Dicrocoelium spp., Dictyocaulus spp., Diphyllobothrium spp., Dipylidium spp., Dirofilaria spp., Dracunculus spp., Echinococcus spp., Echinostoma spp., Enterobius spp., Eucoleus spp., Fasciola spp., Fascioloides spp., Fasciolopsis spp., Filaroides spp., Gongylonema spp., Gyrodactylus spp., Habronema spp., Haemonchus spp., Heligmosomoides spp., Heterakis spp., Hymenolepis spp., Hyostrongylus spp., Litomosoides spp., Loa spp., Metastrongylus spp., Metorchis spp., Mesocestoides spp., Moniezia spp., Muellerius spp., Necator spp., Nematodirus spp., Nippostrongylus spp., Oesophagostomum spp., Ollulanus spp., Onchocerca spp, Opisthorchis spp., Oslerus spp., Ostertagia spp., Oxyuris spp., Paracapillaria spp., Parafilaria spp., Paragonimus spp., Paramphistomum spp., Paranoplocephala spp., Parascaris spp., Passalurus spp., Protostrongylus spp., Schistosoma spp., Setaria spp., Spirocerca spp., Stephanofilaria spp., Stephanurus spp., Strongyloides spp., Strongylus spp., Syngamus spp., Taenia spp., Teladorsagia spp., Thelazia spp., Toxascaris spp., Toxocara spp., Trichinella spp., Trichobilharzia spp., Trichostrongylus spp., Trichuris spp., Uncinaria spp., Wuchereria spp.;
Pflanzenschädlinge aus dem Stamm der Nematoda, d.h. pflanzenparasitäre Nematoden, insbesondere Aglenchus spp., z.B. Aglenchus agricola, Anguina spp., z.B. Anguina tritici, Aphelenchoides spp., z.B. Aphelenchoides arachidis, Aphelenchoides fragariae, Belonolaimus spp., z.B. Belonolaimus gracilis, Belonolaimus longicaudatus, Belonolaimus nortoni, Bursaphelenchus spp., z.B. Bursaphelenchus cocophilus, Bursaphelenchus eremus, Bursaphelenchus xylophilus, Cacopaurus spp., z.B. Cacopaurus pestis, Criconemella spp., z.B. Criconemella curvata, Criconemella onoensis, Criconemella ornata, Criconemella rusium, Criconemella xenoplax (= Mesocriconema xenoplax), Criconemoides spp., z.B. Criconemoides ferniae, Criconemoides onoense, Criconemoides ornatum, Ditylenchus spp., z.B. Ditylenchus dipsaci, Dolichodorus spp., Globodera spp., z.B. Globodera pallida, Globodera rostochiensis, Helicotylenchus spp., z.B. Helicotylenchus dihystera, Hemicriconemoides spp., Hemicycliophora spp., Heterodera spp., z.B. Heterodera avenae, Heterodera glycines, Heterodera schachtii, Hoplolaimus spp., Longidorus spp., z.B. Longidorus africanus, Meloidogyne spp., z.B. Meloidogyne chitwoodi, Meloidogyne fallax, Meloidogyne hapla, Meloidogyne incognita, Meloinema spp., Nacobbus spp., Neotylenchus spp., Paraphelenchus spp., Paratrichodorus spp., z.B. Paratrichodorus minor, Pratylenchus spp., z.B. Pratylenchus penetrans, Pseudohalenchus spp., Psilenchus spp., Punctodera spp., Quinisulcius spp., Radopholus spp., z.B. Radopholus citrophilus, Radopholus similis, Rotylenchulus spp., Rotylenchus spp., Scutellonema spp., Subanguina spp., Trichodorus spp., z.B. Trichodorus obtusus, Trichodorus primitivus, Tylenchorhynchus spp., z.B. Tylenchorhynchus annulatus, Tylenchulus spp., z.B. Tylenchulus semipenetrans, Xiphinema spp., z.B. Xiphinema index.

Weiterhin lässt sich aus dem Unterreich der Protozoa die Ordnung der Coccidia z.B. Eimeria spp. bekämpfen.

Die Verbindungen der Formel (I) können gegebenenfalls in bestimmten Konzentrationen bzw. Aufwandmengen auch als Herbizide, Safener, Wachstumsregulatoren oder Mittel zur Verbesserung der Pflanzeneigenschaften, als Mikrobizide oder Gametozide, beispielsweise als Fungizide, Antimykotika, Bakterizide, Virizide (einschließlich Mittel gegen Viroide) oder als Mittel gegen MLO (Mycoplasmalike-organism) und RLO (Rickettsia-like-organism) verwendet werden. Sie lassen sich gegebenenfalls auch als Zwischen- oder Vorprodukte für die Synthese weiterer Wirkstoffe einsetzen.

### Formulierungen

Die vorliegende Erfindung betrifft weiterhin Formulierungen und daraus bereitete Anwendungsformen als Schädlingsbekämpfungsmittel wie z. B. Drench-, Drip- und Spritzbrühen, umfassend mindestens eine Verbindung der Formel (I). Gegebenenfalls enthalten die Anwendungsformen weitere Schädlingsbekämpfungsmittel und/oder die Wirkung verbessernde Adjuvantien wie Penetrationsförderer, z. B. vegetative Öle wie beispielsweise Rapsöl, Sonnenblumenöl, Mineralöle wie beispielsweise Paraffinöle, Alkylester vegetativer Fettsäuren wie beispielsweise Rapsöl- oder Sojaölmethylester oder Alkanol-alkoxylate und/oder Spreitmittel wie beispielsweise Alkylsiloxane und/oder Salze z.B. organische oder anorganische Ammonium- oder Phosphoniumsalze wie beispielsweise Ammoniumsulfat oder Diammonium-hydrogenphosphat und/oder die Retention fördernde Mittel wie z. B. Dioctylsulfosuccinat oder Hydroxypropyl-guar Polymere und/oder Humectants wie z.B. Glycerin und/oder Dünger wie beispielsweise Ammonium-, Kalium- oder Phosphor-enthaltende Dünger.

Übliche Formulierungen sind beispielsweise wasserlösliche Flüssigkeiten (SL), Emulsionskonzentrate (EC), Emulsionen in Wasser (EW), Suspensionskonzentrate (SC, SE, FS, OD), in Wasser dispergierbare Granulate (WG), Granulate (GR) und Kapselkonzentrate (CS); diese und weitere mögliche Formuliertypen sind beispielsweise durch Crop Life International und in Pesticide Specifications, Manual on development and use of FAO and WHO specifications for pesticides, FAO Plant Production and Protection Papers - 173, prepared by the FAO/WHO Joint Meeting on Pesticide Specifications, 2004, ISBN: 9251048576 beschrieben. Gegebenenfalls enthalten die Formulierungen neben einem oder mehreren Verbindungen der Formel (I) weitere agrochemische Wirkstoffe.

Vorzugsweise handelt es sich um Formulierungen oder Anwendungsformen, welche Hilfsstoffe wie beispielsweise Streckmittel, Lösemittel, Spontanitätsförderer, Trägerstoffe, Emulgiermittel, Dispergiermittel, Frostschutzmittel, Biozide, Verdicker und/oder weitere Hilfsstoffe wie beispielsweise Adjuvantien enthalten. Ein Adjuvant in diesem Kontext ist eine Komponente, die die biologische Wirkung der Formulierung verbessert, ohne dass die Komponente selbst eine biologische Wirkung hat. Beispiele für Adjuvantien sind Mittel, die die Retention, das Spreitverhalten, das Anhaften an der Blattoberfläche oder die Penetration fördern.

Diese Formulierungen werden in bekannter Weise hergestellt, z.B. durch Vermischen der Verbindungen der Formel (I) mit Hilfsstoffen wie beispielsweise Streckmitteln, Lösemitteln und/oder festen Trägerstoffen und/oder weiteren Hilfsstoffen wie beispielsweise oberflächenaktive Stoffe. Die Herstellung der Formulierungen erfolgt entweder in geeigneten Anlagen oder auch vor oder während der Anwendung.

Als Hilfsstoffe können solche Stoffe Verwendung finden, die geeignet sind, der Formulierung der Verbindungen der Formel (I) oder den aus diesen Formulierungen bereiteten Anwendungsformen (wie z.B. gebrauchsfähigen Schädlingsbekämpfungsmitteln wie Spritzbrühen oder Saatgutbeizen) besondere Eigenschaften, wie bestimmte physikalische, technische und/oder biologische Eigenschaften zu verleihen.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (Poly-)Ether, der einfachen und substituierten Amine, Amide, Lactame (wie N-Alkylpyrrolidone) und Lactone, der Sulfone und Sulfoxide (wie Dimethylsulfoxid).

Im Falle der Benutzung von Wasser als Streckmittel können z.B. auch organische Lösemittel als Hilfslösemittel verwendet werden. Als flüssige Lösemittel kommen im Wesentlichen infrage: Aromaten wie Xylol, Toluol oder Alkylnaphthaline, chlorierte Aromaten oder chlorierte aliphatische Kohlenwasser-stoffe wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole wie Butanol oder Glykol sowie deren Ether und Ester, Ketone wie Aceton, Methylethylketon, Methylisobutylketon oder Cyclohexanon, stark polare Lösemittel wie Dimethylformamid und Dimethylsulfoxid sowie Wasser.

Grundsätzlich können alle geeigneten Lösemittel verwendet werden. Geeignete Lösemittel sind beispielsweise aromatische Kohlenwasserstoffe wie z.B. Xylol, Toluol oder Alkylnaphthaline, chlorierte aromatische oder aliphatische Kohlenwasserstoffe wie z.B. Chlorbenzol, Chlorethylen, oder Methylenchlorid, aliphatische Kohlenwasserstoffe wie z.B. Cyclohexan, Paraffine, Erdölfraktionen, mineralische und pflanzliche Öle, Alkohole wie z.B. Methanol, Ethanol, iso-Propanol, Butanol oder Glykol sowie deren Ether und Ester, Ketone wie z.B. Aceton, Methylethylketon, Methylisobutylketon oder Cyclohexanon, stark polare Lösemittel wie Dimethylsulfoxid sowie Wasser.

Grundsätzlich können alle geeigneten Trägerstoffe eingesetzt werden. Als Trägerstoffe kommen insbesondere infrage: z.B. Ammoniumsalze und natürliche Gesteinsmehle wie Kaoline, Tonerden, Talkum, Kreide, Quarz, Attapulgit, Montmorillonit oder Diatomeenerde und synthetische Gesteinsmehl, wie hochdisperse Kieselsäure, Aluminiumoxid und natürliche oder synthetische Silikate, Harze, Wachse und /oder feste Düngemittel. Mischungen solcher Trägerstoffe können ebenfalls verwendet werden. Als Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Papier, Kokosnussschalen, Maiskolben und Tabakstängel.

Auch verflüssigte gasförmige Streckmittel oder Lösemittel können eingesetzt werden. Insbesondere eignen sich solche Streckmittel oder Trägerstoffe, welche bei normaler Temperatur und unter Normaldruck gasförmig sind, z.B. Aerosol-Treibgase wie Halogenkohlenwasserstoffe sowie Butan, Propan, Stickstoff und Kohlendioxid.

Beispiele für Emulgier- und/oder Schaum erzeugende Mittel, Dispergiermittel oder Benetzungsmittel mit ionischen oder nicht-ionischen Eigenschaften oder Mischungen dieser oberflächenaktiven Stoffe sind Salze von Polyacrylsäure, Salze von Lignosulphonsäure, Salze von Phenolsulphonsäure oder Naphthalinsulphonsäure, Polykondensate von Ethylenoxid mit Fettalkoholen oder mit Fettsäuren oder mit Fettaminen, mit substituierten Phenolen (vorzugsweise Alkylphenole oder Arylphenole), Salze von Sulphobernsteinsäureestern, Taurinderivate (vorzugsweise Alkyltaurate), Phosphorsäureester von polyethoxylierten Alkoholen oder Phenole, Fettsäureester von Polyolen und Derivate der Verbindungen enthaltend Sulphate, Sulphonate und Phosphate, z.B. Alkylarylpolyglycolether, Alkylsulfonate, Alkylsulfate, Arylsulfonate, Eiweißhydrolysate, Lignin-Sulfitablaugen und Methylcellulose. Die Anwesenheit einer oberflächenaktiven Substanz ist vorteilhaft, wenn eine der Verbindungen der Formel (I) und/oder einer der inerten Trägerstoffe nicht in Wasser löslich ist und wenn die Anwendung in Wasser erfolgt.

Als weitere Hilfsstoffe können in den Formulierungen und den daraus abgeleiteten Anwendungsformen Farbstoffe wie anorganische Pigmente, z.B. Eisenoxid, Titanoxid, Ferrocyanblau und organische Farbstoffe wie Alizarin-, Azo- und Metallphthalocyaninfarbstoffe und Nähr- und Spurennährstoffe wie Salze von Eisen, Mangan, Bor, Kupfer, Kobalt, Molybdän und Zink vorhanden sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel. Weiterhin enthalten sein können schaumerzeugende Mittel oder Entschäumer.

Ferner können die Formulierungen und daraus abgeleiteten Anwendungsformen als zusätzliche Hilfsstoffe auch Haftmittel wie Carboxymethylcellulose, natürliche und synthetische pulverige, körnige oder latexförmige Polymere enthalten wie Gummiarabikum, Polyvinylalkohol, Polyvinylacetat sowie natürliche Phospholipide wie Kephaline und Lecithine und synthetische Phospholipide. Weitere Hilfsstoffe können mineralische und vegetabile Öle sein.

Gegebenenfalls können noch weitere Hilfsstoffe in den Formulierungen und den daraus abgeleiteten Anwendungsformen enthalten sein. Solche Zusatzstoffe sind beispielsweise Duftstoffe, schützende Kolloide, Bindemittel, Klebstoffe, Verdicker, thixotrope Stoffe, Penetrationsförderer, Retentionsförderer, Stabilisatoren, Sequestiermittel, Komplexbildner, Humectans, Spreitmittel. Im Allgemeinen können die Verbindungen der Formel (I) mit jedem festen oder flüssigen Zusatzstoff, welches für Formulierungszwecke gewöhnlich verwendet wird, kombiniert werden.

Als Retentionsförderer kommen alle diejenigen Substanzen in Betracht, die die dynamische Oberflächenspannung verringern wie beispielsweise Dioctylsulfosuccinat oder die die Visko-Elastizität erhöhen wie beispielsweise Hydroxypropyl-guar Polymere.

Als Penetrationsförderer kommen im vorliegenden Zusammenhang alle diejenigen Substanzen in Betracht, die üblicherweise eingesetzt werden, um das Eindringen von agrochemischen Wirkstoffen in Pflanzen zu verbessern. Penetrationsförderer werden in diesem Zusammenhang dadurch definiert, dass sie aus der (in der Regel wässerigen) Applikationsbrühe und/oder aus dem Spritzbelag in die Kutikula der Pflanze eindringen und dadurch die Stoffbeweglichkeit (Mobilität) der Wirkstoffe in der Kutikula erhöhen können. Die in der Literatur (Baur et al., 1997, Pesticide Science 51, 131-152) beschriebene Methode kann zur Bestimmung dieser Eigenschaft eingesetzt werden. Beispielhaft werden genannt Alkoholalkoxylate wie beispielsweise Kokosfettethoxylat (10) oder Isotridecylethoxylat (12), Fettsäureester wie beispielsweise Rapsöl- oder Sojaölmethylester, Fettamine Alkoxylate wie beispielsweise Tallowamine-ethoxylat (15) oder Ammonium- und/oder Phosphonium-Salze wie beispielsweise Ammoniumsulfat oder Diammonium-hydrogenphosphat.

Die Formulierungen enthalten bevorzugt zwischen 0,00000001 und 98 Gew.-% der Verbindung der Formel (I), besonders bevorzugt zwischen 0,01 und 95 Gew.-% der Verbindung der Formel (I), ganz besonders bevorzugt zwischen 0,5 und 90 Gew.-% der Verbindung der Formel (I), bezogen auf das Gewicht der Formulierung.

Der Gehalt an der Verbindung der Formel (I) in den aus den Formulierungen bereiteten Anwendungsformen (insbesondere Schädlingsbekämpfungsmittel) kann in weiten Bereichen variieren. Die Konzentration der Verbindung der Formel (I) in den Anwendungsformen kann üblicherweise zwischen 0,00000001 und 95 Gew.-% der Verbindung der Formel (I), vorzugsweise zwischen 0,00001 und 1 Gew.-%, bezogen auf das Gewicht der Anwendungsform, liegen. Die Anwendung geschieht in einer den Anwendungsformen angepaßten üblichen Weise.

### Mischungen

Die Verbindungen der Formel (I) können auch in Mischung mit einem oder mehreren geeigneten Fungiziden, Bakteriziden, Akariziden, Molluskiziden, Nematiziden, Insektiziden, Mikrobiologika, Nützlingen, Herbizide, Düngemitteln, Vogelrepellentien, Phytotonics, Sterilantien, Safenern, Semiochemicals und/oder Pflanzenwachstumsregulatoren verwendet werden, um so z.B. das Wirkungsspektrum zu verbreitern, die Wirkdauer zu verlängern, die Wirkgeschwindigkeit zu steigern, Repellenz zu verhindern oder Resistenzentwicklungen vorzubeugen. Desweiteren können solche Wirkstoffkombinationen das Pflanzenwachstum und/oder die Toleranz gegenüber abiotischen Faktoren wie z. B. hohen oder niedrigen Temperaturen, gegen Trockenheit - oder gegen erhöhten Wasser- bzw. Bodensalzgehalt verbessern. Auch lässt sich das Blüh- und Fruchtverhalten verbessern, die Keimfähigkeit und Bewurzelung optimieren, die Ernte erleichtern und Ernteerträge steigern, die Reife beeinflussen, die Qualität und/oder den Ernährungswert der Ernteprodukte steigern, die Lagerfähigkeit verlängern und/oder die Bearbeitbarkeit der Ernteprodukte verbessern.

Weiterhin können die Verbindungen der Formel (I) in Mischung mit weiteren Wirkstoffen oder Semiochemicals, wie Lockstoffen und/oder Vogelrepellentien und/oder Pflanzenaktivatoren und/oder Wachstumsregulatoren und/oder Düngemitteln vorliegen. Gleichfalls können die Verbindungen der Formel (I) in Mischungen mit Mitteln zur Verbesserung der Pflanzeneigenschaften wie zum Beispiel Wuchs, Ertrag und Qualität des Erntegutes eingesetzt werden.

In einer besonderen erfindungsgemäßen Ausführungsform liegen die Verbindungen der Formel (I) in Formulierungen bzw. in den aus diesen Formulierungen bereiteten Anwendungsformen in Mischung mit weiteren Verbindungen vor, vorzugsweise solchen wie nachstehend beschrieben.

Wenn eine der im Folgenden genannten Verbindungen in verschiedenen tautomeren Formen vorkommen kann sind auch diese Formen mit umfasst, auch wenn sie sie nicht in jedem Fall explizit genannt wurden.

### Insektizide / Akarizide / Nematizide

Die hier mit ihrem "common name" genannten Wirkstoffe sind bekannt und beispielsweise im Pestizidhandbuch ("The Pesticide Manual" 16th Ed., British Crop Protection Council 2012) beschrieben oder im Internet recherchierbar (z.B. http://www.alanwood.net/pesticides).
(1) Acetylcholinesterase (AChE) Inhibitoren, wie beispielsweise Carbamate, z.B. Alanycarb, Aldicarb, Bendiocarb, Benfuracarb, Butocarboxim, Butoxycarboxim, Carbaryl, Carbofuran, Carbosulfan, Ethiofencarb, Fenobucarb, Formetanate, Furathiocarb, Isoprocarb, Methiocarb, Methomyl, Metolcarb, Oxamyl, Pirimicarb, Propoxur, Thiodicarb, Thiofanox, Triazamate, Trimethacarb, XMC und Xylylcarb oder organophosphate, z.B. Acephate, Azamethiphos, Azinphos-ethyl, Azinphos-methyl, Cadusafos, Chlorethoxyfos, Chlorfenvinphos, Chlormephos, Chlorpyrifos, Chlorpyrifos-methyl, Coumaphos, Cyanophos, Demeton-S-methyl, Diazinon, Dichlorvos/DDVP, Dicrotophos, Dimethoate, Dimethylvinphos, Disulfoton, EPN, Ethion, Ethoprophos, Famphur, Fenamiphos, Fenitrothion, Fenthion, Fosthiazate, Heptenophos, Imicyafos, Isofenphos, Isopropyl O-(methoxyaminothio-phosphoryl) salicylat, Isoxathion, Malathion, Mecarbam, Methamidophos, Methidathion, Mevinphos, Monocrotophos, Naled, Omethoate, Oxydemeton-methyl, Parathion, Parathion-methyl, Phenthoate, Phorate, Phosalone, Phosmet, Phosphamidon, Phoxim, Pirimiphos-methyl, Profenofos, Propetamphos, Prothiofos, Pyraclofos, Pyridaphenthion, Quinalphos, Sulfotep, Tebupirimfos, Temephos, Terbufos, Tetrachlorvinphos, Thiometon, Triazophos, Triclorfon und Vamidothion.
(2) GABA-gesteuerte Chlorid-Kanal-Antagonisten, wie beispielsweise Cyclodien-organochlorine, z.B. Chlordane und Endosulfan oder Phenylpyrazole (Fiprole), z.B. Ethiprole und Fipronil.
(3) Natrium-Kanal-Modulatoren / Spannungsabhängige Natrium-Kanal-Blocker, wie beispielsweise Pyrethroide, z.B. Acrinathrin, Allethrin, d-cis-trans Allethrin, d-trans Allethrin, Bifenthrin, Bioallethrin, Bioallethrin S-cyclopentenyl Isomer, Bioresmethrin, Cycloprothrin, Cyfluthrin, beta-Cyfluthrin, Cyhalothrin, lambda-Cyhalothrin, gamma-Cyhalothrin, Cypermethrin, alpha-Cypermethrin, beta-Cypermethrin, theta-Cypermethrin, zeta-Cypermethrin, Cyphenothrin [(1R)-trans-Isomere], Deltamethrin, Empenthrin [(EZ)-(1R)-Isomere), Esfenvalerate, Etofenprox, Fenpropathrin, Fenvalerate, Flucythrinate, Flumethrin, tau-Fluvalinate, Halfenprox, Imiprothrin, Kadethrin, Permethrin, Phenothrin [(1R)-trans-Isomer), Prallethrin, Pyrethrine (pyrethrum), Resmethrin, Silafluofen, Tefluthrin, Tetramethrin, Tetramethrin [(1R)- Isomere)], Tralomethrin und Transfluthrin oder DDT oder Methoxychlor.
(4) Nikotinerge Acetylcholin-Rezeptor (nAChR) Agonisten, wie beispielsweise Neonikotinoide, z.B. Acetamiprid, Clothianidin, Dinotefuran, Imidacloprid, Nitenpyram, Thiacloprid und Thiamethoxam oder Nikotin oder Sulfoxaflor oder Flupyradifurone.
(5) Nikotinerge Acetylcholin-Rezeptor (nAChR) allosterische Aktivatoren, wie beispielsweise Spinosine, z.B. Spinetoram und Spinosad.
(6) Chlorid-Kanal-Aktivatoren, wie beispielsweise Avermectine/Milbemycine, z.B. Abamectin, Emamectin-benzoat, Lepimectin und Milbemectin.
(7) Juvenilhormon-Imitatoren, wie beispielsweise Juvenilhormon-Analoge, z.B. Hydroprene, Kinoprene und Methoprene oder Fenoxycarb oder Pyriproxyfen.
(8) Wirkstoffe mit unbekannten oder nicht spezifischen Wirkmechanismen, wie beispielsweise Alkylhalide, z.B. Methylbromid und andere Alkylhalide; oderChloropicrin oder Sulfurylfluorid oder Borax oder Brechweinstein.
(9) Selektive Fraßhemmer, z.B. Pymetrozine oder Flonicamid.
(10) Milbenwachstumsinhibitoren, z.B. Clofentezine, Hexythiazox und Diflovidazin oder Etoxazole.
(11) Mikrobielle Disruptoren der Insektendarmmembran, z.B. Bacillus thuringiensis Subspezies israelensis, Bacillus sphaericus, Bacillus thuringiensis Subspezies aizawai, Bacillus thuringiensis Subspezies kurstaki, Bacillus thuringiensis Subspezies tenebrionis und BT Pflanzenproteine: Cry1Ab, Cry1Ac, Cry1Fa, Cry2Ab, mCry3A, Cry3Ab, Cry3Bb, Cry34/35Ab1.
(12) Inhibitoren der oxidativen Phosphorylierung, ATP-Disruptoren, wie beispielsweise Diafenthiuron oder Organozinnverbindungen, z.B. Azocyclotin, Cyhexatin und Fenbutatin-oxid oder Propargite oder Tetradifon.
(13) Entkoppler der oxidativen Phoshorylierung durch Unterbrechung des H-Protongradienten, wie beispielsweise Chlorfenapyr, DNOC und Sulfluramid.
(14) Nikotinerge Acetylcholin-Rezeptor-Antagonisten, wie beispielsweise Bensultap, Cartaphydrochlorid, Thiocyclam und Thiosultap-Natrium.
(15) Inhibitoren der Chitinbiosynthese, Typ 0, wie beispielsweise Bistrifluron, Chlorfluazuron, Diflubenzuron, Flucycloxuron, Flufenoxuron, Hexaflumuron, Lufenuron, Novaluron, Noviflumuron, Teflubenzuron und Triflumuron.
(16) Inhibitoren der Chitinbiosynthese, Typ 1, wie beispielsweise Buprofezin.
(17) Häutungshemmer (insbesondere bei Dipteren, d.h.Zweiflüglern), wie beispielsweise Cyromazine.
(18) Ecdyson-Rezeptor Agonisten, wie beispielsweise Chromafenozide, Halofenozide, Methoxyfenozide und Tebufenozide.
(19) Oktopaminerge Agonisten, wie beispielsweise Amitraz.
(20) Komplex-III-Elektronentransportinhibitoren, wie beispielsweise Hydramethylnon oder Acequinocyl oder Fluacrypyrim.
(21) Komplex-I-Elektronentransportinhibitoren, beispielsweise METI-Akarizide, z.B. Fenazaquin, Fenpyroximate, Pyrimidifen, Pyridaben, Tebufenpyrad und Tolfenpyrad oder Rotenone (Derris).
(22) Spannungsabhängige Natriumkanal-Blocker, z.B. Indoxacarb oder Metaflumizone.
(23) Inhibitoren der Acetyl-CoA-Carboxylase, wie beispielsweise Tetron- und Tetramsäurederivate, z.B. Spirodiclofen, Spiromesifen und Spirotetramat.
(24) Komplex-IV-Elektronentransportinhibitoren, wie beispielsweise Phosphine, z.B. Aluminiumphosphid, Calciumphosphid, Phosphin und Zinkphosphid oder Cyanid.
(25) Komplex-II-Elektronentransportinhibitoren, wie beispielsweise Cyenopyrafen und Cyflumetofen.
(28) Ryanodinrezeptor-Effektoren, wie beispielsweise Diamide, z.B. Chlorantraniliprole, Cyantraniliprole und Flubendiamide.

Weitere Wirkstoffe mit unbekanntem oder nicht eindeutigem Wirkmechanismus, wie beispielsweise Afidopyropen, Afoxolaner, Azadirachtin, Benclothiaz, Benzoximate, Bifenazate, Bromopropylate, Chinomethionat, Cryolite, Cyclaniliprole, Cycloxaprid, Cyhalodiamide Dicloromezotiaz, Dicofol, Diflovidazin, Flometoquin, Fluensulfone, Flufenerim, Flufenoxystrobin, Flufiprole, Fluhexafon, Fluopyram, Fluralaner, Fufenozide, Guadipyr, Heptafluthrin, Imidaclothiz, Iprodione, Meperfluthrin, Paichongding, Pyflubumide, Pyridalyl, Pyrifluquinazon, Pyriminostrobin, Tetramethylfluthrin, Tetraniliprole, Tetrachlorantraniliprole, Tioxazafen, Triflumezopyrim und Iodmethan; desweiteren Präparate auf Basis von Bacillus firmus (I-1582, BioNeem, Votivo), sowie folgende bekannte wirksame Verbindungen: 1-{2-Fluor-4-methyl-5-[(2,2,2-trifluorethyl)sulfinyl]phenyl}-3-(trifluormethyl)-1H-1,2,4-triazol-5-amin (bekannt aus WO2006/043635), {1'-[(2E)-3-(4-Chlorphenyl)prop-2-en-1-yl]-5-fluorspiro[indol-3,4'-piperidin]-1(2H)-yl}(2-chlorpyridin-4-yl)methanon (bekannt aus WO2003/106457), 2-Chlor-N-[2-{1-[(2E)-3-(4-chlorphenyl)prop-2-en-1-yl]piperidin-4-yl}-4-(trifluormethyl)phenyl]isonicotinamid (bekannt aus WO2006/003494), 3-(2,5-Dimethylphenyl)-4-hydroxy-8-methoxy-1,8-diazaspiro[4.5]dec-3-en-2-on (bekannt aus WO2009/049851), 3-(2,5-Dimethylphenyl)-8-methoxy-2-oxo-1,8-diazaspiro[4.5]dec-3-en-4-yl-ethylcarbonat (bekannt aus WO2009/049851), 4-(But-2-in-1-yloxy)-6-(3,5-dimethylpiperidin-1-yl)-5-fluorpyrimidin (bekannt aus WO2004/099160), 4-(But-2-in-1-yloxy)-6-(3-chlorphenyl)pyrimidin (bekannt aus WO2003/076415), PF1364 (CAS-Reg.No. 1204776-60-2), Methyl-2-[2-({[3-brom-1-(3-chlorpyridin-2-yl)-1H-pyrazol-5-yl]carbonyl}amino)-5-chlor-3-methylbenzoyl]-2-methylhydrazincarboxylat (bekannt aus WO2005/085216), Methyl-2-[2-({[3-brom-1-(3-chlorpyridin-2-yl)-1H-pyrazol-5-yl]carbonyl}amino)-5-cyan-3-methylbenzoyl]-2-ethylhydrazincarboxylat (bekannt aus WO2005/085216), Methyl-2-[2-({[3-brom-1-(3-chlorpyridin-2-yl)-1H-pyrazol-5-yl]carbonyl}amino)-5-cyan-3-methylbenzoyl]-2-methylhydrazincarboxylat (bekannt aus WO2005/085216), Methyl-2-[3,5-dibrom-2-({[3-brom-1-(3-chlorpyridin-2-yl)-1H-pyrazol-5-yl]carbonyl}amino)benzoyl]-2-ethylhydrazincarboxylat (bekannt aus WO2005/085216), N-[2-(5-Amino-1,3,4-thiadiazol-2-yl)-4-chlor-6-methylphenyl]-3-brom-1-(3-chlorpyridin-2-yl)-1H-pyrazol-5-carboxamid (bekannt aus CN102057925), 8-Chlor-N-[(2-chlor-5-methoxyphenyl)sulfonyl]-6-(trifluormethyl)imidazo[1,2-a]pyridin-2-carboxamid (bekannt aus WO2010/129500), 4-[5-(3,5-Dichlorphenyl)-5-(trifluormethyl)-4,5-dihydro-1,2-oxazol-3-yl]-2-methyl-N-(1-oxidothietan-3-yl)benzamid (bekannt aus WO2009/080250), N-[(2E)-1-[(6-Chlorpyridin-3-yl)methyl]pyridin-2(1H)-yliden]-2,2,2-trifluoracetamid (bekannt aus WO2012/029672), 1-[(2-Chlor-1,3-thiazol-5-yl)methyl]-4-oxo-3-phenyl-4H-pyrido[1,2-a]pyrimidin-1-ium-2-olat (bekannt aus WO2009/099929), 1-[(6-Chlorpyridin-3-yl)methyl]-4-oxo-3-phenyl-4H-pyrido[1,2-a]pyrimidin-1-ium-2-olat (bekannt aus WO2009/099929), 4-(3-{2,6-Dichlor-4-[(3,3-dichlorprop-2-en-1-yl)oxy]phenoxy}propoxy)-2-methoxy-6-(trifluormethyl)pyrimidin (bekannt aus CN101337940), N-[2-(tert-Butylcarbamoyl)-4-chlor-6-methylphenyl]-1-(3-chlorpyridin-2-yl)-3-(fluormethoxy)-1H-pyrazol-5-carboxamid (bekannt aus WO2008/134969, 3-[Benzoyl(methyl)amino]-N-[2-brom-4-[1,2,2,2-tetrafluor-1-(trifluormethyl)ethyl]-6-(trifluormethyl)phenyl]-2-fluor-benzamid (bekannt aus WO 2010018714 Butyl-[2-(2,4-dichlorphenyl)-3-oxo-4-oxaspiro[4.5]dec-1-en-1-yl]-carbonat (bekannt aus CN 102060818), 4-[5-(3,5-Dichlorphenyl)-5-(trifluormethyl)-4H-isoxazol-3-yl]-N-[(Z)-methoxyiminomethyl]-2-methyl-benzamid (bekannt aus WO2007/026965), (3E)-3-[1-[(6-Chlor-3-pyridyl)methyl]-2-pyridyliden]-1,1,1-trifluor-propan-2-on (bekannt aus WO2013/144213, N-(Methylsulfonyl)-6-[2-(pyridin-3-yl)-1,3-thiazol-5-yl]pyridin-2-carboxamid (bekannt aus WO2012/000896), N-[3-(Benzylcarbamoyl)-4-chlorphenyl]-1-methyl-3-(pentafluorethyl)-4-(trifluormethyl)-1H-pyrazol-5-carboxamid (bekannt aus WO2010/051926).

### Fungizide

Die hier mit ihrem "common name" spezifizierten Wirkstoffe sind bekannt, beispielsweise beschrieben im "Pesticide Manual" oder im Internet (beispielsweise: http://www.alanwood.net/pesticides).

Alle aufgeführten fungiziden Mischpartner der Klassen (1) bis (15) können optional Salze mit entsprechenden Basen oder Säuren bilden, sofern geeignete funktionelle Gruppen vorliegen. Außerdem sind für die aufgeführten fungiziden Mischpartner der Klassen (1) bis (15) auch tautomere Formen eingeschlossen, sofern Tautomerie möglich ist.
1) Inhibitoren der Ergosterolbiosynthese, zum Beispiel (1.01) Aldimorph, (1.02) Azaconazol, (1.03) Bitertanol, (1.04) Bromuconazol, (1.05) Cyproconazol, (1.06) Diclobutrazol, (1.07) Difenoconazol, (1.08) Diniconazol, (1.09) Diniconazol-M, (1.10) Dodemorph, (1.11) Dodemorphacetat, (1.12) Epoxiconazol, (1.13) Etaconazol, (1.14) Fenarimol, (1.15) Fenbuconazol, (1.16) Fenhexamid, (1.17) Fenpropidin, (1.18) Fenpropimorph, (1.19) Fluquinconazol, (1.20) Flurprimidol, (1.21) Flusilazol, (1.22) Flutriafol, (1.23) Furconazol, (1.24) Furconazol-cis, (1.25) Hexaconazol, (1.26) Imazalil, (1.27) Imazalilsulfat, (1.28) Imibenconazol, (1.29) Ipconazol, (1.30) Metconazol, (1.31) Myclobutanil, (1.32) Naftifin, (1.33) Nuarimol, (1.34) Oxpoconazol, (1.35) Paclobutrazol, (1.36) Pefurazoat, (1.37) Penconazol, (1.38) Piperalin, (1.39) Prochloraz, (1.40) Propiconazol, (1.41) Prothioconazol, (1.42) Pyributicarb, (1.43) Pyrifenox, (1.44) Quinconazol, (1.45) Simeconazol, (1.46) Spiroxamin, (1.47) Tebuconazol, (1.48) Terbinafin, (1.49) Tetraconazol, (1.50) Triadimefon, (1.51) Triadimenol, (1.52) Tridemorph, (1.53) Triflumizol, (1.54) Triforin, (1.55) Triticonazol, (1.56) Uniconazol, (1.57) Uniconazol-p, (1.58) Viniconazol, (1.59) Voriconazol, (1.60) 1-(4-Chlorphenyl)-2-(1H-1,2,4-triazol-1-yl)cycloheptanol, (1.61) 1-(2,2-Dimethyl-2,3-dihydro-1H-inden-1-yl)-1H-imidazol-5-carbonsäure¬methylester, (1.62) N'-{5-(Difluormethyl)-2-methyl-4-[3-(trimethylsilyl)propoxy]phenyl}-N-ethyl-N-methylimidoformamid, (1.63) N-Ethyl-N-methyl-N'-{2-methyl-5-(trifluormethyl)-4-[3-(trimethylsilyl)propoxy]phenyl}imidoformamid, (1.64) O-[1-(4-Methoxyphenoxy)-3,3-dimethylbutan-2-yl]-1H-imidazol-1-carbothioat, (1.65) Pyrisoxazol, (1.66) 2-{[3-(2-Chlorphenyl)-2-(2,4-difluorphenyl)oxiran-2-yl]methyl}-2,4-dihydro-3H-1,2,4-triazol-3-thion, (1.67) 1-{[3-(2-Chlorphenyl)-2-(2,4-difluorphenyl)oxiran-2-yl]methyl}-1H-1,2,4-triazol-5-ylthiocyanat, (1.68) 5-(Allylsulfanyl)-1-{[3-(2-chlorphenyl)-2-(2,4-difluorphenyl)oxiran-2-yl]methyl}-1H-1,2,4-triazol, (1.69) 2-[1-(2,4-Dichlorphenyl)-5-hydroxy-2,6,6-trimethylheptan-4-yl]-2,4-dihydro-3H-1,2,4-triazol-3-thion, (1.70) 2-{[rel(2R,3S)-3-(2-Chlorphenyl)-2-(2,4-difluorphenyl)oxiran-2-yl]methyl}-2,4-dihydro-3H-1,2,4-triazol-3-thion, (1.71) 2-{[rel(2R,3R)-3-(2-Chlorphenyl)-2-(2,4-difluorphenyl)-oxiran-2-yl]methyl}-2,4-dihydro-3H-1,2,4-triazol-3-thion, (1.72) 1-{[rel(2R,3S)-3-(2-Chlorphenyl)-2-(2,4-difluorphenyl)oxiran-2-yl]methyl}-1H-1,2,4-triazol-5-ylthiocyanat, (1.73) 1-{[rel(2R,3R)-3-(2-Chlorphenyl)-2-(2,4-difluorphenyl)oxiran-2-yl]methyl}-1H-1,2,4-triazol-5-ylthiocyanat, (1.74) 5-(Allylsulfanyl)-1-{[rel(2R,3S)-3-(2-chlorphenyl)-2-(2,4-difluorphenyl)oxiran-2-yl]methyl}-1H-1,2,4-triazol, (1.75) 5-(Allylsulfanyl)-1-{[rel(2R,3R)-3-(2-chlorphenyl)-2-(2,4-difluorphenyl)oxiran-2-yl]methyl}-1H-1,2,4-triazol, (1.76) 2-[(2S,4S,5S)-1-(2,4-Dichlorphenyl)-5-hydroxy-2,6,6-trimethylheptan-4-yl]-2,4-dihydro-3H-1,2,4-triazol-3-thion, (1.77) 2-[(2R,4S,5S)-1-(2,4-Dichlorphenyl)-5-hydroxy-2,6,6-trimethylheptan-4-yl]-2,4-dihydro-3H-1,2,4-triazol-3-thion, (1.78) 2-[(2R,4R,5R)-1-(2,4-Dichlorphenyl)-5-hydroxy-2,6,6-trimethylheptan-4-yl]-2,4-dihydro-3H-1,2,4-triazol-3-thion, (1.79) 2-[(2S,4R,5R)-1-(2,4-Dichlorphenyl)-5-hydroxy-2,6,6-trimethylheptan-4-yl]-2,4-dihydro-3H-1,2,4-triazol-3-thion, (1.80) 2-[(2S,4S,5R)-1-(2,4-Dichlorphenyl)-5-hydroxy-2,6,6-trimethylheptan-4-yl]-2,4-dihydro-3H-1,2,4-triazol-3-thion, (1.81) 2-[(2R,4S,5R)-1-(2,4-Dichlorphenyl)-5-hydroxy-2,6,6-trimethylheptan-4-yl]-2,4-dihydro-3H-1,2,4-triazol-3-thion, (1.82) 2-[(2R,4R,5S)-1-(2,4-Dichlorphenyl)-5-hydroxy-2,6,6-trimethylheptan-4-yl]-2,4-dihydro-3H-1,2,4-triazol-3-thion, (1.83) 2-[(2S,4R,SS)-1-(2,4-Dichlorphenyl)-5-hydroxy-2,6,6-trimethylheptan-4-yl]-2,4-dihydro-3H-1,2,4-triazol-3-thion, (1.84) 2-[4-(4-Chlorphenoxy)-2-(trifluormethyl)phenyl]-1-(1H-1,2,4-triazol-1-yl)propan-2-ol, (1.85) 2-[4-(4-Chlorphenoxy)-2-(trifluormethyl)phenyl]-1-(1H-1,2,4-triazol-1-yl)butan-2-ol, (1.86) 2-[4-(4-Chlorphenoxy)-2-(trifluormethyl)phenyl]-1-(1H-1,2,4-triazol-1-yl)pentan-2-ol, (1.87) 2-[2-Chlor-4-(4-chlorphenoxy)phenyl]-1-(1H-1,2,4-triazol-1-yl)butan-2-ol, (1.88) 2-[2-Chlor-4-(2,4-dichlorphenoxy)phenyl]-1-(1H-1,2,4-triazol-1-yl)propan-2-ol, (1.89) (2R)-2-(1-Chlorcyclopropyl)-4-[(1R)-2,2-dichlorcyclopropyl]-1-(1H-1,2,4-triazol-1-yl)butan-2-ol, (1.90) (2R)-2-(1-Chlorcyclopropyl)-4-[(1S)-2,2-dichlorcyclopropyl]-1-(1H-1,2,4-triazol-1-yl)butan-2-ol, (1.91) (2S)-2-(1-Chlorcyclopropyl)-4-[(1S)-2,2-dichlorcyclopropyl]-1-(1H-1,2,4-triazol-1-yl)butan-2-ol, (1.92) (2S)-2-(1-Chlorcyclopropyl)-4-[(1R)-2,2-dichlorcyclopropyl]-1-(1H-1,2,4-triazol-1-yl)butan-2-ol, (1.93) (1S,2R,5R)-5-(4-Chlorbenzyl)-2-(chlormethyl)-2-methyl-1-(1H-1,2,4-triazol-1-ylmethyl)cyclopentanol, (1.94) (1R,2S,5S)-5-(4-Chlorbenzyl)-2-(chlormethyl)-2-methyl-1-(1H-1,2,4-triazol-1-ylmethyl)cyclopentanol, (1.95) 5-(4-Chlorbenzyl)-2-(chlormethyl)-2-methyl-1-(1H-1,2,4-triazol-1-ylmethyl)cyclopentanol.
2) Inhibitoren der Atmungskette am Komplex I oder II, zum Beispiel (2.01) Bixafen, (2.02) Boscalid, (2.03) Carboxin, (2.04) Diflumetorim, (2.05) Fenfuram, (2.06) Fluopyram, (2.07) Flutolanil, (2.08) Fluxapyroxad, (2.09) Furametpyr, (2.10) Furmecyclox, (2.11) Isopyrazam (Mischung von syn-epimerem Racemat 1RS,4SR,9RS und anti-epimerem Racemat 1RS,4SR,9SR), (2.12) Isopyrazam (anti-epimeres Racemat 1RS,4SR,9SR), (2.13) Isopyrazam (anti-epimeres Enantiomer 1R,4S,9S), (2.14) Isopyrazam (anti-epimeres Enantiomer 1S,4R,9R), (2.15) Isopyrazam (syn-epimeres Racemat 1RS,4SR,9RS), (2.16) Isopyrazam (syn-epimeres Enantiomer 1R,4S,9R), (2.17) Isopyrazam (syn-epimeres Enantiomer 1S,4R,9S), (2.18) Mepronil, (2.19) Oxycarboxin, (2.20) Penflufen, (2.21) Penthiopyrad, (2.22) Sedaxan, (2.23) Thifluzamid, (2.24) 1-Methyl-N-[2-(1,1,2,2-tetrafluorethoxy)phenyl]-3-(trifluormethyl)-1H-pyrazol-4-carboxamid, (2.25) 3-(Difluormethyl)-1-methyl-N-[2-(1,1,2,2-tetrafluorethoxy)phenyl]-1H-pyrazol-4-carboxamid, (2.26) 3-(Difluormethyl)-N-[4-fluor-2-(1,1,2,3,3,3-hexafluorpropoxy)phenyl]-1-methyl-1H-pyrazol-4-carboxamid, (2.27) N-[1-(2,4-Dichlorphenyl)-1-methoxypropan-2-yl]-3-(difluormethyl)-1-methyl-1H-pyrazol-4-carboxamid, (2.28) 5,8-Difluor-N-[2-(2-fluor-4-{[4-(trifluormethyl)pyridin-2-yl]oxy}phenyl)ethyl]chinazolin-4-amin, (2.29) Benzovindiflupyr, (2.30) N-[(1S,4R)-9-(Dichlormethylen)-1,2,3,4-tetrahydro-1,4-methanonaphthalin-5-yl]-3-(difluormethyl)-1-methyl-1H-pyrazol-4-carboxamid, (2.31) N-[(1R,4S)-9-(Dichlormethylen)-1,2,3,4-tetrahydro-1,4-methanonaphthalin-5-yl]-3-(difluormethyl)-1-methyl-1H-pyrazol-4-carboxamid, (2.32) 3-(Difluormethyl)-1-methyl-N-(1,1,3-trimethyl-2,3-dihydro-1H-inden-4-yl)-1H-pyrazo1-4-carboxamid, (2.33) 1,3,5-Trimethyl-N-(1,1,3-trimethyl-2,3-dihydro-1H-inden-4-yl)-1H-pyrazol-4-carboxamid, (2.34) 1-Methyl-3-(trifluormethyl)-N-(1,1,3-trimethyl-2,3-dihydro-1H-inden-4-yl)-1H-pyrazol-4-carboxamid, (2.35) 1-Methyl-3-(trifluormethyl)-N-[(3R)-1,1,3-trimethyl-2,3-dihydro-1H-inden-4-yl]-1H-pyrazol-4-carboxamid, (2.36) 1-Methyl-3-(trifluormethyl)-N-[(3S)-1,1,3-trimethyl-2,3-dihydro-1H-inden-4-yl]-1H-pyrazol-4-carboxamid, (2.37) 3-(Difluormethyl)-1-methyl-N-[(3S)-1,1,3-trimethyl-2,3-dihydro-1H-inden-4-yl]-1H-pyrazol-4-carboxamid, (2.38) 3-(Difluormethyl)-1-methyl-N-[(3R)-1,1,3-trimethyl-2,3-dihydro-1H-inden-4-yl]-1H-pyrazol-4-carboxamid, (2.39) 1,3,5-Trimethyl-N-[(3R)-1,1,3-trimethyl-2,3-dihydro-1H-inden-4-yl]-1H-pyrazol-4-carboxamid, (2.40) 1,3,5-Trimethyl-N-[(3S)-1,1,3-trimethyl-2,3-dihydro-1H-inden-4-yl]-1H-pyrazol-4-carboxamid, (2.41) Benodanil, (2.42) 2-Chlor-N-(1,1,3-trimethyl-2,3-dihydro-1H-inden-4-yl)pyridin-3-carboxamid, (2.43) Isofetamid, (2.44) 1-Methyl-3-(trifluormethyl)-N-[2'-(trifluormethyl)biphenyl-2-yl]-1H-pyrazol-4-carboxamid, (2.45) N-(4'-Chlorbiphenyl-2-yl)-3-(difluormethyl)-1-methyl-1H-pyrazol-4-carboxamid, (2.46) N-(2',4'-Dichlorbiphenyl-2-yl)-3 - (difluormethyl)-1-methyl-1H-pyrazol-4-carboxamid, (2.47) 3-(Difluormethyl)-1-methyl-N-[4'-(trifluormethyl)biphenyl-2-yl]-1H-pyrazol-4-carboxamid, (2.48) N-(2',5'-Difluorbiphenyl-2-yl)-1 - methyl-3-(trifluormethyl)-1H-pyrazol-4-carboxamid, (2.49) 3-(Difluormethyl)-1-methyl-N-[4'-(prop-1-in-1-yl)biphenyl-2-yl]-1H-pyrazol-4-carboxamid, (2.50) 5-Fluor-1,3-dimethyl-N-[4'-(prop-1-in-1-yl)biphenyl-2-yl]-1H-pyrazol-4-carboxamid, (2.51) 2-Chlor-N-[4'-(prop-1-in-1-yl)biphenyl-2-yl]nicotinamid, (2.52) 3-(Difluormethyl)-N-[4'-(3,3-dimethylbut-1-in-1-yl)biphenyl-2-yl]-1-methyl-1H-pyrazol-4-carboxamid, (2.53) N-[4'-(3,3-Dimethylbut-1-in-1-yl)biphenyl-2-yl]-5-fluor-1,3-dimethyl-1H-pyrazol-4-carboxamid, (2.54) 3-(Difluormethyl)-N-(4'-ethinylbiphenyl-2-yl)-1-methyl-1H-pyrazol-4-carboxamid, (2.55) N-(4'-Ethinylbiphenyl-2-yl)-5-fluor-1,3-dimethyl-1H-pyrazol-4-carboxamid, (2.56) 2-Chlor-N-(4'-ethinylbiphenyl-2-yl)nicotinamid, (2.57) 2-Chlor-N-[4'-(3,3-dimethylbut-1-in-1-yl)biphenyl-2-yl]nicotinamid, (2.58) 4-(Difluormethyl)-2-methyl-N-[4'-(trifluormethyl)biphenyl-2-yl]-1,3-thiazol-5-carboxamid, (2.59) 5-Fluor-N-[4'-(3-hydroxy-3-methylbut-1-in-1-yl)biphenyl-2-yl]-1,3-dimethyl-1H-pyrazol-4-carboxamid, (2.60) 2-Chlor-N-[4'-(3-hydroxy-3-methylbut-1-in-1-yl)biphenyl-2-yl]nicotinamid, (2.61) 3-(Difluormethyl)-N-[4'-(3-methoxy-3-methylbut-1-in-1-yl)biphenyl-2-yl]-1-methyl-1H-pyrazol-4-carboxamid, (2.62) 5-Fluor-N-[4'-(3-methoxy-3-methylbut-1-in-1-yl)biphenyl-2-yl]-1,3-dimethyl-1H-pyrazol-4-carboxamid, (2.63) 2-Chlor-N-[4'-(3-methoxy-3-methylbut-1-in-1-yl)biphenyl-2-yl]nicotinamid, (2.64) 1,3-Dimethyl-N-(1,1,3-trimethyl-2,3-dihydro-1H-inden-4-yl)-1H-pyrazol-4-carboxamid, (2.65) 1,3-Dimethyl-N-[(3R)-1,1,3-trimethyl-2,3-dihydro-1H-inden-4-yl]-1H-pyrazol-4-carboxamid, (2.66) 1,3-Dimethyl-N-[(3S)-1,1,3-trimethyl-2,3-dihydro-1H-inden-4-yl]-1H-pyrazol-4-carboxamid, (2.67) 3-(Difluormethyl)-N-methoxy-1-methyl-N-[1-(2,4,6-trichlorphenyl)propan-2-yl]-1H-pyrazol-4-carboxamid, (2.68) 3-(Difluormethyl)-N-(7-fluor-1,1,3-trimethyl-2,3-dihydro-1H-inden-4-yl)-1-methyl-1H-pyrazol-4-carboxamid, (2.69) 3-(Difluormethyl)-N-[(3R)-7-fluor-1,1,3-trimethyl-2,3-dihydro-1H-inden-4-yl]-1-methyl-1H-pyrazol-4-carboxamid, (2.70) 3-(Difluormethyl)-N-[(3S)-7-fluor-1,1,3-trimethyl-2,3-dihydro-1H-inden-4-yl]-1-methyl-1H-pyrazol-4-carboxamid.
3) Inhibitoren der Atmungskette am Komplex III, zum Beispiel (3.01) Ametoctradin, (3.02) Amisulbrom, (3.03) Azoxystrobin, (3.04) Cyazofamid, (3.05) Coumethoxystrobin, (3.06) Coumoxystrobin, (3.07) Dimoxystrobin, (3.08) Enoxastrobin, (3.09) Famoxadon, (3.10) Fenamidon, (3.11) Flufenoxystrobin, (3.12) Fluoxastrobin, (3.13) Kresoxim-methyl, (3.14) Metominostrobin, (3.15) Orysastrobin, (3.16) Picoxystrobin, (3.17) Pyraclostrobin, (3.18) Pyrametostrobin, (3.19) Pyraoxystrobin, (3.20) Pyribencarb, (3.21) Triclopyricarb, (3.22) Trifloxystrobin, (3.23) (2E)-2-(2-{[6-(3-Chlor-2-methylphenoxy)-5-fluorpyrimidin-4-yl]oxy}phenyl)-2-(methoxyimino)-N-methyl¬acetamid, (3.24) (2E)-2-(Methoxyimino)-N-methyl-2-(2-{[({(1E)-1-[3-(trifluormethyl)phenyl]¬ethyliden}¬amino)oxy]methyl}phenyl)acetamid, (3.25) (2E)-2-(Methoxyimino)-N-methyl-2-{2-[(E)-({1-[3-(trifluormethyl)phenyl]ethoxy}imino)methyl]phenyl}acetamid, (3.26) (2E)-2-{2-[({[(1E)-1-(3-{[(E)-1-Fluor-2-phenylvinyl]oxy}phenyl)ethyliden]amino}oxy)methyl]phenyl}-2-(methoxyimino)-N-methyl-acetamid, (3.27) Fenaminostrobin, (3.28) 5-Methoxy-2-methyl-4-(2-{[({(1E)-1-[3-(trifluormethyl)phenyl]ethyliden}amino)oxy]methyl}phenyl)-2,4-dihydro-3H-1,2,4-triazol-3-on, (3.29) (2E)-2-{2-[({Cyclopropyl[(4-methoxyphenyl)imino]methyl}sulfanyl)methyl]phenyl}-3-methoxy-acrylsäuremethylester, (3.30) N-(3-Ethyl-3,5,5-trimethylcyclohexyl)-3-formamido-2-hydroxybenzamid, (3.31) 2-{2-[(2,5-Dimethylphenoxy)methyl]phenyl}-2-methoxy-N-methylacetamid, (3.32) 2-{2-[(2,5-Dimethylphenoxy)methyl]phenyl}-2-methoxy-N-methylacetamid, (3.33) (2E,3Z)-5-{[1-(4-Chlorphenyl)-1H-pyrazol-3-yl]oxy}-2-(methoxyimino)-N,3-dimethylpent-3-enamid.
4) Inhibitoren der Mitose und Zellteilung, zum Beispiel (4.01) Benomyl, (4.02) Carbendazim, (4.03) Chlorfenazol, (4.04) Diethofencarb, (4.05) Ethaboxam, (4.06) Fluopicolid, (4.07) Fuberidazol, (4.08) Pencycuron, (4.09) Thiabendazol, (4.10) Thiophanat-methyl, (4.11) Thiophanat, (4.12) Zoxamid, (4.13) 5-Chlor-7-(4-methylpiperidin-1-yl)-6-(2,4,6-trifluorphenyl)[1,2,4]triazolo[1,5-a]pyrimidin, (4.14) 3-Chlor-5-(6-chlorpyridin-3-yl)-6-methyl-4-(2,4,6-trifluorphenyl)pyridazin.
5) Verbindungen, die dazu fähig sind, an mehreren Stellen anzugreifen ("Multisite Action"), zum Beispiel (5.01) Bordeaux-Mischung, (5.02) Captafol, (5.03) Captan, (5.04) Chlorothalonil, (5.05) Kupferhydroxid, (5.06) Kupfernaphthenat, (5.07) Kupferoxid, (5.08) Kupferoxychlorid, (5.09) Kupfer(2+)-sulfat, (5.10) Dichlofluanid, (5.11) Dithianon, (5.12) Dodin, (5.13) Dodin freie Base, (5.14) Ferbam, (5.15) Fluorofolpet, (5.16) Folpet, (5.17) Guazatin, (5.18) Guazatinacetat, (5.19) Iminoctadin, (5.20) Iminoctadinalbesilat, (5.21) Iminoctadintriacetat, (5.22) Mancopper, (5.23) Mancozeb, (5.24) Maneb, (5.25) Metiram, (5.26) Metiram-Zink, (5.27) Oxin-Kupfer, (5.28) Propamidin, (5.29) Propineb, (5.30) Schwefel und Schwefelzubereitungen einschließlich Calciumpolysulfid, (5.31) Thiram, (5.32) Tolylfluanid, (5.33) Zineb, (5.34) Ziram, (5.35) Anilazin.
6) Verbindungen, die dazu fähig sind, eine Abwehrreaktion des Wirtes zu induzieren, zum Beispiel (6.01) Acibenzolar-S-methyl, (6.02) Isotianil, (6.03) Probenazol, (6.04) Tiadinil, (6.05) Laminarin.
7) Inhibitoren der Aminosäure- und/oder Proteinbiosynthese, zum Beispiel (7.01) Andoprim, (7.02) Blasticidin-S, (7.03) Cyprodinil, (7.04) Kasugamycin, (7.05) Kasugamycinhydrochlorid-hydrat, (7.06) Mepanipyrim, (7.07) Pyrimethanil, (7.08) 3-(5-Fluor-3,3,4,4-tetramethyl-3,4-dihydroisochinolin-1-yl)chinolin, (7.09) Oxytetracyclin, (7.10) Streptomycin.
8) Inhibitoren der ATP-Produktion, zum Beispiel (8.01) Fentinacetat, (8.02) Fentinchlorid, (8.03) Fentinhydroxid, (8.04) Silthiofam.
9) Inhibitoren der Zellwandsynthese, zum Beispiel (9.01) Benthiavalicarb, (9.02) Dimethomorph, (9.03) Flumorph, (9.04) Iprovalicarb, (9.05) Mandipropamid, (9.06) Polyoxine, (9.07) Polyoxorim, (9.08) Validamycin A, (9.09) Valifenalat, (9.10) Polyoxin B, (9.11) (2E)-3-(4-tert.-Butylphenyl)-3-(2-chlorpyridin-4-yl)-1-(morpholin-4-yl)prop-2-en-1-on, (9.12) (2Z)-3-(4-tert.-Butylphenyl)-3-(2-chlorpyridin-4-yl)-1-(morpholin-4-yl)prop-2-en-1-on.
10) Inhibitoren der Lipid- und Membransynthese, zum Beispiel (10.01) Biphenyl, (10.02) Chloroneb, (10.03) Dicloran, (10.04) Edifenphos, (10.05) Etridiazol, (10.06) Iodocarb, (10.07) Iprobenfos, (10.08) Isoprothiolan, (10.09) Propamocarb, (10.10) Propamocarbhydrochlorid, (10.11) Prothiocarb, (10.12) Pyrazophos, (10.13) Quintozen, (10.14) Tecnazen, (10.15) Tolclofos-methyl.
11) Inhibitoren der Melaninbiosynthese, zum Beispiel (11.01) Carpropamid, (11.02) Diclocymet, (11.03) Fenoxanil, (11.04) Phthalid, (11.05) Pyroquilon, (11.06) Tricyclazol, (11.07) 2,2,2-Trifluorethyl {3-methyl-1-[(4-methylbenzoyl)amino]butan-2-yl}carbamat.
12) Inhibitoren der Nukleinsäuresynthese, zum Beispiel (12.01) Benalaxyl, (12.02) Benalaxyl-M (Kiralaxyl), (12.03) Bupirimat, (12.04) Clozylacon, (12.05) Dimethirimol, (12.06) Ethirimol, (12.07) Furalaxyl, (12.08) Hymexazol, (12.09) Metalaxyl, (12.10) Metalaxyl-M (Mefenoxam), (12.11) Ofurace, (12.12) Oxadixyl, (12.13) Oxolinsäure, (12.14) Octhilinon.
13) Inhibitoren der Signalvermittlung, zum Beispiel (13.01) Chlozolinat, (13.02) Fenpiclonil, (13.03) Fludioxonil, (13.04) Iprodion, (13.05) Procymidon, (13.06) Quinoxyfen, (13.07) Vinclozolin, (13.08) Proquinazid.
14) Verbindungen, die als Entkoppler wirken können, zum Beispiel (14.01) Binapacryl, (14.02) Dinocap, (14.03) Ferimzon, (14.04) Fluazinam, (14.05) Meptyldinocap.
15) Weitere Verbindungen, zum Beispiel (15.001) Benthiazol, (15.002) Bethoxazin, (15.003) Capsimycin, (15.004) Carvon, (15.005) Chinomethionat, (15.006) Pyriofenon (Chlazafenon), (15.007) Cufraneb, (15.008) Cyflufenamid, (15.009) Cymoxanil, (15.010) Cyprosulfamid, (15.011) Dazomet, (15.012) Debacarb, (15.013) Dichlorophen, (15.014) Diclomezin, (15.015) Difenzoquat, (15.016) Difenzoquatmetilsulfat, (15.017) Diphenylamin, (15.018) Ecomat, (15.019) Fenpyrazamin, (15.020) Flumetover, (15.021) Fluoroimid, (15.022) Flusulfamid, (15.023) Flutianil, (15.024) Fosetyl-Aluminium, (15.025) Fosetyl-Calcium, (15.026) Fosetyl-Natrium, (15.027) Hexachlorbenzol, (15.028) Irumamycin, (15.029) Methasulfocarb, (15.030) Methylisothiocyanat, (15.031) Metrafenon, (15.032) Mildiomycin, (15.033) Natamycin, (15.034) Nickeldimethyldithiocarbamat, (15.035) Nitrothalisopropyl, (15.036) Oxamocarb, (15.037) Oxyfenthiin, (15.038) Pentachlorphenol und Salze, (15.039) Phenothrin, (15.040) phosphorige Säure und deren Salze, (15.041) Propamocarb-fosetylat, (15.042) Propanosin-Natrium, (15.043) Pyrimorph, (15.044) Pyrrolnitrin, (15.045) Tebufloquin, (15.046) Tecloftalam, (15.047) Tolnifanid, (15.048) Triazoxid, (15.049) Trichlamid, (15.050) Zarilamid, (15.051) 2-Methylpropansäure-(3S,6S,7R,8R)-8-benzyl-3-[({3-[(isobutyryloxy)methoxy]-4-methoxypyridin-2-yl}carbonyl)amino]-6-methyl-4,9-dioxo-1,5-dioxonan-7-ylester, (15.052) 1-(4-{4-[(5R)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, (15.053) 1-(4-{4-[(5S)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, (15.054) Oxathiapiprolin, (15.055) 1H-Imidazol-1-carbonsäure-1-(4-methoxyphenoxy)-3,3-dimethylbutan-2-ylester, (15.056) 2,3,5,6-Tetrachlor-4-(methylsulfonyl)pyridin, (15.057) 2,3-Dibutyl-6-chlorthieno[2,3-d]pyrimidin-4(3H)-on, (15.058) 2,6-Dimethyl-1H,5H-[1,4]dithiino[2,3-c:5,6-c']dipyrrol-1,3,5,7(2H,6H)-tetron, (15.059) 2-[5-Methyl-3-(trifluormethyl)-1H-pyrazol-1-yl]-1-(4-{4-[(5R)-5-phenyl-4,5-dihydro-1,2-oxazol-3-yl]-1,3-thiazol-2-yl}piperidin-1-yl)ethanon, (15.060) 2-[5-Methyl-3-(trifluormethyl)-1H-pyrazol-1-yl]-1-(4-{4-[(5S)-5-phenyl-4,5-dihydro-1,2-oxazol-3-yl]-1,3-thiazol-2-yl}piperidin-1-yl)ethanon, (15.061) 2-[5-Methyl-3-(trifluormethyl)-1H-pyrazol-1-yl]-1-{4-[4-(5-phenyl-4,5-dihydro-1,2-oxazol-3-yl)-1,3-thiazol-2-yl]piperidin-1-yl}ethanon, (15.062) 2-Butoxy-6-iod-3-propyl-4H-chromen-4-on, (15.063) 2-Chlor-5-[2-chlor-1-(2,6-difluor-4-methoxyphenyl)-4-methyl-1H-imidazol-5-yl]pyridin, (15.064) 2-Phenylphenol und Salze, (15.065) 3-(4,4,5-Trifluor-3,3-dimethyl-3,4-dihydroisochinolin-1-yl)chinolin, (15.066) 3,4,5-Trichlorpyridin-2,6-dicarbonsäurenitril, (15.067) 3-Chlor-5-(4-chlorphenyl)-4-(2,6-difluorphenyl)-6-methylpyridazin, (15.068) 4-(4-Chlorphenyl)-5-(2,6-difluorphenyl)-3,6-dimethylpyridazin, (15.069) 5-Amino-1,3,4-thiadiazol-2-thiol, (15.070) 5-Chlor-N'-phenyl-N'-(prop-2-in-1-yl)thiophen-2-sulfonohydrazid, (15.071) 5-Fluor-2-[(4-fluorbenzyl)oxy]pyrimidin-4-amin, (15.072) 5-Fluor-2-[(4-methylbenzyl)oxy]pyrimidin-4-amin, (15.073) 5-Methyl-6-octyl[1,2,4]triazolo[1,5-a]pyrimidin-7-amin, (15.074) (2Z)-3-Amino-2-cyano-3-phenylacrylsäureethylester, (15.075) N'-(4-{[3-(4-Chlorbenzyl)-1,2,4-thiadiazol-5-yl]oxy}-2,5-dimethylphenyl)-N-ethyl-N-methylimidoformamid, (15.076) N-(4-Chlorbenzyl)-3-[3-methoxy-4-(prop-2-in-1-yloxy)phenyl]propanamid, (15.077) N-[(4-Chlorphenyl)(cyano)methyl]-3-[3-methoxy-4-(prop-2-in-1-yloxy)phenyl]propanamid, (15.078) N-[(5-Brom-3-chlorpyridin-2-yl)methyl]-2,4-dichlornicotinamid, (15.079) N-[1-(5-Brom-3-chlorpyridin-2-yl)ethyl]-2,4-dichlornicotinamid, (15.080) N-[1-(5-Brom-3-chlorpyridin-2-yl)ethyl]-2-fluor-4-iodnicotinamid, (15.081) N-{(E)-[(Cyclopropylmethoxy)imino][6-(difluormethoxy)-2,3-difluorphenyl]methyl}-2-phenylacetamid, (15.082) N-{(Z)-[(Cyclopropylmethoxy)imino][6-(difluormethoxy)-2,3-difluorphenyl]methyl}-2-phenylacetamid, (15.083) N'-{4-[(3-tert.-Butyl-4-cyano-1,2-thiazol-5-yl)oxy]-2-chlor-5-methylphenyl}-N-ethyl-N-methylimidoformamid, (15.084) N-Methyl-2-(1-{[5-methyl-3-(trifluormethyl)-1H-pyrazol-1-yl]acetyl}piperidin-4-yl)-N-(1,2,3,4-tetrahydronaphthalin-1-yl)-1,3-thiazol-4-carboxamid, (15.085) N-Methyl-2-(1-{[5-methyl-3-(trifluormethyl)-1H-pyrazol-1-yl]acetyl}piperidin-4-yl)-N-[(1R)-1,2,3,4-tetrahydronaphthalin-1-yl]-1,3-thiazol-4-carboxamid, (15.086) N-Methyl-2-(1-{[5-methyl-3-(trifluormethyl)-1H-pyrazol-1-yl]acetyl}piperidin-4-yl)-N-[(1S)-1,2,3,4-tetrahydronaphthalin-1-yl]-1,3-thiazol-4-carboxamid, (15.087) {6-[({[(1-Methyl-1H-tetrazol-5-yl)(phenyl)methylen]amino}oxy)methyl]pyridin-2-yl}-carbamidsäurepentylester, (15.088) Phenazin-1 -carbonsäure, (15.089) Chinolin-8-ol, (15.090) Chinolin-8-olsulfat (2:1), (15.091) {6-[({[(1-Methyl-1H-tetrazol-5-yl)(phenyl)methylen]-amino}¬oxy)methyl]pyridin-2-yl}carbamidsäure-tert.-butylester, (15.092) (5-Brom-2-methoxy-4-methyl-pyridin-3-yl)(2,3,4-trimethoxy-6-methylphenyl)methanon, (15.093) N-[2-(4-{[3-(4-Chlorphenyl)prop-2-in-1-yl]oxy}-3-methoxyphenyl)ethyl]-N2-(methylsulfonyl)valinamid, (15.094) 4-Oxo-4-[(2-phenylethyl)amino]butansäure, (15.095) {6-[({[(Z)-(l-Methyl-1H-tetrazol-5-yl)(phenyl)methylen]¬amino}oxy)methyl]pyridin-2-yl}carbamidsäurebut-3-in-1-ylester, (15.096) 4-Amino-5-fluor¬pyrimidin-2-ol (tautomere Form: 4-Amino-5-fluorpyrimidin-2(1H)-on), (15.097) 3,4,5-Trihydroxybenzoesäurepropylester, (15.098) [3-(4-Chlor-2-fluorphenyl)-5-(2,4-difluorphenyl)-1,2-oxazol-4-yl](pyridin-3-yl)methanol, (15.099) (S)-[3-(4-Chlor-2-fluorphenyl)-5-(2,4-difluorphenyl)-1,2-oxazol-4-yl](pyridin-3-yl)methanol, (15.100) (R)-[3-(4-Chlor-2-fluorphenyl)-5-(2,4-difluorphenyl)-1,2-oxazol-4-yl](pyridin-3-yl)methanol, (15.101) 2-Fluor-6-(trifluormethyl)-N-(1,1,3-trimethyl-2,3-dihydro-1H-inden-4-yl)benzamid, (15.102) 2-(6-Benzylpyridin-2-yl)chinazolin, (15.103) 2-[6-(3-Fluor-4-methoxyphenyl)-5-methylpyridin-2-yl]chinazolin, (15.104) 3-(4,4-Difluor-3,3-dimethyl-3,4-dihydroisochinolin-1-yl)chinolin, (15.105) Abscisinsäure, (15.106) N'-[5-Brom-6-(2,3-dihydro-1H-inden-2-yloxy)-2-methylpyridin-3-yl]-N-ethyl-N-methylimidoformamid, (15.107) N'-{5-Brom-6-[1-(3,5-difluorphenyl)ethoxy]-2-methylpyridin-3-yl}-N-ethyl-N-methylimidoformamid, (15.108) N'-{5-Brom-6-[(1R)-1-(3,5-difluorphenyl)ethoxy]-2-methylpyridin-3-yl}-N-ethyl-N-methylimidoformamid, (15.109) N'-{5-Brom-6-[(1S)-1-(3,5-difluorphenyl)ethoxy]-2-methylpyridin-3-yl}-N-ethyl-N-methylimidoformamid, (15.110) N'-{5-Brom-6-[(cis-4-isopropylcyclohexyl)oxy]-2-methylpyridin-3-yl}-N-ethyl-N-methylimidoformamid, (15.111) N'-{5-Brom-6-[(trans-4-isopropylcyclohexyl)oxy]-2-methylpyridin-3-yl}-N-ethyl-N-methylimidoformamid, (15.112) N-Cyclopropyl-3-(difluormethyl)-5-fluor-N-(2-isopropylbenzyl)-1-methyl-1H-pyrazol-4-carboxamid, (15.113) N-Cyclopropyl-N-(2-cyclopropylbenzyl)-3-(difluormethyl)-5-fluor-1-methyl-1H-pyrazol-4-carboxamid, (15.114) N-(2-tert.-Butylbenzyl)-N-cyclopropyl-3-(difluormethyl)-5-fluor-1-methyl-1H-pyrazol-4-carboxamid, (15.115) N-(5-Chlor-2-ethylbenzyl)-N-cyclopropyl-3-(difluormethyl)-5-fluor-1-methyl-1H-pyrazol-4-carboxamid, (15.116) N-(5-Chlor-2-isopropylbenzyl)-N-cyclopropyl-3-(difluormethyl)-5-fluor-1-methyl-1H-pyrazol-4-carboxamid, (15.117) N-Cyclopropyl-3-(difluormethyl)-N-(2-ethyl-5-fluorbenzyl)-5-fluor-1-methyl-1H-pyrazol-4-carboxamid, (15.118) N-Cyclopropyl-3-(difluormethyl)-5-fluor-N-(5-fluor-2-isopropylbenzyl)-1-methyl-1H-pyrazol-4-carboxamid, (15.119) N-Cyclopropyl-N-(2-cyclopropyl-5-fluorbenzyl)-3-(difluormethyl)-5-fluor-1-methyl-1H-pyrazol-4-carboxamid, (15.120) N-(2-Cyclopentyl-5-fluorbenzyl)-N-cyclopropyl-3-(difluormethyl)-5-fluor-1-methyl-1H-pyrazol-4-carboxamid, (15.121) N-Cyclopropyl-3-(difluormethyl)-5-fluor-N-(2-fluor-6-isopropylbenzyl)-1-methyl-1H-pyrazol-4-carboxamid, (15.122) N-Cyclopropyl-3-(difluormethyl)-N-(2-ethyl-5-methylbenzyl)-5-fluor-1-methyl-1H-pyrazol-4-carboxamid, (15.123) N-Cyclopropyl-3-(difluormethyl)-5-fluor-N-(2-isopropyl-5-methylbenzyl)-1-methyl-1H-pyrazol-4-carboxamid, (15.124) N-Cyclopropyl-N-(2-cyclopropyl-5-methylbenzyl)-3-(difluormethyl)-5-fluor-1-methyl-1H-pyrazol-4-carboxamid, (15.125) N-(2-tert.-Butyl-5-methylbenzyl)-N-cyclopropyl-3-(difluormethyl)-5-fluor-1-methyl-1H-pyrazol-4-carboxamid, (15.126) N-[5-Chlor-2-(trifluormethyl)benzyl]-N-cyclopropyl-3-(difluormethyl)-5-fluor-1-methyl-1H-pyrazol-4-carboxamid, (15.127) N-Cyclopropyl-3-(difluormethyl)-5-fluor-1-methyl-N-[5-methyl-2-(trifluormethyl)benzyl]-1H-pyrazol-4-carboxamid, (15.128) N-[2-Chlor-6-(trifluormethyl)benzyl]-N-cyclopropyl-3-(difluormethyl)-5-fluor-1-methyl-1H-pyrazol-4-carboxamid, (15.129) N-[3-Chlor-2-fluor-6-(trifluormethyl)benzyl]-N-cyclopropyl-3-(difluormethyl)-5-fluor-1-methyl-1H-pyrazol-4-carboxamid, (15.130) N-Cyclopropyl-3-(difluormethyl)-N-(2-ethyl-4,5-dimethylbenzyl)-5-fluor-1-methyl-1H-pyrazol-4-carboxamid, (15.131) N-Cyclopropyl-3-(difluormethyl)-5-fluor-N-(2-isopropylbenzyl)-1-methyl-1H-pyrazol-4-carbothioamid, (15.132) N'-(2,5-Dimethyl-4-phenoxyphenyl)-N-ethyl-N-methylimidoformamid, (15.133) N'-{4-[(4,5-Dichlor-1,3-thiazol-2-yl)oxy]-2,5-dimethylphenyl}-N-ethyl-N-methylimidoformamid, (15.134) N-(4-Chlor-2,6-difluorphenyl)-4-(2-chlor-4-fluorphenyl)-1,3-dimethyl-1H-pyrazol-5-amin, (15.135) 9-Fluor-2,2-dimethyl-5-(chinolin-3-yl)-2,3-dihydro-1,4-benzoxazepin, (15.136) 2-{2-Fluor-6-[(8-fluor-2-methylchinolin-3-yl)oxy]phenyl}propan-2-ol, 15.137 2-2-78-Difluor-2-methylchinolin-3-yl)oxy]-6-fluorhenyl}propan-2-ol, (15.138) 4-(2-Chlor-4-fluorphenyl)-N-(2-fluorphenyl)-1,3-dimethyl-1H-pyrazol-5-amin, (15.139) 4-(2-Chlor-4-fluorphenyl)-N-(2,6-difluorphenyl)-1,3-dimethyl-1H-pyrazol-5-amin, (15.140) 4-(2-Chlor-4-fluorphenyl)-N-(2-chlor-6-fluorphenyl)-1,3-dimethyl-1H-pyrazol-5-amin, (15.141) 4-(2-Brom-4-fluorphenyl)-N-(2-chlor-6-fluorphenyl)-1,3-dimethyl-1H-pyrazol-5-amin, (15.142) N-(2-Brom-6-fluorphenyl)-4-(2-chlor-4-fluorphenyl)-1,3-dimethyl-1H-pyrazol-5-amin, (15.143) 4-(2-Brom-4-fluorphenyl)-N-(2-bromphenyl)-1,3-dimethyl-1H-pyrazol-5-amin, (15.144) 4-(2-Brom-4-fluorphenyl)-N-(2-brom-6-fluorphenyl)-1,3-dimethyl-1H-pyrazol-5-amin, (15.145) 4-(2-Brom-4-fluorphenyl)-N-(2-chlorphenyl)-1,3-dimethyl-1H-pyrazol-5-amin, (15.146) N-(2-Bromphenyl)-4-(2-chlor-4-fluorphenyl)-1,3-dimethyl-1H-pyrazol-5-amin, (15.147) 4-(2-Chlor-4-fluorphenyl)-N-(2-chlorphenyl)-1,3-dimethyl-1H-pyrazol-5-amin, (15.148) 4-(2-Brom-4-fluorphenyl)-N-(2,6-difluorphenyl)-1,3-dimethyl-1H-pyrazol-5-amin, (15.149) 4-(2-Brom-4-fluorphenyl)-N-(2-fluorphenyl)-1,3-dimethyl-1H-pyrazol-5-amin, (15.150) N'-(4-{3-[(Difluormethyl)sulfanyl]phenoxy}-2,5-dimethylphenyl)-N-ethyl-N-methylimidoformamid, (15.151) N'-(2,5-Dimethyl-4-{3-[(1,1,2,2-tetrafluorethyl)sulfanyl]phenoxy}phenyl)-N-ethyl-N-methylimidoformamid, (15.152) N'-(2,5-Dimethyl-4-{3-[(2,2,2-trifluorethyl)sulfanyl]phenoxy}phenyl)-N-ethyl-N-methylimidoformamid, (15.153) N'-(2,5-Dimethyl-4-{3-[(2,2,3,3-tetrafluorpropyl)sulfanyl]phenoxy}phenyl)-N-ethyl-N-methylimidoformamid, (15.154) N'-(2,5-Dimethyl-4-{3-[(pentafluorethyl)sulfanyl]phenoxy}phenyl)-N-ethyl-N-methylimidoformamid, (15.155) N'-(4-{[3-(Difluormethoxy)phenyl]sulfanyl}-2,5-dimethylphenyl)-N-ethyl-N-methyl-imidoformamid, (15.156) N'-(2,5-Dimethyl-4-{[3-(1,1,2,2-tetrafluorethoxy)phenyl]sulfanyl}phenyl)-N-ethyl-N-methylimidoformamid, (15.157) N'-(2,5-Dimethyl-4-{[3-(2,2,2-trifluorethoxy)phenyl]sulfanyl}phenyl)-N-ethyl-N-methylimidoformamid, (15.158) N'-(2,5-Dimethyl-4-{[3-(2,2,3,3-tetrafluorpropoxy)phenyl]sulfanyl}phenyl)-N-ethyl-N-methylimidoformamid, (15.159) N'-(2,5-Dimethyl-4-{[3-(pentafluorethoxy)phenyl]sulfanyl}phenyl)-N-ethyl-N-methylimidoformamid, (15.160) 2-[3,5-Bis(difluormethyl)-1H-pyrazol-1-yl]-1-[4-(4-{5-[2-(prop-2-in-1-yloxy)phenyl]-4,5-dihydro-1,2-oxazol-3-yl}-1,3-thiazol-2-yl)piperidin-1-yl]ethanon, (15.161) 2-[3,5-Bis(difluormethyl)-1H-pyrazol-1-yl]-1-[4-(4-{5-[2-fluor-6-(prop-2-in-1-yloxy)phenyl]-4,5-dihydro-1,2-oxazol-3-yl}-1,3-thiazol-2-yl)piperidin-1-yl]ethanon, (15.162) 2-[3,5-Bis(difluormethyl)-1H-pyrazol-1-yl]-1-[4-(4-{5-[2-chlor-6-(prop-2-in-1-yloxy)phenyl]-4,5-dihydro-1,2-oxazol-3-yl}-1,3-thiazol-2-yl)piperidin-1-yl]ethanon, (15.163) 2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}phenylmethansulfonat, (15.164) 2-{3-[2-(1-{[3,5-Bis(difluormethyl)-1H-pyrazol-1-yl]¬acetyl}piperidin-4-yl)-1,3-thiazol-4-yl]-4,5-dihydro-1,2-oxazol-5-yl}-3-chlorphenylmethansulfonat, (15.165) 2-[3,5-Bis(difluormethyl)-1H-pyrazol-1-yl]-1-[4-(4-{(5S)-5-[2-(prop-2-in-1-yloxy)phenyl]-4,5-dihydro-1,2-oxazol-3-yl}-1,3-thiazol-2-yl)piperidin-1-yl]ethanon, (15.166) 2-[3,5-Bis(difluormethyl)-1H-pyrazol-1-yl]-1-[4-(4-{(5R)-5-[2-(prop-2-in-1-yloxy)phenyl]-4,5-dihydro-1,2-oxazol-3-yl}-1,3-thiazol-2-yl)piperidin-1-yl]ethanon, (15.167) 2-[3,5-Bis(difluormethyl)-1H-pyrazol-1-yl]-1-[4-(4-{(5S)-5-[2-fluor-6-(prop-2-in-1-yloxy)phenyl]-4,5-dihydro-1,2-oxazol-3-yl}-1,3-thiazol-2-yl)piperidin-1-yl]ethanon, (15.168) 2-[3,5-Bis(difluormethyl)-1H-pyrazol-1-yl]-1-[4-(4-{(5R)-5-[2-fluor-6-(prop-2-in-1-yloxy)phenyl]-4,5-dihydro-1,2-oxazol-3-yl}-1,3-thiazol-2-yl)piperidin-1-yl]ethanon, (15.169) 2-[3,5-Bis(difluormethyl)-1H-pyrazol-1-yl]-1-[4-(4-{(5S)-5-[2-chlor-6-(prop-2-in-1-yloxy)phenyl]-4,5-dihydro-1,2-oxazol-3-yl}-1,3-thiazol-2-yl)piperidin-1-yl]ethanon, (15.170) 2-[3,5-Bis(difluormethyl)-1H-pyrazol-1-yl]-1-[4-(4-{(5R)-5-[2-chlor-6-(prop-2-in-1-yloxy)phenyl]-4,5-dihydro-1,2-oxazol-3-yl}-1,3-thiazol-2-yl)piperidin-1-yl]ethanon, (15.171) 2-{(5S)-3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}phenylmethansulfonat, (15.172) 2-{(5R)-3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}phenylmethansulfonat, (15.173) 2-{(5S)-3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3-chlorphenylmethansulfonat, (15.174) 2-{(5R)-3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3-chlorphenylmethansulfonat.

### Biologische Schädlingsbekämpfungsmittel als Mischungspartner

Die Verbindungen der Formel (I) können mit biologischen Schädlingsbekämpfungsmitteln kombiniert werden.

Biologische Schädlingsbekämpfungsmittel umfassen insbesondere Bakterien, Pilze, Hefen, Pflanzenextrakte, und solche Produkte, die von Mikroorganismen gebildet wurden inklusive Proteine und sekundäre Stoffwechselprodukte.

Biologische Schädlingsbekämpfungsmittel umfassen Bakterien wie sporenbildende Bakterien, wurzelbesiedelnde Bakterien und Bakterien, die als biologische Insektizide, Fungizide oder Nematizide wirken.

Beispiele für solche Bakterien, die als biologische Schädlingsbekämpfungsmittel eingesetzt werden bzw. verwendet werden können, sind:
Bacillus amyloliquefaciens, Stamm FZB42 (DSM 231179), oder Bacillus cereus, insbesondere B. cereus Stamm CNCM I-1562 oder Bacillus firmus, Stamm I-1582 (Accession number CNCM I-1582) oder Bacillus pumilus, insbesondere Stamm GB34 (Accession No. ATCC 700814) und Stamm QST2808 (Accession No. NRRL B-30087), oder Bacillus subtilis, insbesondere Stamm GB03 (Accession No. ATCC SD-1397), oder Bacillus subtilis Stamm QST713 (Accession No. NRRL B-21661) oder Bacillus subtilis Stamm OST 30002 (Accession No. NRRL B-50421) Bacillus thuringiensis, insbesondere B. thuringiensis subspecies israelensis (serotype H-14), Stamm AM65-52 (Accession No. ATCC 1276), oder B. thuringiensis subsp. aizawai, insbesondere Stamm ABTS-1857 (SD-1372), oder B. thuringiensis subsp. kurstaki Stamm HD-1, oder B. thuringiensis subsp. tenebrionis Stamm NB 176 (SD-5428), Pasteuria penetrans, Pasteuria spp. (Rotylenchulus reniformis nematode)-PR3 (Accession Number ATCC SD-5834), Streptomyces microflavus Stamm AQ6121 (= QRD 31.013, NRRL B-50550), Streptomyces galbus Stamm AQ 6047 (Acession Number NRRL 30232).

Beispiele für Pilze und Hefen, die als biologische Schädlingsbekämpfungsmittel eingesetzt werden bzw. verwendet werden können, sind:
Beauveria bassiana, insbesondere Stamm ATCC 74040, Coniothyrium minitans, insbesondere Stamm CON/M/91-8 (Accession No. DSM-9660), Lecanicillium spp., insbesondere Stamm HRO LEC 12, Lecanicillium lecanii, (ehemals bekannt als Verticillium lecanii), insbesondere Stamm KV01, Metarhizium anisopliae, insbesondere Stamm F52 (DSM3884/ ATCC 90448), Metschnikowia fructicola, insbesondere Stamm NRRL Y-30752, Paecilomyces fumosoroseus (neu: Isaria fumosorosea), insbesondere Stamm IFPC 200613, oder Stamm Apopka 97 (Accesion No. ATCC 20874), Paecilomyces lilacinus, insbesondere P. lilacinus Stamm 251 (AGAL 89/030550), Talaromyces flavus, insbesondere Stamm V117b, Trichoderma atroviride, insbesondere Stamm SC1 (Accession Number CBS 122089), Trichoderma harzianum, insbesondere T. harzianum rifai T39. (Accession Number CNCM I-952).

Beispiele für Viren, die als biologische Schädlingsbekämpfungsmittel eingesetzt werden bzw. verwendet werden können, sind:
Adoxophyes orana (Apfelschalenwickler) Granulosevirus (GV), Cydia pomonella (Apfelwickler) Granulosevirus (GV), Helicoverpa armigera (Baumwollkapselwurm) Nuklear Polyhedrosis Virus (NPV), Spodoptera exigua (Zuckerrübeneule) mNPV, Spodoptera frugiperda (Heerwurm) mNPV, Spodoptera littoralis (Afrikanischer Baumwollwurm) NPV.

Es sind auch Bakterien und Pilze umfasst, die als ,Inokulant' Pflanzen oder Pflanzenteilen oder Pflanzenorganen beigegeben werden und durch ihre besonderen Eigenschaften das Pflanzenwachstum und die Pflanzengesundheit fördern. Als Beispiele sind genannt:
Agrobacterium spp., Azorhizobium caulinodans, Azospirillum spp., Azotobacter spp., Bradyrhizobium spp., Burkholderia spp., insbesondere Burkholderia cepacia (ehemals bekannt als Pseudomonas cepacia), Gigaspora spp., oder Gigaspora monosporum, Glomus spp., Laccaria spp., Lactobacillus buchneri, Paraglomus spp., Pisolithus tinctorus, Pseudomonas spp., Rhizobium spp., insbesondere Rhizobium trifolii, Rhizopogon spp., Scleroderma spp., Suillus spp., Streptomyces spp..

Beispiele für Pflanzenextrakte und solche Produkte, die von Mikroorganismen gebildet wurden inklusive Proteine und sekundäre Stoffwechselprodukte, die als biologische Schädlingsbekämpfungsmittel eingesetzt werden bzw. verwendet werden können, sind:
Allium sativum, Artemisia absinthium, Azadirachtin, Biokeeper WP, Cassia nigricans, Celastrus angulatus, Chenopodium anthelminticum, Chitin, Armour-Zen, Dryopteris filix-mas, Equisetum arvense,Fortune Aza, Fungastop, Heads Up (Chenopodium quinoa-Saponinextrakt), Pyrethrum/Pyrethrins, Quassia amara, Quercus, Quillaja, Regalia, "Requiem™ Insecticide", Rotenon, Ryania/Ryanodine, Symphytum officinale, Tanacetum vulgare, Thymol, Triact 70, TriCon, Tropaeulum majus, Urtica dioica, Veratrin, Viscum album, Brassicacaeen-Extrakt, insbesondere Raps- oder Senfpulver.

### Safener als Mischpartner

Die Verbindungen der Formel (I) können mit Safenern kombiniert werden, wie zum Beispiel Benoxacor, Cloquintocet (-mexyl), Cyometrinil, Cyprosulfamide, Dichlormid, Fenchlorazole (-ethyl), Fenclorim, Flurazole, Fluxofenim, Furilazole, Isoxadifen (-ethyl), Mefenpyr (-diethyl), Naphthalic anhydride, Oxabetrinil, 2-Methoxy-N-({4-[(methylcarbamoyl)amino]phenyl}sulfonyl)benzamid (CAS 129531-12-0), 4-(Dichloroacetyl)-1-oxa-4-azaspiro[4.5]decane (CAS 71526-07-3), 2,2,5-Trimethyl-3-(dichloroacetyl)-1,3-oxazolidine (CAS 52836-31-4).

### Pflanzen und Pflanzenteile

Erfindungsgemäß können alle Pflanzen und Pflanzenteile behandelt werden. Unter Pflanzen werden hierbei alle Pflanzen und Pflanzenpopulationen verstanden wie erwünschte und unerwünschte Wildpflanzen oder Kulturpflanzen (einschließlich natürlich vorkommender Kulturpflanzen), beispielsweise Getreide (Weizen, Reis, Triticale, Gerste, Roggen, Hafer), Mais, Soja, Kartoffel, Zuckerrüben, Zuckerrohr, Tomaten, Erbsen und andere Gemüsesorten, Baumwolle, Tabak, Raps, sowie Obstpflanzen (mit den Früchten Äpfel, Birnen, Zitrusfrüchten und Weintrauben). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Verbindungen der Formel (I) erfolgt direkt oder durch Einwirkung auf deren Umgebung, Lebensraum oder Lager-raum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Wie bereits oben erwähnt können erfindungsgemäß alle Pflanzen und deren Teile behandelt werden. In einer bevorzugten Ausführungsform werden wild vorkommende oder durch konventionelle biologische Zuchtmethoden wie Kreuzung oder Protoplastenfusion erhaltene Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert. Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Rassen, Bio- und Genotypen sein.

### Transgene Pflanze, Saatgutbehandlung und Integrationsereignisse

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernte¬produkte. Weitere und besonders hervorgehobene Beispiele für solche Eigenschaften sind eine erhöhte Abwehrfähigkeit der Pflanzen gegen tierische und mikrobielle Schädlinge, wie Insekten, Spinnentiere, Nematoden, Milben, Schnecken, bewirkt z.B. durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden, ferner eine erhöhte Abwehrfähigkeit der Pflanzen gegen pflanzenpathogene Pilze, Bakterien und/oder Viren, bewirkt z.B. durch Systemisch Akquirierte Resistenz (SAR), Systemin, Phytoalexine, Elicitoren sowie Resistenzgene und entsprechend exprimierte Proteine und Toxine, sowie eine erhöhte Toleranz der Pflanzen gegen bestimmte herbizide Wirkstoffe, beispielsweise Imidazolinonen, Sulfonyl-harnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele transgener Pflanzen werden die wichtigen Kulturpflanzen, wie Getreide (Weizen, Reis, Triticale, Gerste, Roggen, Hafer), Mais, Soja, Kartoffel, Zuckerrüben, Zuckerrohr, Tomaten, Erbsen und andere Gemüsesorten, Baumwolle, Tabak, Raps, sowie Obstpflanzen (mit den Früchten Äpfel, Birnen, Zitrusfrüchten und Weintrauben) erwähnt, wobei Mais, Soja, Weizen, Reis, Kartoffel, Baumwolle, Zuckerrohr, Tabak und Raps besonders hervorgehoben werden. Als Eigenschaften ("Traits") werden besonders hervorgehoben die erhöhte Abwehrfähigkeit der Pflanzen gegen Insekten, Spinnentiere, Nematoden und Schnecken.

### Pflanzenschutz - Behandlungsarten

Die Behandlung der Pflanzen und Pflanzenteile mit den Verbindungen der Formel (I) erfolgt direkt oder durch Einwirkung auf deren Umgebung, Lebensraum oder Lagerraum nach den üblichen Behandlungsmethoden, z.B. durch Tauchen, (Ver-) Spritzen, (Ver-)Sprühen, Berieseln, Verdampfen, Zerstäuben, Vernebeln, (Ver-)Streuen, Verschäumen, Bestreichen, Verstreichen, Injizieren, Gießen (drenchen), Tröpfchenbewässerung und bei Vermehrungsmaterial, insbesondere bei Saatgut, weiterhin durch Trockenbeizen, Nassbeizen, Schlämmbeizen, Inkrustieren, ein- oder mehrschichtiges Umhüllen, usw. Es ist ferner möglich, die Verbindungen der Formel (I) nach dem Ultra-Low-Volume-Verfahren auszubringen oder die Anwendungsform oder die Verbindung der Formel (I) selbst in den Boden zu injizieren.

Eine bevorzugte direkte Behandlung der Pflanzen ist die Blattapplikation, d.h. Verbindungen der Formel (I) werden auf das Blattwerk aufgebracht, wobei die Behandlungsfrequenz und die Aufwandmenge auf den Befallsdruck des jeweiligen Schädlings abgestimmt sein sollte.

Bei systemisch wirksamen Verbindungen gelangen die Verbindungen der Formel (I) auch über das Wurzelwerk in die Pflanzen. Die Behandlung der Pflanzen erfolgt dann durch Einwirkung der Verbindungen der Formel (I) auf den Lebensraum der Pflanze. Das kann beispielsweise durch Drenchen, Einmischen in den Boden oder die Nährlösung sein, d.h. der Standort der Pflanze (z.B. Boden oder hydroponische Systeme) wird mit einer flüssigen Form der Verbindungen der Formel (I) getränkt, oder durch die Bodenapplikation, d.h. die Verbindungen der Formel (I) werden in fester Form, (z.B. in Form eines Granulats) in den Standort der Pflanzen eingebracht. Bei Wasserreiskulturen kann das auch durch Zudosieren der Verbindung der Formel (I) in einer festen Anwendungsform (z.B. als Granulat) in ein überflutetes Reisfeld sein.

### Saatgutbehandlung

Die Bekämpfung von tierischen Schädlingen durch die Behandlung des Saatguts von Pflanzen ist seit langem bekannt und ist Gegenstand ständiger Verbesserungen. Dennoch ergeben sich bei der Behandlung von Saatgut eine Reihe von Problemen, die nicht immer zufriedenstellend gelöst werden können. So ist es erstrebenswert, Verfahren zum Schutz des Saatguts und der keimenden Pflanze zu entwickeln, die das zusätzliche Ausbringen von Schädlingsbekämpfungsmitteln bei der Lagerung, nach der Saat oder nach dem Auflaufen der Pflanzen überflüssig machen oder zumindest deutlich verringern. Es ist weiterhin erstrebenswert, die Menge des eingesetzten Wirkstoffs dahingehend zu optimieren, dass das Saatgut und die keimende Pflanze vor dem Befall durch tierische Schädlinge bestmöglich geschützt werden, ohne jedoch die Pflanze selbst durch den eingesetzten Wirkstoff zu schädigen. Insbesondere sollten Verfahren zur Behandlung von Saatgut auch die intrinsischen insektiziden bzw. nematiziden Eigenschaften schädlingsresistenter bzw. - toleranter transgener Pflanzen einbeziehen, um einen optimalen Schutz des Saatguts und der keimenden Pflanze bei einem minimalen Aufwand an Schädlingsbekämpfungsmitteln zu erreichen.

Die vorliegende Erfindung bezieht sich daher insbesondere auch auf ein Verfahren zum Schutz von Saatgut und keimenden Pflanzen vor dem Befall von Schädlingen, indem das Saatgut mit einer der Verbindungen der Formel (I) behandelt wird. Das erfindungsgemäße Verfahren zum Schutz von Saatgut und keimenden Pflanzen vor dem Befall von Schädlingen umfasst ferner ein Verfahren, in dem das Saatgut gleichzeitig in einem Vorgang oder sequentiell mit einer Verbindung der Formel (I) und Mischungspartner behandelt wird. Es umfasst ferner auch ein Verfahren, in dem das Saatgut zu unterschiedlichen Zeiten mit einer Verbindung der Formel (I) und Mischungspartner behandelt wird.

Die Erfindung bezieht sich ebenfalls auf die Verwendung der Verbindungen der Formel (I) zur Behandlung von Saatgut zum Schutz des Saatguts und der daraus entstehenden Pflanze vor tierischen Schädlingen.

Weiterhin bezieht sich die Erfindung auf Saatgut, welches zum Schutz vor tierischen Schädlingen mit einer Verbindung der Formel (I) behandelt wurde. Die Erfindung bezieht sich auch auf Saatgut, welches zur gleichen Zeit mit einer Verbindung der Formel (I) und Mischungspartner behandelt wurde. Die Erfindung bezieht sich weiterhin auf Saatgut, welches zu unterschiedlichen Zeiten mit einer Verbindung der Formel (I) und Mischungspartner behandelt wurde. Bei Saatgut, welches zu unterschiedlichen Zeiten mit einer Verbindung der Formel (I) und Mischungspartner behandelt wurde, können die einzelnen Substanzen in unterschiedlichen Schichten auf dem Saatgut enthalten sein. Dabei können die Schichten, die eine Verbindung der Formel (I) und Mischungspartner enthalten, gegebenenfalls durch eine Zwischenschicht getrennt sein. Die Erfindung bezieht sich auch auf Saatgut, bei dem eine Verbindung der Formel (I) und Mischungspartner als Bestandteil einer Umhüllung oder als weitere Schicht oder weitere Schichten zusätzlich zu einer Umhüllung aufgebracht sind.

Des Weiteren bezieht sich die Erfindung auf Saatgut, welches nach der Behandlung mit einer Verbindung der Formel (I) einem Filmcoating - Verfahren unterzogen wird, um Staubabrieb am Saatgut zu vermeiden.

Einer der auftretenden Vorteile, wenn eine der Verbindungen der Formel (I) systemisch wirkt, ist es, dass die Behandlung des Saatguts nicht nur das Saatgut selbst, sondern auch die daraus hervorgehenden Pflanzen nach dem Auflaufen vor tierischen Schädlingen schützt. Auf diese Weise kann die unmittelbare Behandlung der Kultur zum Zeitpunkt der Aussaat oder kurz danach entfallen.

Ein weiterer Vorteil ist darin zu sehen, dass durch die Behandlung des Saatguts mit einer Verbindung der Formel (I) Keimung und Auflauf des behandelten Saatguts gefördert werden können.

Ebenso ist es als vorteilhaft anzusehen, dass Verbindungen der Formel (I) insbesondere auch bei transgenem Saatgut eingesetzt werden können.

Verbindungen der Formel (I) können ferner in Kombination mit Mitteln der Signaltechnologie eingesetzt werden, wodurch eine bessere Besiedlung mit Symbionten, wie zum Beispiel Rhizobien, Mycorrhiza und/oder endophytischen Bakterien oder Pilzen, stattfindet und/oder es zu einer optimierten Stickstofffixierung kommt.

Die Verbindungen der Formel (I) eignen sich zum Schutz von Saatgut jeglicher Pflanzensorte, die in der Landwirtschaft, im Gewächshaus, in Forsten oder im Gartenbau eingesetzt wird. Insbesondere handelt es sich dabei um Saatgut von Getreide (z. B. Weizen, Gerste, Roggen, Hirse und Hafer), Mais, Baum-wolle, Soja, Reis, Kartoffeln, Sonnenblume, Kaffee, Tabak, Canola, Raps, Rübe (z.B. Zuckerrübe und Futterrübe), Erdnuss, Gemüse (z. B. Tomate, Gurke, Bohne, Kohlgewächse, Zwiebeln und Salat), Obstpflanzen, Rasen und Zierpflanzen. Besondere Bedeutung kommt der Behandlung des Saatguts von Getreide (wie Weizen, Gerste, Roggen und Hafer), Mais, Soja, Baumwolle, Canola, Raps und Reis zu.

Wie vorstehend bereits erwähnt, kommt auch der Behandlung von transgenem Saatgut mit einer Verbindung der Formel (I) eine besondere Bedeutung zu. Dabei handelt es sich um das Saatgut von Pflanzen, die in der Regel zumindest ein heterologes Gen enthalten, das die Expression eines Polypeptids mit insbesondere insektiziden bzw. nematiziden Eigenschaften steuert. Die heterologen Gene in transgenem Saatgut können dabei aus Mikroorganismen wie Bacillus, Rhizobium, Pseudomonas, Serratia, Trichoderma, Clavibacter, Glomus oder Gliocladium stammen. Die vorliegende Erfindung eignet sich besonders für die Behandlung von trans-genem Saatgut, das zumindest ein heterologes Gen enthält, das aus Bacillus sp. stammt. Besonders bevorzugt handelt es sich dabei um ein heterologes Gen, das aus Bacillus thuringiensis stammt.

Im Rahmen der vorliegenden Erfindung wird die Verbindung der Formel (I) auf das Saatgut aufgebracht. Vorzugsweise wird das Saatgut in einem Zustand behandelt, in dem es so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hülle, Wolle oder Fruchtfleisch befreit wurde. So kann zum Beispiel Saatgut verwendet werden, das geerntet, gereinigt und bis zu einem lagerfähigen Feuchtigkeitsgehalt getrocknet wurde. Alternativ kann auch Saatgut verwendet werden, das nach dem Trocknen z.B. mit Wasser behandelt und dann erneut getrocknet wurde, zum Beispiel Priming. Im Falle von Reissaatgut ist es auch möglich Saatgut zu verwenden, das zum Beispiel in Wasser bis zu einem bestimmten Stadium vorgequollen wurde (pigeon breast Stadium), was zu einer verbesserten Keimung und zu einem gleichmäßigeren Auflaufen führt.

Im Allgemeinen muss bei der Behandlung des Saatguts darauf geachtet werden, dass die Menge der auf das Saatgut aufgebrachten Verbindung der Formel (I) und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei Wirkstoffen zu beachten, die in bestimmten Aufwandmengen phytotoxische Effekte zeigen können.

Die Verbindungen der Formel (I) werden in der Regel in Form einer geeigneten Formulierung auf das Saatgut aufgebracht. Geeignete Formulierungen und Verfahren für die Saatgutbehandlung sind dem Fachmann bekannt.

Die Verbindungen der Formel (I) können in die üblichen Beizmittel-Formulierungen überführt werden, wie Lösungen, Emulsionen, Suspensionen, Pulver, Schäume, Slurries oder andere Hüllmassen für Saatgut, sowie ULV-Formulierungen.

Diese Formulierungen werden in bekannter Weise hergestellt, indem man Verbindungen der Formel (I) mit üblichen Zusatzstoffen vermischt, wie zum Beispiel übliche Streckmittel sowie Lösungs- oder Verdünnungsmittel, Farbstoffe, Netzmittel, Dispergiermittel, Emulgatoren, Entschäumer, Konservierungsmittel, sekundäre Verdickungsmittel, Kleber, Gibberelline und auch Wasser.

Als Farbstoffe, die in den erfindungsgemäß verwendbaren Beizmittel-Formulierungen enthalten sein können, kommen alle für derartige Zwecke üblichen Farbstoffe in Betracht. Dabei sind sowohl in Wasser wenig lösliche Pigmente als auch in Wasser lösliche Farbstoffe verwendbar. Als Beispiele genannt seien die unter den Bezeichnungen Rhodamin B, C.I. Pigment Red 112 und C.I. Solvent Red 1 bekannten Farbstoffe.

Als Netzmittel, die in den erfindungsgemäß verwendbaren Beizmittel-Formulierungen enthalten sein können, kommen alle zur Formulierung von agrochemischen Wirkstoffen üblichen, die Benetzung fördernden Stoffe in Frage. Vorzugsweise verwendbar sind Alkylnaphthalin-Sulfonate, wie Diisopropyl- oder Diisobutyl-naphthalin-Sulfonate.

Als Dispergiermittel und/oder Emulgatoren, die in den erfindungsgemäß verwendbaren Beizmittel-Formulierungen enthalten sein können, kommen alle zur Formulierung von agrochemischen Wirkstoffen üblichen nichtionischen, anionischen und kationischen Dispergiermittel in Betracht. Vor-zugsweise verwendbar sind nichtionische oder anionische Dispergiermittel oder Gemische von nichtionischen oder anionischen Dispergiermitteln. Als geeignete nichtionische Dispergiermittel sind insbesondere Ethylenoxid-Propylenoxid Blockpolymere, Alkylphenolpolyglykolether sowie Tristryrylphenolpolyglykolether und deren phosphatierte oder sulfatierte Derivate zu nennen. Geeignete anionische Dispergiermittel sind insbesondere Ligninsulfonate, Polyacrylsäuresalze und Arylsulfonat-Formaldehydkondensate.

Als Entschäumer können in den erfindungsgemäß verwendbaren Beizmittel-Formulierungen alle zur Formulierung von agrochemischen Wirkstoffen üblichen schaumhemmenden Stoffe enthalten sein. Vorzugsweise verwendbar sind Silikonentschäumer und Magnesiumstearat.

Als Konservierungsmittel können in den erfindungsgemäß verwendbaren Beizmittel-Formulierungen alle für derartige Zwecke in agrochemischen Mitteln einsetzbaren Stoffe vorhanden sein. Beispielhaft genannt seien Dichlorophen und Benzylalkoholhemiformal.

Als sekundäre Verdickungsmittel, die in den erfindungsgemäß verwendbaren Beizmittel-Formulierungen enthalten sein können, kommen alle für derartige Zwecke in agrochemischen Mitteln einsetzbaren Stoffe in Frage. Vorzugsweise in Betracht kommen Cellulosederivate, Acrylsäurederivate, Xanthan, modifizierte Tone und hochdisperse Kieselsäure.

Als Kleber, die in den erfindungsgemäß verwendbaren Beizmittel-Formulierungen enthalten sein können, kommen alle üblichen in Beizmitteln einsetzbaren Bindemittel in Frage. Vorzugsweise genannt seien Polyvinylpyrrolidon, Polyvinylacetat, Polyvinylalkohol und Tylose.

Als Gibberelline, die in den erfindungsgemäß verwendbaren Beizmittel-Formulierungen enthalten sein können, kommen vorzugsweise die Gibberelline A1, A3 (= Gibberellinsäure), A4 und A7 infrage, besonders bevorzugt verwendet man die Gibberellinsäure. Die Gibberelline sind bekannt (vgl. R. Wegler "Chemie der Pflanzenschutz- und Schädlingsbekämpfungsmittel", Bd. 2, Springer Verlag, 1970, S. 401-412).

Die erfindungsgemäß verwendbaren Beizmittel-Formulierungen können entweder direkt oder nach vorherigem Verdünnen mit Wasser zur Behandlung von Saatgut der verschiedensten Art eingesetzt werden. So lassen sich die Konzentrate oder die daraus durch Verdünnen mit Wasser erhältlichen Zubereitungen einsetzen zur Beizung des Saatgutes von Getreide, wie Weizen, Gerste, Roggen, Hafer und Triticale, sowie des Saatgutes von Mais, Reis, Raps, Erbsen, Bohnen, Baumwolle, Sonnenblumen, Soja und Rüben oder auch von Gemüsesaatgut der verschiedensten Natur. Die erfindungsgemäß verwendbaren Beizmittel-Formulierungen oder deren verdünnte Anwendungsformen können auch zum Beizen von Saatgut transgener Pflanzen eingesetzt werden.

Zur Behandlung von Saatgut mit den erfindungsgemäß verwendbaren Beizmittel-Formulierungen oder daraus hergestellten Anwendungsformen kommen alle üblicherweise für die Beizung einsetzbaren Mischgeräte in Betracht. Im einzelnen geht man bei der Beizung so vor, dass man das Saatgut in einen Mischer im diskontinuierlichem oder kontinuierlichem Betrieb gibt, die jeweils gewünschte Menge an Beizmittel-Formulierungen entweder als solche oder nach vorherigem Verdünnen mit Wasser hinzufügt und bis zur gleichmäßigen Verteilung der Formulierung auf dem Saatgut mischt. Gegebenenfalls schließt sich ein Trocknungsvorgang an.

Die Aufwandmenge an den erfindungsgemäß verwendbaren Beizmittel-Formulierungen kann inner-halb eines größeren Bereiches variiert werden. Sie richtet sich nach dem jeweiligen Gehalt der Verbindungen der Formel (I) in den Formulierungen und nach dem Saatgut. Die Aufwandmengen bei der Verbindung der Formel (I) liegen im Allgemeinen zwischen 0,001 und 50 g pro Kilogramm Saatgut, vorzugsweise zwischen 0,01 und 15 g pro Kilogramm Saatgut.

### Tiergesundheit

Auf dem Gebiet der Tiergesundheit, d.h. dem Gebiet der Tiermedizin, sind die Verbindungen der Formel (I) gegen Tierparasiten, insbesondere Ektoparasiten oder Endoparasiten, wirksam. Der Begriff Endoparasiten umfasst insbesondere Helminthen und Protozoa wie Kokzidien. Ektoparasiten sind typischerweise und bevorzugt Arthropoden, insbesondere Insekten und Akariden.

Auf dem Gebiet der Tiermedizin eignen sich die Verbindungen der Formel (I), die eine günstige Toxizität gegenüber Warmblütern aufweisen, für die Bekämpfung von Parasiten, die in der Tierzucht und Tierhaltung bei Nutztieren, Zuchttieren, Zootieren, Laboratoriumstieren, Versuchstieren und Haustieren auftreten. Sie sind gegen alle oder einzelne Entwicklungsstadien der Parasiten wirksam.

Zu den landwirtschaftlichen Nutztieren zählen zum Beispiel Säugetiere wie Schafe, Ziegen, Pferde, Esel, Kamele, Büffel, Kaninchen, Rentiere, Damhirsche und insbesondere Rinder und Schweine; Geflügel wie Truthähne, Enten, Gänse und insbesondere Hühner; Fische und Krustentiere, z.B. in der Aquakultur und auch Insekten wie Bienen.

Zu den Haustieren zählen zum Beispiel Säugetiere wie Hamster, Meerschweinchen, Ratten, Mäuse, Chinchillas, Frettchen und insbesondere Hunde, Katzen, Stubenvögel, Reptilien, Amphibien und Aquariumfische.

Gemäß einer bevorzugten Ausführungsform werden die Verbindungen der Formel (I) an Säugetiere verabreicht.

Gemäß einer weiteren bevorzugten Ausführungsform werden die Verbindungen der Formel (I) an Vögel, nämlich Stubenvögel und insbesondere Geflügel, verabreicht.

Durch Verwendung der Verbindungen der Formel (I) für die Bekämpfung von Tierparasiten sollen Krankheit, Todesfälle und Leistungsminderungen (bei Fleisch, Milch, Wolle, Häuten, Eiern, Honig und dergleichen) verringert bzw. vorgebeugt werden, so dass eine wirtschaftlichere und einfachere Tierhaltung ermöglicht wird und ein besseres Wohlbefinden der Tiere erzielbar ist.

In Bezug auf das Gebiet der Tiergesundheit bedeutet der Begriff "Bekämpfung" oder "bekämpfen", dass durch die Verbindungen der Formel (I) wirksam das Auftreten des jeweiligen Parasiten in einem Tier, das mit solchen Parasiten in einem harmlosen Ausmaß infiziert ist, reduziert werden kann. Genauer gesagt bedeutet "bekämpfen" im vorliegenden Zusammenhang, dass die Verbindung der Formel (I) den jeweiligen Parasiten abtöten, sein Wachstum verhindern oder seine Vermehrung verhindern kann.

Zu den Arthropoden zählen:
aus der Ordnung Anoplurida, zum Beispiel Haematopinus spp., Linognathus spp., Pediculus spp., Phtirus spp., Solenopotes spp.; aus der Ordnung Mallophagida und den Unterordnungen Amblycerina and Ischnocerina, zum Beispiel Trimenopon spp., Menopon spp., Trinoton spp., Bovicola spp., Werneckiella spp., Lepikentron spp., Damalina spp., Trichodectes spp., Felicola spp.; aus der Ordnung Diptera und den Unterordnungen Nematocerina und Brachycerina, zum Beispiel Aedes spp., Anopheles spp., Culex spp., Simulium spp., Eusimulium spp., Phlebotomus spp., Lutzomyia spp., Culicoides spp., Chrysops spp., Odagmia spp., Wilhelmia spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp., Rhinoestrus spp., Tipula spp.; aus der Ordnung Siphonapterida, zum Beispiel Pulex spp., Ctenocephalides spp., Tunga spp., Xenopsylla spp., Ceratophyllus spp.;
aus der Ordnung Heteropterida, zum Beispiel Cimex spp., Triatoma spp., Rhodnius spp., Panstrongylus spp.; sowie Lästlinge und Hygieneschädlinge aus der Ordnung Blattarida.

### Weiterhin zählen zu den Arthropoden:

Aus der Unterklasse Akari (Acarina) und der Ordnung Metastigmata, zum Beispiel aus der Familie Argasidae, wie Argas spp., Ornithodorus spp., Otobius spp., aus der Familie Ixodidae, wie Ixodes spp., Amblyomma spp., Rhipicephalus (Boophilus) spp. Dermacentor spp., Haemophysalis spp., Hyalomma spp., Rhipicephalus spp. (die ursprüngliche Gattung der mehrwirtigen Zecken); aus der Ordnung Mesostigmata, wie Dermanyssus spp., Ornithonyssus spp., Pneumonyssus spp., Raillietia spp., Pneumonyssus spp., Sternostoma spp., Varroa spp., Acarapis spp.; aus der Ordnung Actinedida (Prostigmata), zum Beispiel Acarapis spp., Cheyletiella spp., Ornithocheyletia spp., Myobia spp., Psorergates spp., Demodex spp., Trombicula spp., Neotrombiculla spp., Listrophorus spp.; und aus der Ordnung Acaridida (Astigmata), zum Beispiel Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Zu parasitären Protozoen zählen:
Mastigophora (Flagellata), wie zum Beispiel Trypanosomatidae, zum Beispiel Trypanosoma b. brucei, T.b. gambiense, T.b. rhodesiense, T. congolense, T. cruzi, T. evansi, T. equinum, T. lewisi, T. percae, T. simiae, T. vivax, Leishmania brasiliensis, L. donovani, L. tropica, wie zum Beispiel Trichomonadidae, zum Beispiel Giardia lamblia, G. canis;
Sarcomastigophora (Rhizopoda), wie Entamoebidae, zum Beispiel Entamoeba histolytica, Hartmanellidae, zum Beispiel Acanthamoeba sp., Harmanella sp.;
Apicomplexa (Sporozoa), wie Eimeridae, zum Beispiel Eimeria acervulina, E. adenoides, E. alabamensis, E. anatis, E. anserina, E. arloingi, E. ashata, E. auburnensis, E. bovis, E. brunetti, E. canis, E. chinchillae, E. clupearum, E. columbae, E. contorta, E. crandalis, E. debliecki, E. dispersa, E. ellipsoidales, E. falciformis, E. faurei, E. flavescens, E. gallopavonis, E. hagani, E. intestinalis, E. iroquoina, E. irresidua, E. labbeana, E. leucarti, E. magna, E. maxima, E. media, E. meleagridis, E. meleagrimitis, E. mitis, E. necatrix, E. ninakohlyakimovae, E. ovis, E. parva, E. pavonis, E. perforans, E. phasani, E. piriformis, E. praecox, E. residua, E. scabra, E. spec., E. stiedai, E. suis, E. tenella, E. truncata, E. truttae, E. zuernii, Globidium spec., Isospora belli, I. canis, I. felis, I. ohioensis, I. rivolta, I. spec., I. suis, Cystisospora spec., Cryptosporidium spec., insbesondere C. parvum; wie Toxoplasmadidae, zum Beispiel Toxoplasma gondii, Hammondia heydornii, Neospora caninum, Besnoitia besnoitii; wie Sarcocystidae, zum Beispiel Sarcocystis bovicanis, S. bovihominis, S. ovicanis, S. ovifelis, S. neurona, S. spec., S. suihominis, wie Leucozoidae, zum Beispiel Leucozytozoon simondi, wie Plasmodiidae, zum Beispiel Plasmodium berghei, P. falciparum, P. malariae, P. ovale, P. vivax, P. spec., wie Piroplasmea, zum Beispiel Babesia argentina, B. bovis, B. canis, B. spec., Theileria parva, Theileria spec., wie Adeleina, zum Beispiel Hepatozoon canis, H. spec..

Zu pathogenen Endoparasiten, bei denen es sich um Helminthen handelt, zählen Plattwürmer (z.B. Monogenea, Cestodes und Trematodes), Rundwürmer, Acanthocephala und Pentastoma. Dazu zählen:
Monogenea: z.B.: Gyrodactylus spp., Dactylogyrus spp., Polystoma spp.;
Cestodes: aus der Ordnung Pseudophyllidea zum Beispiel: Diphyllobothrium spp., Spirometra spp., Schistocephalus spp., Ligula spp., Bothridium spp., Diplogonoporus spp.;
aus der Ordnung Cyclophyllida zum Beispiel: Mesocestoides spp., Anoplocephala spp., Paranoplocephala spp., Moniezia spp., Thysanosoma spp., Thysaniezia spp., Avitellina spp., Stilesia spp., Cittotaenia spp., Andyra spp., Bertiella spp., Taenia spp., Echinococcus spp., Hydatigera spp., Davainea spp., Raillietina spp., Hymenolepis spp., Echinolepis spp., Echinocotyle spp., Diorchis spp., Dipylidium spp., Joyeuxiella spp., Diplopylidium spp.;
Trematodes: aus der Klasse Digenea zum Beispiel: Diplostomum spp., Posthodiplostomum spp., Schistosoma spp., Trichobilharzia spp., Ornithobilharzia spp., Austrobilharzia spp., Gigantobilharzia spp., Leucochloridium spp., Brachylaima spp., Echinostoma spp., Echinoparyphium spp., Echinochasmus spp., Hypoderaeum spp., Fasciola spp., Fascioloides spp., Fasciolopsis spp., Cyclocoelum spp., Typhlocoelum spp., Paramphistomum spp., Calicophoron spp., Cotylophoron spp., Gigantocotyle spp., Fischoederius spp., Gastrothylacus spp., Notocotylus spp., Catatropis spp., Plagiorchis spp., Prosthogonimus spp., Dicrocoelium spp., Eurytrema spp., Troglotrema spp., Paragonimus spp., Collyriclum spp., Nanophyetus spp., Opisthorchis spp., Clonorchis spp. Metorchis spp., Heterophyes spp., Metagonimus spp.;
Rundwürmer: Trichinellida zum Beispiel: Trichuris spp., Capillaria spp., Paracapillaria spp., Eucoleus spp., Trichomosoides spp., Trichinella spp.;
aus der Ordnung Tylenchida zum Beispiel: Micronema spp., Strongyloides spp.;
aus der Ordnung Rhabditida zum Beispiel: Strongylus spp., Triodontophorus spp., Oesophagodontus spp., Trichonema spp., Gyalocephalus spp., Cylindropharynx spp., Poteriostomum spp., Cyclococercus spp., Cylicostephanus spp., Oesophagostomum spp., Chabertia spp., Stephanurus spp., Ancylostoma spp., Uncinaria spp., Necator spp., Bunostomum spp., Globocephalus spp., Syngamus spp., Cyathostoma spp., Metastrongylus spp., Dictyocaulus spp., Muellerius spp., Protostrongylus spp., Neostrongylus spp., Cystocaulus spp., Pneumostrongylus spp., Spicocaulus spp., Elaphostrongylus spp. Parelaphostrongylus spp., Crenosoma spp., Paracrenosoma spp., Oslerus spp., Angiostrongylus spp., Aelurostrongylus spp., Filaroides spp., Parafilaroides spp., Trichostrongylus spp., Haemonchus spp., Ostertagia spp., Teladorsagia spp., Marshallagia spp., Cooperia spp., Nippostrongylus spp., Heligmosomoides spp., Nematodirus spp., Hyostrongylus spp., Obeliscoides spp., Amidostomum spp., Ollulanus spp.;
aus der Ordnung Spirurida zum Beispiel: Oxyuris spp., Enterobius spp., Passalurus spp., Syphacia spp., Aspiculuris spp., Heterakis spp.; Ascaris spp., Toxascaris spp., Toxocara spp., Baylisascaris spp., Parascaris spp., Anisakis spp., Ascaridia spp.; Gnathostoma spp., Physaloptera spp., Thelazia spp., Gongylonema spp., Habronema spp., Parabronema spp., Draschia spp., Dracunculus spp.; Stephanofilaria spp., Parafilaria spp., Setaria spp., Loa spp., Dirofilaria spp., Litomosoides spp., Brugia spp., Wuchereria spp., Onchocerca spp., Spirocerca spp.;
Acanthocephala: aus der Ordnung Oligacanthorhynchida z.B: Macracanthorhynchus spp., Prosthenorchis spp.; aus der Ordnung Polymorphida zum Beispiel: Filicollis spp.; aus der Ordnung Moniliformida zum Beispiel: Moniliformis spp.;
aus der Ordnung Echinorhynchida zum Beispiel Acanthocephalus spp., Echinorhynchus spp., Leptorhynchoides spp.;
Pentastoma: aus der Ordnung Porocephalida zum Beispiel Linguatula spp..

Auf dem Gebiet der Tiermedizin und der Tierhaltung erfolgt die Verabreichung der Verbindungen der Formel (I) nach allgemein fachbekannten Verfahren, wie enteral, parenteral, dermal oder nasal in Form von geeigneten Präparaten. Die Verabreichung kann prophylaktisch oder therapeutisch erfolgen.

So bezieht sich eine Ausführungsform der vorliegenden Erfindung auf die Verwendung einer Verbindung der Formel (I) als Arzneimittel.

Ein weiterer Aspekt bezieht sich auf die Verwendung einer Verbindung der Formel (I) als Antiendoparasitikum, insbesondere als ein Helminthizid oder ein Mittel gegen Protozoen. Verbindungen der Formel (I) eignen sich für die Verwendung als Antiendoparasitikum, insbesondere als ein Helminthizid oder Mittel gegen Protozoen, beispielsweise in der Tierzucht, in der Tierhaltung, in Ställen und auf dem Hygienesektor.

Ein weiterer Aspekt wiederum betrifft die Verwendung einer Verbindung der Formel (I) als Antiektoparasitikum, insbesondere ein Arthropodizid wie ein Insektizid oder ein Akarizid Ein weiterer Aspekt betrifft die Verwendung einer Verbindung der Formel (I) als Antiektoparasitikum, insbesondere ein Arthropodizid wie ein Insektizid oder Akarizid, zum Beispiel in der Tierhaltung, in der Tierzucht, in Ställen oder auf dem Hygienesektor.

### Vektorkontrolle

Die Verbindungen der Formel (I) können auch in der Vektorkontrolle eingesetzt werden. Ein Vektor im Sinne der vorliegenden Erfindung ist ein Arthropode, insbesondere ein Insekt oder Arachnide, der in der Lage ist, Krankheitserreger wie z. B. Viren, Würmer, Einzeller und Bakterien aus einem Reservoir (Pflanze, Tier, Mensch, etc.) auf einen Wirt zu übertragen. Die Krankheitserreger können entweder mechanisch (z.B. Trachoma durch nicht-stechende Fliegen) auf einem Wirt, oder nach Injektion (z.B. Malaria-Parasiten durch Mücken) in einen Wirt übertragen werden.

Beispiele für Vektoren und die von ihnen übertragenen Krankheiten bzw. Krankheitserreger sind:
1) Mücken
   - Anopheles: Malaria, Filariose;
   - Culex: Japanische Encephalitis, Filariasis, weitere virale Erkrankungen, Übertragung von Würmern;
   - Aedes: Gelbfieber, Dengue-Fieber, Filariasis, weitere virale Erkrankungen;
   - Simulien: Übertragung von Würmern insbesondere Onchocerca volvulus;
2) Läuse: Hautinfektionen, Fleckfieber (epidemic typhus);
3) Flöhe: Pest, endemisches Fleckfieber;
4) Fliegen: Schlafkrankheit (Trypanosomiasis); Cholera, weitere bakterielle Erkrankungen;
5) Milben: Acariose, Fleckfieber, Rickettsipocken, Tularämie, Saint-Louis-Enzephalitis, virale Hirnhautentzündung (FSME), Krim-Kongo-Fieber, Borreliose;
6) Zecken: Borelliosen wie Borrelia duttoni, Frühsommer-Meningoenzephalitis, Q-Fieber (Coxiella burnetii), Babesien (Babesia canis canis).

Beispiele für Vektoren im Sinne der vorliegenden Erfindung sind Insekten wie Aphiden, Fliegen, Zikaden oder Thripse, die Pflanzenviren auf Pflanzen übertragen können. Weitere Vektoren, die Pflanzenviren übertragen können, sind Spinnmilben, Läuse, Käfer und Nematoden.

Weitere Beispiele für Vektoren im Sinne der vorliegenden Erfindung sind Insekten und Arachniden wie Mücken, insbesondere der Gattungen Aedes, Anopheles, z.B. A. gambiae, A. arabiensis, A. funestus, A. dirus (Malaria) und Culex, Läuse, Flöhe, Fliegen, Milben und Zecken, die Krankheitserreger auf Tiere und/oder Menschen übertragen können.

Eine Vektorkontrolle ist auch möglich, wenn die Verbindungen der Formel (I) Resistenz-brechend sind.

Verbindungen der Formel (I) sind zur Verwendung in der Prävention von Krankheiten bzw. vor Krankheitserregern, die durch Vektoren übertragen werden, geeignet. Somit ist ein weiterer Aspekt der vorliegenden Erfindung die Verwendung von Verbindungen der Formel (I) zur Vektorkontrolle, z.B. in der Landwirtschaft, im Gartenbau, in Forsten, in Gärten und Freizeiteinrichtungen sowie im Vorrats- und Materialschutz.

### Schutz von technischen Materialen

Die Verbindungen der Formel (I) eignen sich zum Schutz von technischen Materialien gegen Befall oder Zerstörung durch Insekten, z.B. aus der Ordnung Coleoptera, Hymenoptera, Isoptera, Lepidoptera, Psocoptera und Zygentoma.

Unter technischen Materialien sind im vorliegenden Zusammenhang nicht lebende Materialien zu verstehen, wie vorzugsweise Kunststoffe, Klebstoffe, Leime, Papiere und Kartone, Leder, Holz, Holzverarbeitungsprodukte und Anstrichmittel. Die Anwendung der Erfindung zum Schutz von Holz ist besonders bevorzugt.

In einer weiteren Ausführungsform werden die Verbindungen der Formel (I) zusammen mit mindestens einem weiteren Insektizid und/oder mindestens einem Fungizid eingesetzt.

In einer weiteren Ausführungsform liegen die Verbindungen der Formel (I) als ein anwendungsfertiges (ready-to-use) Schädlingsbekämpfungsmittel vor, d.h., es kann ohne weitere Änderungen auf das entsprechende Material aufgebracht werden. Als weitere Insektizide oder als Fungizide kommen insbesondere die oben genannten in Frage.

Überraschenderweise wurde auch gefunden, dass die Verbindungen der Formel (I) zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, verwendet werden können. Gleichfalls können die Verbindungen der Formel (I) allein oder in Kombinationen mit anderen Wirkstoffen als Antifouling-Mittel eingesetzt werden.

### Bekämpfung von tierischen Schädlingen auf dem Hygienesektor

Die Verbindungen der Formel (I) eignen sich zur Bekämpfung von tierischen Schädlingen auf dem Hygienesektor. Insbesondere kann die Erfindung im Haushalts-, Hygiene- und Vorratsschutz verwendet werden, vor allem zur Bekämpfung von Insekten, Spinnentieren und Milben, die in geschlossenen Räumen, wie beispielsweise Wohnungen, Fabrikhallen, Büros, Fahrzeugkabinen vorkommen. Zur Bekämpfung der tierischen Schädlinge werden die Verbindungen der Formel (I) allein oder in Kombination mit anderen Wirk- und/oder Hilfsstoffen verwendet. Bevorzugt werden sie in Haushaltsinsektizid-Produkten verwendet. Die Verbindungen der Formel (I) sind gegen sensible und resistente Arten sowie gegen alle Entwicklungsstadien wirksam.

Zu diesen Schädlingen gehören beispielsweise Schädlinge aus der Klasse Arachnida, aus den Ordnungen Scorpiones, Araneae und Opiliones, aus den Klassen Chilopoda und Diplopoda, aus der Klasse Insecta die Ordnung Blattodea, aus den Ordnungen Coleoptera, Dermaptera, Diptera, Heteroptera, Hymenoptera, Isoptera, Lepidoptera, Phthiraptera, Psocoptera, Saltatoria oder Orthoptera, Siphonaptera und Zygentoma und aus der Klasse Malacostraca die Ordnung Isopoda.

Die Anwendung erfolgt beispielsweise in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

### Erläuterung der Verfahren und Zwischenprodukte

Die folgenden Herstellungs- und Verwendungsbeispiele illustrieren die Erfindung, Die Produkte wurden mittels ¹H-NMR Spektroskopie und/oder LC/MS (Liquid Chromatography Mass Spectrometry) charakterisiert.

Die Bestimmung der logP Werte erfolgte gemäß OECD Guideline 117 (EC Directive 92/69/EEC) durch HPLC (High Performance Liquid Chromatography) an reversed-phase (RP) Säulen (C18), mit nachfolgenden Methoden:
[a] Die Bestimmung mit der LC-MS im sauren Bereich erfolgt bei pH 2,7 mit 0,1 % wässriger Ameisensäure und Acetonitril (enthält 0,1% Ameisensäure) als Eluenten; linearer Gradient von 10% Acetonitril bis 95% Acetonitril.
[b] Die Bestimmung mit der LC-MS im neutralen Bereich erfolgt bei pH 7.8 mit 0,001 molarer wässriger Ammoniumhydrogencarbonat-Lösung und Acetonitril als Eluenten; linearer Gradient von 10% Acetonitril bis 95% Acetonitril.

Die Eichung erfolgt mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinander folgenden Alkanonen).

Die NMR-Spektren wurden mit einem Bruker Avance 400, ausgestattet mit einem Durchflussprobenkopf (60 µl Volumen), bestimmt. In Einzelfällen wurden die NMR Spektren mit einem Avance DRX 500 oder Bruker Avance II 600 gemessen.

### NMR-Daten ausgewählter Beispiele

### NMR-Peak-Listenverfahren

Die ¹H-NMR-Daten ausgewählter Beispiele werden in Form von ¹H-NMR-Peaklisten notiert. Zu jedem Signalpeak wird erst der δ-Wert in ppm und dann die Signalintensität in runden Klammern aufgeführt. Die δ-Wert - Signalintensitäts- Zahlenpaare von verschiedenen Signalpeaks werden durch Semikolons voneinander getrennt aufgelistet.

Die Peakliste eines Beispieles hat daher die Form:
δ (Intensität₁⁾; δ₂ (Intensität₂);........; δᵢ (Intensitätᵢ⁾;......; δₙ (Intensitätₙ)

Die Intensität scharfer Signale korreliert mit der Höhe der Signale in einem gedruckten Beispiel eines NMR-Spektrums in cm und zeigt die wirklichen Verhältnisse der Signalintensitäten. Bei breiten Signalen können mehrere Peaks oder die Mitte des Signals und ihre relative Intensität im Vergleich zum intensivsten Signal im Spektrum gezeigt werden.

Zur Kalibrierung der chemischen Verschiebung von ¹H-NMR-Spektren benutzen wir Tetramethylsilan und/oder die chemische Verschiebung des Lösungsmittels, besondern im Falle von Spektren, die in DMSO gemessen werden. Daher kann in NMR-Peaklisten der Tetramethylsilan-Peak vorkommen, muss es aber nicht.

Die Listen der 1H-NMR-Peaks sind ähnlich den klassischen ¹H-NMR-Ausdrucken und enthalten somit gewöhnlich alle Peaks, die bei einer klassischen NMR-Interpretation aufgeführt werden.

Darüber hinaus können sie wie klassische ¹H-NMR-Ausdrucke Lösungsmittelsignale, Signale von Stereoisomeren der Zielverbindungen, die ebenfalls Gegenstand der Erfindung sind, und/oder Peaks von Verunreinigungen zeigen.

Bei der Angabe von Verbindungssignalen im Delta-Bereich von Lösungsmitteln und/oder Wasser sind in unseren Listen von ¹H-NMR-Peaks die gewöhnlichen Lösungsmittelpeaks, zum Beispiel Peaks von DMSO in DMSO-D₆ und der Peak von Wasser, gezeigt, die gewöhnlich im Durchschnitt eine hohe Intensität aufweisen.

Die Peaks von Stereoisomeren der Targetverbindungen und/oder Peaks von Verunreinigungen haben gewöhnlich im Durchschnitt eine geringere Intensität als die Peaks der Zielverbindungen (zum Beispiel mit einer Reinheit von >90%).

Solche Stereoisomere und/oder Verunreinigungen können typisch für das jeweilige Herstellungsverfahren sein. Ihre Peaks können somit dabei helfen, die Reproduktion unseres Herstellungsverfahrens anhand von "Nebenprodukt-Fingerabdrücken" zu erkennen.

Einem Experten, der die Peaks der Zielverbindungen mit bekannten Verfahren (MestreC, ACD-Simulation, aber auch mit empirisch ausgewerteten Erwartungswerten) berechnet, kann je nach Bedarf die Peaks der Zielverbindungen isolieren, wobei gegebenenfalls zusätzliche Intensitätsfilter eingesetzt werden. Diese Isolierung wäre ähnlich dem betreffenden Peak-Picking bei der klassischen 1H-NMR-Interpretation.

Weitere Details zu ¹H-NMR-Peaklisten können der Research Disclosure Database Number 564025 entnommen werden.

### Synthese von Verbindungen der Formel (I)

### Beispiel 1: 1,4-Dimethyl-2-[2-(pyridin-3-yl)-2H-indazol-5-yl]-1,2,4-triazolidin-3,5-dion

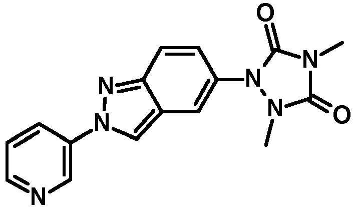

### Methode A: Kupfer-Katalyse

In einem Mikrowellen geeigneten Gefäß wurden 1,00 g (3,65 mmol) 5-Brom-2-(pyridin-3-yl)-2*H-*indazol (Herstellung vgl. WO 2016/071499 A1 und WO 2016/087421 A1), 0,49 g (3,83 mmol) 1,4-Dimethyl-1,2,4-triazolidin-3,5-dion, 115 µl (0,10 g, 0,73 mmol) *trans*-*N,N'*-Dimethylcyclohexan-1,2-diamin, 0,07 g (0,36 mmol) Kupfer(I)-iodid, 0,18 g (1,09 mmol) Kaliumiodid und 1,51 g (10,94 mmol) Kaliumcarbonat vorgelegt und mit 100 ml Toluol versetzt. Das Reaktionsgemisch wurde 60 Minuten bei 150 °C in einer Anton Paar Monowave 400 Mikrowelle gerührt, anschließend abgekühlt und unter Vakuum vom Lösungsmittel befreit.

Die Reaktion wurde zweimal wiederholt. Die jeweiligen Rückstände wurden vereint, mit Acetonitril verrührt und über Celite filtriert. Das Filtrat wurde unter Vakuum vom Lösungsmittel befreit, in Methanol aufgenommen und auf Isolute aufgezogen. Nach Aufreinigung mittels MPLC über RP-18 mit Gradienten Acetonitril/Wasser/0,1 % Ameisensäure wurden 1,18 g (100 % Reinheit nach LC/MS, 33 % der Theorie) der Titelverbindung isoliert.

### Methode B: Palladium-Katalyse

In einem 50 ml Rundkolben wurden 100 mg (0,37 mmol) 5-Brom-2-(pyridin-3-yl)-2*H*-indazol (Herstellung vgl. WO 2016/071499 A1 und WO 2016/087421 A1), 49 mg (0,38 mmol) 1,4-Dimethyl-1,2,4-triazolidin-3,5-dion, 21 mg (0,04 mmol) Tris(dibenzylideneacetone) dipalladium(0), 37 mg (0,07 mg) 5-(Di-*tert*-butylphosphino)-1-,3-,5-triphenyl-1*H*-[1,4-]bipyrazol und 357 mg (1,09 mmol) Cäsiumcarbonat vorgelegt, der Kolben wurde mit Argon geflutet und 10 ml entgaster tert-Amylalkohol wurden zugegeben. Das Reaktionsgemisch wurde über Nacht bei 100 °C gerührt, anschließend abgekühlt und unter Vakuum vom Lösungsmittel befreit. Der Rückstand wurde in Acetonitril aufgenommen und auf Isolute aufgezogen. Nach Aufreinigung mittels MPLC über RP-18 mit Gradienten Acetonitril/Wasser/0,1 % Ameisensäure wurden 69 mg (98 % Reinheit nach LC/MS, 58 % der Theorie) der Titelverbindung isoliert.
logP(HCOOH) 1,24; logP(neutral) 1,31
**¹H-NMR (400,0 MHz, d₆-DMSO**): δ = 9,35 (d,1H), 9,29 (s,1H), 8,69 (d,1H), 8,49-8,52 (m,1H), 7,87 (d,1H), 7,78 (s,1H), 7,69-7,66 (m,1H), 7,39 (dd,1H), 3,05 (s,3H), 3,03 (s,3H) ppm.

### Beispiel 2: 4-Methyl-2-[2-(pyridin-3-yl)-2H-indazol-5-yl]-1,2,4-triazin-3,5(2H,4H)-dion

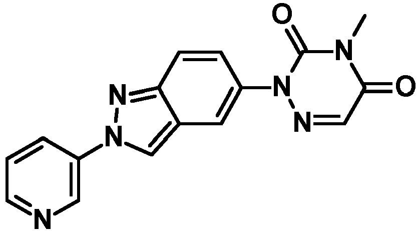

### Methode A: Kupfer-Katalyse

In einem 50 ml Rundkolben wurden 600 mg (2,19 mmol) 5-Brom-2-(pyridin-3-yl)-2H-indazol (Herstellung vgl. WO 2016/071499 A1 und WO 2016/087421 A1), 292 mg (2,30 mmol) 4-Methyl-1,2,4-triazin-3,5(2*H*,4*H*)-dion, 70 µl (62 mg, 0,44 mmol) *trans*-*N,N*'-Dimethylcyclohexan-1,2-diamin, 42 mg (0,22 mmol) Kupfer(I)-iodid, 109 mg (0,66 mmol) Kaliumiodid und 908 mg (6,66 mmol) Kaliumcarbonat vorgelegt. Der Kolben wurde mit Argon geflutet und 20 ml Toluol wurden zugegeben. Das Reaktionsgemisch wurde über Nacht bei Rückfluß gerührt, anschließend abgekühlt und unter Vakuum vom Lösungsmittel befreit. Der Rückstand wurde in Acetonitril aufgenommen, über Celite filtriert und das Filtrat unter Vakuum vom Lösungsmittel befreit. Das Rohgemisch wurde in Acetonitril suspendiert und auf Isolute aufgezogen. Nach Aufreinigung mittels MPLC über RP-18 mit Gradienten Acetonitril/Wasser/0,1 % Ameisensäure wurden 30 mg (97 % Reinheit nach LC/MS, 4 % der Theorie) der Titelverbindung isoliert.
logP(HCOOH) 1,38; logP(neutral) 1,47
**¹H-NMR (400,0 MHz, d₆-DMSO**): δ = 9,50-9,34 (m,2H), 8,72-8,65 (m,1H), 8,52 (d,1H), 7,92 (d,1H), 7,84 (d,1H), 7,76 (s,1H), 7,74-7,65 (m,1H), 7,43 (dd,1H), 3,24 (s,3H) ppm.

Weitere Verbindungen der Formel (I) sind in der folgenden Tabelle 1 und zugehörige analytische Daten in der Tabelle 2 aufgeführt.

**Tabelle 1**

| | |
|---|---|
| Verbindungen der Formel **(I)** | |
| | 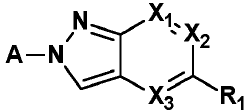 |

| **Beispiel-Nr.** | **Struktur** |
|---|---|
| **3** | 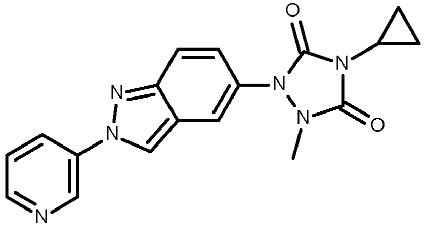 |
| **4** | 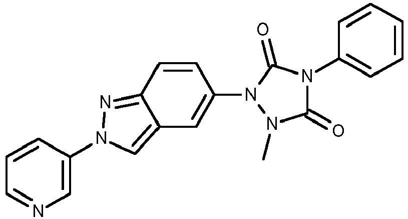 |
| **5** | 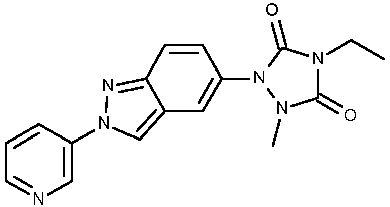 |
| **6** | 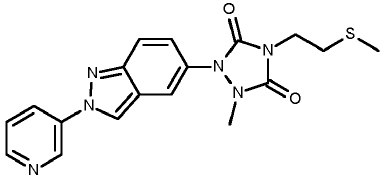 |
| **7** | 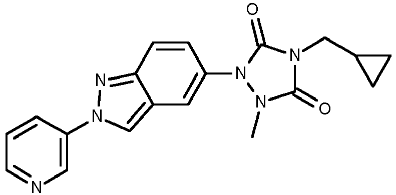 |
| **8** | 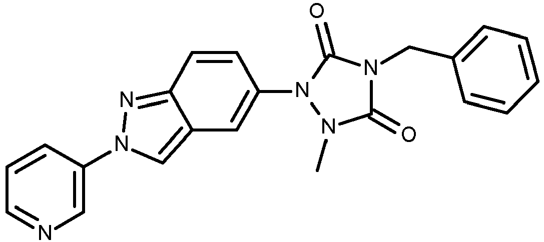 |
| **9** | 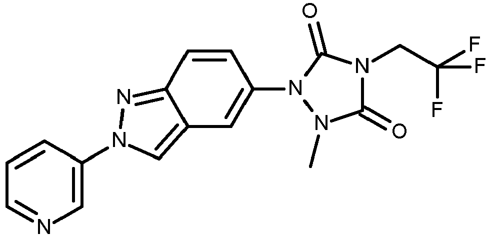 |
| **10** | 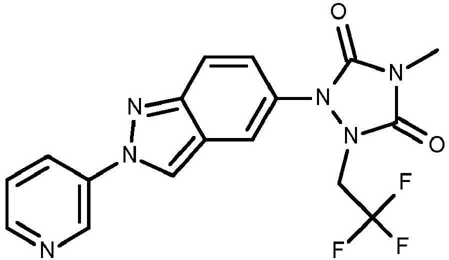 |
| **11** | 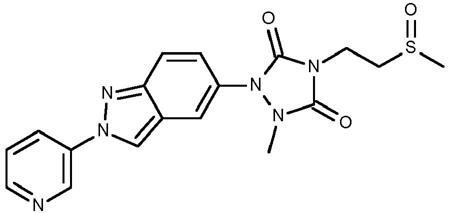 |
| **12** | 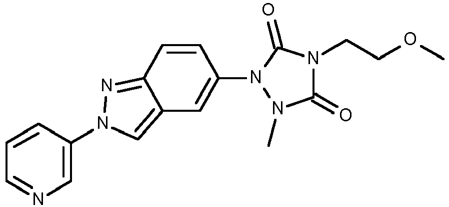 |
| **13** | 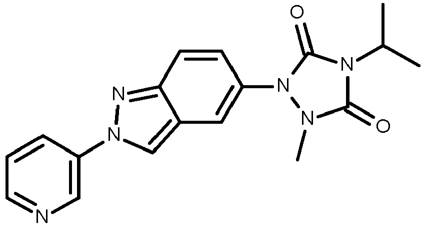 |
| **14** | 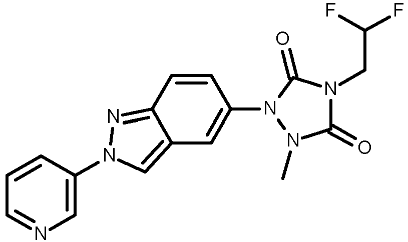 |
| **15** | 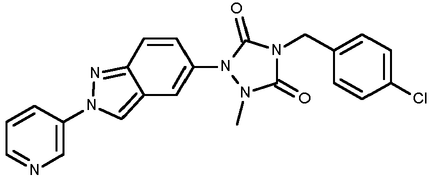 |
| **16** | 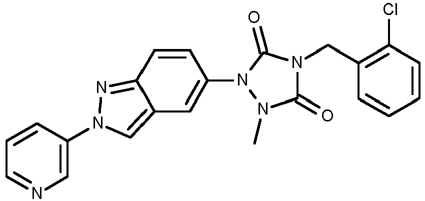 |
| **17** | 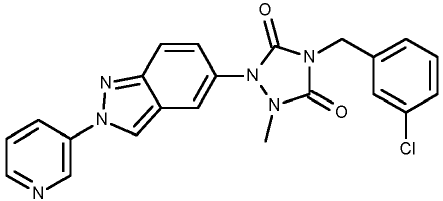 |
| **18** | 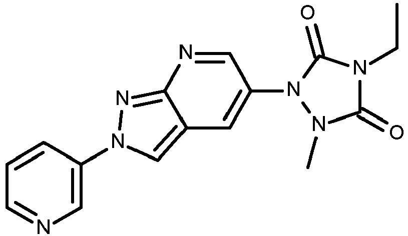 |
| **19** | 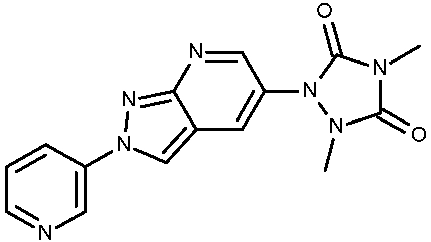 |
| **20** | 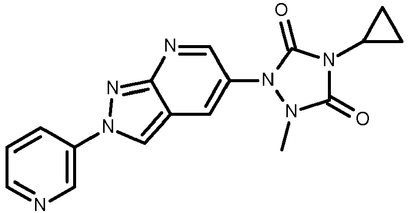 |
| **21** | 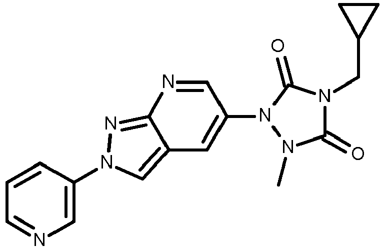 |
| **22** | 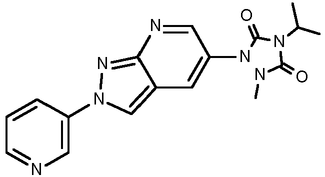 |
| **23** | 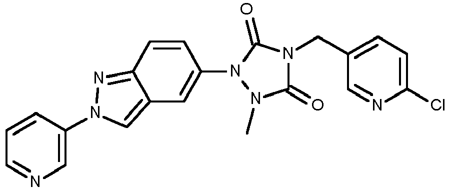 |
| **24** | 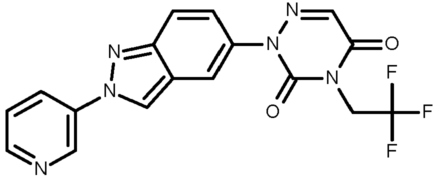 |
| **25** | 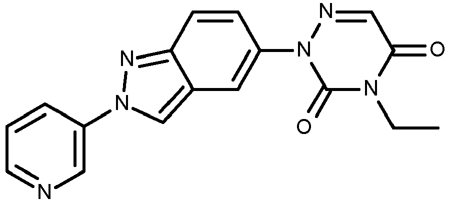 |
| **26** | 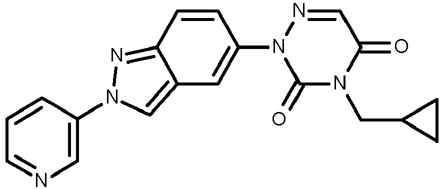 |
| **27** | 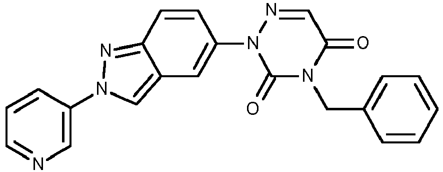 |
| **28** | 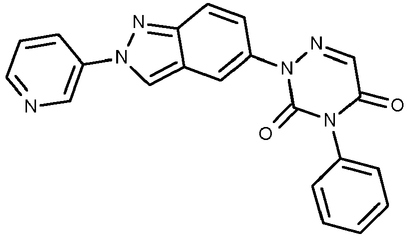 |
| **29** | 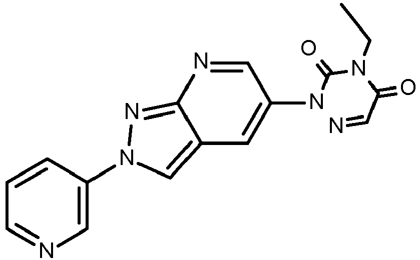 |

**Tabelle 2**

| | |
|---|---|
| ¹H-NMR-Daten der Verbindungen 3 bis 29 | |

| **Beispiel-Nr.** | **¹H-NMR-Daten [σ (ppm)]^{a}** |
|---|---|
| **3** | 9.3431(2.4);9.3366(2.4);9.2801(4.3);8.6871(1.6);8.6843(1.7);8.675 4(1.7);8.6725(1.7);8.5167(0.9);8.5109(1.0);8.5070(0.9);8.4957(1.0) ;8.4899(1.1);8.4863(0.9);7.8693(2.0);7.8461(2.1);7.7585(2.6);7.754 2(2.6);7.6836(1.3);7.6717(1.3);7.6628(1.3);7.6508(1.2);7.3856(1.7) ;7.3808(1.6);7.3625(1.6);7.3576(1.5);3.3191(21.4);3.0081(16.0);2.7 360(0.3);2.7265(0.7);2.7187(0.8);2.7154(0.7);2.7094(1.3);2.7000(1. 0);2.6920(0.8);2.6814(0.5);2.6705(0.6);2.6667(0.5);2.5058(82.9);2. 5 016(108.7);2.4973(79.3);2.3326(0.5);2.3282(0.6);0.9520(0.4);0.93 08(2.8);0.9209(1.7);0.9120(1.4);0.9054(1.6);0.8953(2.0);0.8873(1.5 );0.8817(1.6);0.8630(0.4);0.1458(0.3);0.0075(2.8);-0.0002(69.8);-0.0079(3.0);-0.1496(0.4) |
| **4** | 9.3575(2.6);9.3515(2.7);9.3252(4.5);8.6931(1.9);8.6842(1.9);8.681 3(1.9);8.5281(1.2);8.5256(1.2);8.5072(1.2);8.5044(1.3);7.9203(2.2) ;7.9095(2.9);7.9050 |
| | (3.2);7.8976(2.6);7.6927(1.4);7.6803(1.4);7.6718(1.4);7.6599(1.3); 7.5701(0.8);7.5553(10.1);7.5495(5.9);7.5401(4.5);7.5193(1.0);7.50 95(1.9);7.5050(1.8);7.4861(1.6);7.4815(1.8);7.4738(0.9);7.4676(1.2 );7.4597(1.1);7.4521(1.2);7.4375 |
| | (0.6);7.4309(0.4);3.3224(69.9);3.1446(16.0);2.6702(0.7);2.5056(87. 9);2.5015 (114.2);2.4974(90.6);2.3285(0.7);0.1456(0.4);-0.0004(77.5);-0.1499(0.4) ppm |
| **5** | 9.3770(2.1);9.3709(2.2);9.3037(4.0);8.7094(1.6);8.7002(1.6);8.697 5(1.6);8.5765(1.0);8.5740(1.0);8.5528(1.0);7.8826(1.9);7.8596(2.1) ;7.7937(2.5);7.7902 |
| | (2.7);7.7326(1.2);7.7207(1.1);7.7119(1.1);7.6999(1.1);7.4037(1.7); 7.3988(1.7);7.3806(1.5);7.3757(1.6);4.3450(2.0);3.5749(0.9);3.556 8(3.1);3.5388(3.2);3.5208(1.0);3.0511(16.0);2.6762(0.4);2.6716(0.5 );2.6673(0.4);2.5436(1.0);2.5295(2.4);2.5070(78.2);2.5026(102.5);2 .4983(77.9);2.3339(0.4);2.3295(0.6);1.2210(3.4);1.2031(7.4);1.185 1(3.3);0.0078(1.9);-0.0001(49.1);-0.0080(2.5) |
| **6** | 9.3465(2.3);9.3404(2.5);9.2927(3.8);9.2099(0.6);8.6879(1.7);8.676 3(1.6);8.6608(0.3);8.5182(1.0);8.4945(1.1);8.3152(0.3);7.8920(1.8) ;7.8688(2.0);7.8071(2.6);7.8038(2.6);7.7876(0.3);7.7488(0.3);7.686 4(1.2);7.6747(1.2);7.6665(1.4);7.6540(1.3);7.3845(1.6);7.3799(1.6) ;7.3616(1.5);7.3565(1.5);5.7559(0.5);3.8127(0.3);3.7400(1.7);3.723 0(3.7);3.7061(1.8);3.3203(285.5);3.0593(14.1);2.8084 |
| | (1.9);2.7915(3.8);2.7747(1.7);2.6703(2.5);2.5056(325.2);2.5013(42 5.6);2.4971(327.5);2.3282(2.6);2.0991(16.0);0.1458(0.6);0.0076(5. 3);-0.0001(128.0); -0.1499(0.6) |
| **7** | 9.3487(2.2);9.3425(2.2);9.2901(3.7);9.2884(3.8);8.6906(1.6);8.687 2(1.7);8.6788(1.6);8.6754(1.7);8.5234(0.9);8.5199(1.0);8.5170(1.0) ;8.5133(0.9);8.5026(1.0);8.4990(1.0);8.4961(1.1);8.4925(0.9);7.886 7(1.9);7.8636(2.1);7.8102(2.4);7.8070(2.4);7.6855(1.2);7.6737(1.2) ;7.6656(1.1);7.6528(1.1);7.4003(1.8);7.3953(1.7);7.3773(1.6);7.372 2(1.6);3.3953(3.7);3.3774(3.8);3.3216(44.8);3.0674(16.0);2.6711(0. 4);2.5242(1.6);2.5109(25.5);2.5065(50.1);2.5020(65.6);2.4975(48.2 );2.4931(23.9);2.3289(0.4);1.1614(0.5);1.1542(0.5);1.1509(0.4);1.1 423(0.8);1.1306(0.5);1.1228(0.5);0.5272(0.6);0.5161(1.8);0.5117(2. 0);0.5013(1.0);0.4960(1.8);0.4916(1.8);0.4815(0.7);0.3591(0.8);0.3 482(2.3);0.3371(2.0);0.3332(2.3);0.3217(0.5);0.0079(1.6);-0.0002(38.8);-0.0084(1.6) |
| **8** | 9.3466(2.3);9.3403(2.3);9.2920(4.1);8.6895(1.6);8.6867(1.7);8.678 0(1.7);8.6748(1.7);8.5209(0.9);8.5175(1.1);8.5146(1.1);8.5111(0.9) ;8.5002(1.1);8.4967(1.2);8.4939(1.2);8.4903(1.0);8.1362(0.7);7.887 4(2.0);7.8643(2.3);7.8253(2.6);7.8218(2.8);7.6843(1.3);7.6726(1.3) ;7.6633(1.3);7.6519(1.3);7.4059(2.2);7.4013(2.3);7.3832(4.0);7.378 2(2.6);7.3671(7.9);7.3636(7.0);7.3476(1.4);7.3383(1.2);7.3340(1.0) ;7.3250(0.8);7.3168(1.3);7.3078(0.5);7.3010(0.5);4.7050(7.2);3.324 2(24.1);3.0828(16.0);2.6715(0.3);2.5069(43.4);2.5026(56.5);2.4982 (41.8);2.3293(0.3);2.0751(1.4);0.0079(0.9);-0.0001(19.3) |
| **9** | 9.3130(3.6);8.5397(1.4);8.5196(1.5);7.9084(2.1);7.8847(2.6);7.878 2(3.5);7.8750(3.5);7.7823(0.4);7.4064(1.6);7.4018(1.8);7.3832(1.5) ;7.3788(1.6);5.7562(1.5);4.4198(0.9);4.3964(2.9);4.3733(3.0);4.350 1(1.0);3.3223(44.9);3.0903(16.0);2.6721(0.8);2.5030(148.1);2.3295 (0.8);-0.0003(37.5) |
| **10** | 9.3478(1.9);9.3421(1.9);9.3306(4.6);8.6940(1.4);8.6830(1.4);8.522 3(0.9);8.5190(1.1);8.5163(1.1);8.5129(1.0);8.5016(1.0);8.4980(1.1) ;8.4953(1.2);8.4919(1.0);7.8898(2.8);7.8857(2.8);7.8736(2.1);7.850 5(2.2);7.6899(1.2);7.6781(1.2);7.6692(1.2);7.6574(1.1);7.3689(1.7) ;7.3641(1.7);7.3460(1.6);7.3410(1.6);5.7558(0.9);4.4471(0.8);4.424 4(2.4);4.4016(2.5);4.3786(0.8);3.3208(71.5);3.0663(16.0);2.6748(0. 6);2.6707(0.8);2.6662(0.6);2.5059(100.5);2.5016(131.6);2.4972(97. 0);2.3329(0.6);2.3283(0.7);2.3240(0.6);0.0077(2.7);-0.0003(66.6); -0.0083(3.0) |
| **11** | 9.3478(2.5);9.3416(2.6);9.2985(4.4);8.6901(1.8);8.6878(1.8);8.678 6(1.8);8.6760(1.8);8.5173(1.1);8.5000(1.0);8.4945(1.2);7.8859(2.0) ;7.8627(2.2);7.8055(2.8);7.8015(2.8);7.6861(1.3);7.6742(1.3);7.665 3(1.3);7.6534(1.2);7.4017(1.7);7.3969(1.7);7.3785(1.6);7.3737(1.6) ;5.7547(5.6);3.9529(1.0);3.9362(2.2);3.9259(2.2);3.9201 (1.3);3.909 4(1.0);3.3231(4.0);3.2075(0.5);3.1911(1.0);3.1743(1.1);3.1577(1.4) ;3.1414(0.6);3.0487(15.4);3.0208(1.5);3.0042(1.1);2.9874(1.0);2.97 10(0.4);2.6720(0.4);2.6176(16.0);2.5069(49.0);2.5027(61.4);2.4987 (46.7);2.3295(0.4);-0.0002(56.3) |
| **12** | 9.3481(2.3);9.3417(2.3);9.2892(4.2);8.6897(1.6);8.6870(1.6);8.678 0(1.7);8.6752(1.6);8.5217(0.9);8.5183(1.1);8.5155(1.0);8.5123(0.8) ;8.5011(1.0);8.4975(1.1);8.4947(1.1);8.4913(0.8);8.1664(1.2);7.886 0(2.0);7.8628(2.2);7.8000(2.6);7.7958(2.6);7.6852(1.2);7.6734(1.2) ;7.6644(1.2);7.6526(1.2);7.3853(1.7);7.3803(1.6);7.3622(1.6);7.357 2(1.5);3.7011(1.4);3.6870(3.6);3.6733(2.4);3.5890(2.6);3.5754(4.0) ;3.5614(1.5);3.3343(0.7);3.3097(0.7);3.3026(0.6);3.2682(19.6);3.23 15(0.3);3.0568(16.0);2.5073(33.9);2.5030(43.4);2.4986(31.8);-0.0002(0.6) |
| **13** | 9.3481(2.7);9.3425(2.8);9.2839(4.5);9.2111(0.4);8.6865(1.9);8.676 5(2.0);8.5182(1.2);8.5155(1.2);8.4972(1.4);8.1786(0.8);7.8768(2.2) ;7.8538(2.4);7.7794(3.1);7.7767(3.1);7.6842(1.3);7.6725(1.4);7.663 8(1.4);7.6520(1.3);7.3867(1.8);7.3821(1.7);7.3637(1.7);7.3589(1.6) ;4.2839(0.4);4.2670(1.0);4.2497(1.4);4.2324(1.1);4.2156(0.4);3.344 1(0.4);3.3362(0.4);3.3185(0.4);3.3104(0.4);3.0336(16.0);2.5030(50. 2);1.4144(14.3);1.3971(14.2);-0.0002(0.8) |
| **14** | 9.3498(2.2);9.3434(2.2);9.3061(3.9);8.6933(1.5);8.6902(1.6);8.681 7(1.6);8.6784(1.6);8.5245(0.8);8.5209(1.0);8.5181(0.9);8.5145(0.8) ;8.5036(0.9);8.5001(1.0);8.4973(1.0);8.4937(0.9);7.9012(1.8);7.878 0(2.0);7.8419(2.4);7.8380(2.4);7.6877(1.2);7.6760(1.2);7.6669(1.2) ;7.6550(1.1);7.4015(1.7);7.3965(1.6);7.3785(1.5);7.3734(1.5);6.426 4(0.6);6.2983(0.6);6.2895(1.2);6.2808(0.6);6.1526(0.6);3.9948(0.8) ;3.9861(0.9);3.9563(1.9);3.94 76(1.9);3.9177(1.0);3.9089(0.9);3.321 9(10.2);3.0800(16.0);2.5245(0.9);2.5112(18.0);2.5069(35.6);2.5024 (46.6);2.4980(34.2);2.4937(17.0);2.0864(0.3);-0.0002(0.9) |
| **15** | 9.3463(2.4);9.3400(2.4);9.2932(4.2);8.6898(1.7);8.6866(1.7);8.678 1(1.7);8.6748(1.7);8.5204(0.9);8.5169(1.1);8.5139(1.0);8.5105(0.9) ;8.4996(1.0);8.4961(1.1);8.4931(1.2);8.4896(1.0);7.8859(2.0);7.862 8(2.2);7.8227(2.6);7.8192(2.7);7.6846(1.3);7.6726(1.3);7.6636(1.3) ;7.6518(1.2);7.4578(2.8);7.4529(1.1);7.4415(1.6);7.4365(6.1);7.401 2(6.4);7.3795(4.1);7.3746(2.1);4.6988(6.7);3.3218(35.0);3.0778(16. 0);2.6756(0.3);2.6717(0.4);2.6668(0.3);2.5245(1.6);2.5110(29.4);2. 5069(56.6);2.5024(72.9);2.4980(53.6);2.4940(27.0);2.3338(0.3);2.3 292(0.4);2.3248(0.3);-0.0002(6.8) |
| **16** | 9.3508(2.4);9.3444(2.5);9.3045(4.2);8.6922(1.7);8.6891 (1.8);8.680 6(1.8);8.6773(1.8);8.5248(1.0);8.5212(1.1);8.5184(1.0);8.5150(0.9) ;8.5040(1.0);8.5004(1.1);8.4976(1.1);8.4942(0.9);7.9017(2.0);7.878 6(2.3);7.8565(2.6);7.8523(2.7);7.6873(1.3);7.6755(1.3);7.6665(1.3) ;7.6547(1.3);7.5196(0.8);7.5148(1.1);7.5099(1.0);7.5027(1.4);7.495 6(1.4);7.4374(1.9);7.4325(1.8);7.4143(1.7);7.4094(1.7);7.3741(6.9) ;7.3673(4.7);4.7906(7.2);3.3225(31.6);3.1073(16.0);2.6759(0.3);2.6 713(0.4);2.6671(0.3);2.5108(28.6);2.5067(54.1);2.5023(69.5);2.497 9(51.4);2.3292(0.4);1.3975(5.9);1.2346(0.9);-0.0002(6.5) |
| **17** | 9.3436(1.9);9.3393(1.9);9.2901(3.5);9.2888(3.5);8.6861(1.4);8.683 9(1.4);8.6783(1.4);8.6760(1.4);8.5134(0.8);8.5111(0.9);8.5091(0.9) ;8.5067(0.8);8.4996(0.9);8.4972(0.9);8.4953(1.0);8.4928(0.9);7.882 8(1.7);7.8675(1.9);7.8336(2.0);7.8314(2.1);7.6762(1.0);7.6685(1.0) ;7.6624(1.0);7.6546(1.0);7.4400(2.0);7.4321(0.7);7.4190(1.8);7.407 4(3.1);7.4046(2.0);7.4022(1.3);7.3992(2.0);7.3963(1.3);7.3926(1.7) ;7.3892(2.0);7.3857(0.6);7.3320(1.4);7.3200(1.0);5.7502(5.5);4.715 7(5.8);3.3066(33.7);3.0859(16.0);2.6128(0.4);2.5220(0.7);2.5189(0. 8);2.5158(0.8);2.5070(20.1);2.5040(44.0);2.5010(62.0);2.4980(44.4 );2.4949(20.4);2.3851(0.4);1.3980(1.2);1.2346(0.5);-0.0002(4.6) |
| **18** | 9.3781(1.7);9.3718(1.8);9.3493(5.6);8.7786(2.9);8.7723(3.0);8.725 7(1.3);8.7164(1.3);8.7140(1.3);8.5643(0.8);8.5609(0.9);8.5579(0.9) ;8.5543(0.8);8.5435(0.9);8.5400(1.0);8.5370(1.0);8.5334(0.8);8.328 3(3.0);8.3220(3.0);7.7146(1.1);7.7029(1.1);7.6939(1.1);7.6820(1.0) ;3.5888(0.9);3.5708(3.0);3.5528(3.1);3.5348(1.0);3.3224(5.5);3.079 2(16.0);2.5252(0.5);2.5114(13.8);2.5072(28.6);2.5028 (38.3);2.4983(27.6);2.4940(13.3);1.2341(3.4);1.2161(7.4);1.1981(3. 2);-0.0002(9.5);-0.0084(0.4) |
| **19** | 9.4006(1.9);9.3613(5.2);8.7822(3.3);8.7760(3.3);8.7458(1.4);8.734 3(1.4);8.5985(0.9);8.5791(0.9);8.3216(3.4);8.3154(3.2);8.1367(0.4) ;7.7491(0.9);7.7371(1.0);7.7281(0.9);7.7160(0.8);4.6244(0.7);3.075 1(16.0);3.0338(14.9);2.6714(0.5);2.5065(75.0);2.5026(93.0);2.4989 (68.2);2.3291(0.5);-0.0001(34.9) |
| **20** | 9.3739(1.8);9.3678(1.9);9.3422(5.6);8.7597(3.1);8.7534(3.2);8.723 6(1.4);8.7117(1.4);8.5598(0.8);8.5563(1.0);8.5534(1.0);8.5499(0.8) ;8.5389(0.9);8.5354(1.0);8.5326(1.0);8.5290(0.9);8.2906(3.3);8.284 3(3.2);7.7133(1.2);7.7014(1.1);7.6923(1.1);7.6807(1.1);3.3224(6.2) ;3.0370(16.0);2.7459(0.3);2.7363(0.7);2.7285(0.8);2.7239(0.7);2.71 91(1.2);2.7098(1.0);2.7017(0.7);2.6919(0.4);2.5247(0.7);2.5070(34. 1);2.5026(45.2);2.4982(32.6);0.9648(0.5);0.9490(2.2);0.9439(2.8);0 .9335(1.6);0.9210(1.4);0.9142(1.5);0.9060(2.1);0.8960(1.4);0.8915( 1.5);0.8737(0.4);0.0077(0.6);-0.0002(18.7);-0.0083(0.7) |
| **21** | 9.3799(2.2);9.3736(2.3);9.3518(5.7);8.7839(3.1);8.7777(3.2);8.726 5(1.6);8.7175(1.6);8.7148(1.6);8.5663(0.9);8.5630(1.0);8.5573(0.9) ;8.5455(1.0);8.5420(1.1);8.5397(1.1);8.5362(0.9);8.3532(3.2);8.346 9(3.1);7.7158(1.3);7.7039(1.3);7.6950(1.2);7.6830(1.2);3.4093(4.0) ;3.3914(4.1);3.3227(15.8);3.0980(16.0);2.5069(39.9);2.5027(51.9); 2.4985(37.8);1.1692(0.5);1.1620(0.5);1.1502(0.8);1.1382(0.5);1.13 11(0.6);0.5354(0.6);0.5240(2.0);0.5199(2.2);0.5094(1.1);0.5041(2.0 );0.4999(2.0);0.4898(0.8);0.3697(0.8);0.3585(2.6);0.3440(2.5);0.33 24(0.6);-0.0002(1.0) |
| **22** | 9.3459(5.3);8.7703(2.2);8.7650(2.2);8.5620(1.2);8.5421(1.3);8.316 7(3.3);8.3105(3.1);7.7072(0.7);4.2926(0.4);4.2755(1.0);4.2583(1.4) ;4.2410(1.1);4.2238(0.4);3.3241 (27.6);3.0622(16.0);2.6713(0.4);2.5 069(49.6);2.5028(62.6);2.4987(46.4);2.3291(0.3);1.4223(14.1);1.40 50(13.9);-0.0001(44.2) |
| **23** | 9.3454(2.2);9.3392(2.2);9.2960(4.1);8.6903(1.6);8.6875(1.6);8.678 5(1.6);8.6757(1.6);8.5202(0.9);8.5167(1.0);8.5142(1.0);8.5104(0.9) ;8.4991(1.0);8.4928(1.1);8.4533(2.3);8.4473(2.2);8.1388(0. 7);7.952 8(0.7);7.8854(2.0);7.8778(1.4);7.8716(1.4);7.8623(2.3);7.8574(1.8) ;7.8509(1.4);7.8247(2.6);7.8204(2.6);7.6855(1.3);7.6734(1.3);7.664 7(1.3);7.6530(1.2);7.5590(2.7);7.5385(2.4);7.4104(1.8);7.4055(1.6) ;7.3874(1.6);7.3823(1.6);4.7578(6.6);3.3218(60.7);3.0712(16.0);2.9 792(0.3);2.8905(4.7);2.7310(4.2);2.6893(16.0);2.6752(0.9);2.6706( 1.2);2.6663(0.8);2.5239(2.8);2.5102(73.7);2.5061(150.5);2.5017(19 9.4);2.4972(142.8);2.4931(68.5);2.4450(0.3);2.3325(0.8);2.3282(1. 1);2.3240(0.8);1.2349(0.4);-0.0002(2.2) |
| **24** | 9.3511(15.4);8.6937(4.0);8.6907(4.4);8.6820(4.2);8.6789(4.4);8.52 99(2.3);8.5264(2.7);8.5235(2.7);8.5201(2.4);8.5091(2.5);8.5055(2.8 );8.5027(2.9);8.4992(2.4);7.9599(6.6);7.9563(6.7);7.8980(16.0);7.8 723(5.0);7.8492(5.6);7.6900(3.2);7.6782(3.2);7.6693(3.2);7.6574(3. 0);7.4423(4.5);7.4374(4.5);7.4192(4.0);7.4143(4.1);5.7560(1.7);4.7 396(1.9);4.7171(6.1);4.6944(6.4);4.6717(2.2);3.3221(44.3);2.6758( 0.5);2.6716(0.6);2.6673(0.5);2.5069(80.6);2.5025(105.0);2.4981(79 .5);2.3336(0.4);2.3293(0.6);2.3249(0.5);2.0864(0.4);2.0749(0.4);1.2 324(0.4);-0.0002(1.8) |
| **25** | 9.3594(4.4);9.3535(4.5);9.3381(9.8);9.2109(0.5);8.6891(3.3);8.677 5(3.2);8.5296(2.0);8.5261(2.4);8.5233(2.3);8.5200(2.0);8.5088(2.3) ;8.5052(2.6);8.5025(2.6);8.4991(2.1);7.9435(5.9);7.9399(6.1);7.849 3(4.5);7.8262(5.1);7.7541(14.6);7.6878(2.7);7.6760(2.7);7.6670(2.8 );7.6552(2.7);7.4528(4.2);7.4479(4.1);7.4297(3.7);7.4248(3.8);3.91 77(1.9);3.9001(6.3);3.8823(6.4);3.8647(2.0);3.3244(9.2);2.6729(0.4 );2.5079(48.9);2.5035(63.0);2.4991(47.1);2.3303(0.4);1.2298(0.4);1 .2151(7.2);1.1974(16.0);1.1796(7.0);1.1103(0.4);-0.0002(0.6) |
| **26** | 9.3588(6.3);9.3525(6.3);9.3396(10.5);8.6906(4.3);8.6875(4.5);8.67 89(4.5);8.6757(4.5);8.5312(2.4);8.5277(2.8);8.5249(2.7);8.5213(2.4 );8.5104(2.6);8.5068(2.8);8.5039(2.9);8.5004(2.4);7.9611(6.7);7.95 78(6.6);7.8531(5.1);7.8300(5.7);7.7854(16.0);7.6878(3.3);7.6760(3. 2);7.6670(3.2);7.6551(3.1);7.4599(4.7);7.4550(4.6);7.4368(4.2);7.4 319(4.2);3.7598(9.0);3.7421(9.0);3.3237(48.2);2.6763(0.4);2.6719( 0.6);2.6672(0.4);2.5072(69.4);2.5028(88.5);2.4984(65.3);2.3335(0. 4);2.3296(0.5);2.3251(0.4);2.0750(1.0);1.2394(0.4);1.2331(0.7);1.2 208(1.3);1.2136(1.3);1.2019(2.1);1.1901(1.3);1.1825(1.4);1.1708(0. 7);1.1644(0.5);0.5188(1.5);0.5073(4.7);0.5029(5.5);0.4929(3.1);0.4 873(4.8);0.4829(5.0);0.4731(2.1);0.4361(0.3);0.3948(2.2);0.3831(6. 3);0.3731(5.5);0.3691(5.8);0.3574(1.4);0.0076(0.7);-0.0002(15.4);-0.0083(0.6) |
| **27** | 9.3535(5.2);9.3472(5.4);9.3354(9.8);9.3343(9.7);8.6881(3.6);8.685 1(3.8);8.6764(3.8);8.6733(3.8);8.5260(2.1);8.5224(2.5);8.5195(2.4) ;8.5160(2.2);8.5052(2.4);8.5016(2.6);8.4986(2.7);8.4951(2.2);7.960 8(6.1);7.9574(6.0);7.8489(4.7);7.8347(16.0);7.8258(5.5);7.6849(2.9 );7.6730(2.9);7.6640(2.9);7.6522(2.8);7.4603(4.3);7.4553(4.2);7.43 72(3.9);7.4322(4.0);7.4168(4.8);7.3991(8.6);7.3670(4.3);7.3622(1.6 );7.3495(8.6);7.3306(4.6);7.3082(3.5);7.2969(1.2);7.2905(3.7);7.28 35(0.8);7.2727(1.0);5.0441(14.3);3.3208(59.9);2.6753(0.7);2.6709( 1.0);2.6665(0.7);2.5239(3.2);2.5063(123.6);2.5018(160.6);2.4974(1 19.3);2.3331(0.7);2.3286(1.0);2.3242(0.8);2.0858(0.4);1.3974(2.4); 1.2335(1.0);0.0079(0.6);-0.0002(15.7) |
| **28** | 9.4821(0.4);9.4684(0.4);9.4625(0.4);9.3422(11.2);8.8049(0.4);8.79 54(0.4);8.7146(0.6);8.5232(4.6);8.5031(4.7);8.3154(0.5);7.9790(9.8 );7.9546(0.8);7.9054 |
| | (16.0);7.8818(1.9);7.8634(6.5);7.8404(7.0);7.6857(2.7);7.6358(0.7) ;7.6084(1.1);7.5509(4.6);7.5331(11.7);7.5140(11.3);7.5027(8.6);7.4 803(10.2);7.4659(7.2);7.4469(3.9);7.4294(13.4);7.4105(10.5);7.331 1(1.0);7.3123(0.9);7.2653(0.4);7.2447(0.4);7.2344(0.4);3.6078(0.3) ;3.5910(0.3);3.4585(0.8);3.3507(1.7);3.2142(0.7);3.1180(0.4);3.106 4(0.3);2.9816(0.5);2.9638(0.5);2.9183(0.5);2.8902(1.6);2.7317(1.6) ;2.6707(1.6);2.5019(249.1);2.4064(0.7);2.3287(1.6);2.0736(0.8);1.5 439(0.4);1.2584(0.4);1.2362(0.4);0.9812(0.4);0.9627(0.6);0.9467(0. 4);0.1453(0.3);-0.0003(59.4);-0.1498(0.3) |
| **29** | 9.8282(3.3);9.4005(12.8);9.3761(3.1);8.7994(6.1);8.7933(6.3);8.72 90(2.6);8.7179(2.6);8.5591(2.5);8.5390(2.6);8.4646(7.8);8.4584(7.4 );8.3188(1.4);8.3126 (1.6);8.2483(1.6);8.2420(1.4);7.8293(14.1);7.7188(2.6);7.7073(2.9) ;7.6985(3.2);7.6868(3.3);5.7571(2.5);3.9255(2.0);3.9078(6.3);3.890 0(6.4);3.8716(2.0);3.3214(119.4);2.6742(1.7);2.6706(2.2);2.5799(0. 5);2.5628(0.5);2.5058(292.1);2.5016(374.0);2.4973(271.4);2.3286( 2.1);2.3244(1.6);1.2220(7.2);1.2042(16.0);1.1866(7.1);1.1709(0.3); -0.0002(10.8) |

| | |
|---|---|
| ^{a 1}H-NMR (400.0 MHz, d₆-DMSO) für die Beispiele 3-16 und 18-29. ¹H-NMR (601.6 MHz, d₆-DMSO) für Beispiel 17. | |

### Synthese von Verbindungen der Formel (B'-1)

### (B'-1)-1

### 4-(Cyclopropylmethyl)-1-methyl-1,2,4-triazolidin-3,5-dion

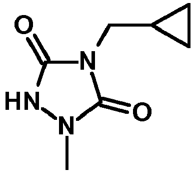

### Reaktionsschritt 1 Ethyl-1-methylhydrazincarboxylat

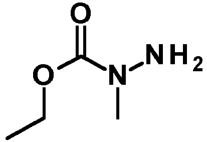

200 ml einer 50 %igen wässrigen Methylhydrazin-Lösung wurden auf -10 °C abgekühlt. Dazu wurde portionsweise eine Lösung aus 62,6 g (577 mmol) Ethylcarbonochloridat in 200 ml Dichlormethan gegeben, sodass die Temperatur unter 0 °C gehalten wurde. Das Reaktionsgemisch wurde über Nacht bei Raumtemperatur gerührt, anschließend mit gesättigter Natriumchlorid-Lösung versetzt und gegen Dichlormethan extrahiert (3 x 200 ml). Die organischen Phasen wurden vereint, über Natriumsulfat getrocknet, filtriert und unter Vakuum vom Lösungsmittel befreit. Nach Destillation unter Vakuum wurden 60,0 g (88 % der Theorie) der Titelverbindung isoliert.

### Reaktionsschritt 2 4-(Cyclopropylmethyl)-1-methyl-1,2,4-triazolidin-3,5-dion

Zu einer Lösung aus 6,08 g (51,5 mmol) Ethyl-1-methylhydrazincarboxylat (vgl. Reaktionsschritt 1) in 150 ml Dichlormethan wurden 5,00 g (51,5 mmol) (Isocyanatomethyl)cyclopropan gegeben und das Reaktionsgemisch wurde über Nacht bei Raumtemperatur gerührt. Unter Vakuum wurden alle flüchtigen Bestandteile entfernt. Der Rückstand wurde mit einer gesättigten methanolischen Natriummethylat-Lösung (hergestellt aus 2,37 g Natrium und 100 ml Methanol) versetzt und 6 Stunden unter Rückfluß erhitzt. Nach Abkühlen wurden bei Raumtemperatur 12 ml konzentrierte Salzsäure zugegeben. Die Lösung wurde unter Vakuum vom Lösungsmittel befreit. Der Rückstand wurde in Essigsäureethylester aufgenommen und bis zur Rückflusstemperatur erhitzt. Die Suspension wurde heiß filtriert und anschließend das Lösungsmittel unter Vakuum vom Filtrat entfernt. Der resultierende Rückstand wurde über Kieselgel chromatographiert und man erhielt 4,80 g (55 % der Theorie nach 2 Reaktionsschritten) der Titelverbindung.
**¹H-NMR (400,0 MHz, d₆-DMSO**): δ = 10,40 (bs,1H), 3,28-3,15 (bd,2H), 3,05 (bs,3H), 1,10-0,98 (m,1H), 0,50-0,40 (m,2H), 0,30-0,20 (m,2H) ppm.

### (B'-1)-2

### 1-Methyl-4-(2,2,2-trifluorethyl)-1,2,4-triazolidin-3,5-dion

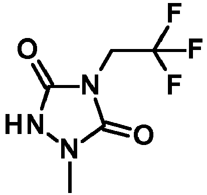

Ein Gemisch aus 5,00 g (42,3 mmol) Ethyl-1-methylhydrazincarboxylat, 9,53 g (42,3 mmol) 2,2,2-Trifluorethyl-(2,2,2-trifluorethyl)carbamat und 50 ml *N,N*-Diisopropylethylamin (Hünig-Base) wurde 48 Stunden unter Rückfluß erhitzt und anschließend unter Vakuum von flüchtigen Bestandteilen befreit. Der Rückstand wurde mit einer methanolischen Natriummethylat-Lösung (hergestellt aus 1,95 g Natrium und 100 ml Methanol) versetzt und 6 Stunden unter Rückfluß erhitzt. Nach Abkühlen wurden 10 ml konzentrierte Salzsäure bei Raumtemperatur zugegeben. Die Lösung wurde unter Vakuum vom Lösungsmittel befreit. Der Rückstand wurde in Essigsäureethylester aufgenommen und bis zur Rückflusstemperatur erhitzt. Die Suspension wurde heiß filtriert und anschließend das Lösungsmittel unter Vakuum vom Filtrat entfernt. Der resultierende Rückstand wurde über Kieselgel chromatographiert. Es wurden 5,20 g (62% der Theorie nach 2 Reaktionsschritten) der Titelverbindung isoliert.
**¹H-NMR (500,0 MHz, d₆-DMSO**): δ = 10,80 (bs,1H), 4,18 (q,2H), 3,05 (s,3H) ppm.

### (B'-1)-3

### 4-Methyl-1-(2,2,2-trifluorethyl)-1,2,4-triazolidin-3,5-dion

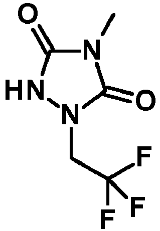

Zu einer Suspension aus 15,5 g (134 mmol) 4-Methyl-1,2,4-triazolidin-3,5-dion in 150 ml Tetrahydrofuran wurden 28,1 ml (202 mmol) Triethylamin und 33,4 g (161 mmol) 2,2,2-Trifluorethyltrifluormethansulfonat gegeben und das resultierende Gemisch wurde 48 Stunden bei Raumtemperatur gerührt. Anschließend wurde es mit 300 ml Chloroform verdünnt und filtriert. Das Filtrat wurde unter Vakuum vom Lösungsmittel befreit. Der Rückstand wurde über Kieselgel chromatographiert. Es wurden 2,20 g (8% der Theorie) der Titelverbindung isoliert.
**¹H-NMR (500,0 MHz, CDCl₃):** δ = 7,98 (bs,1H), 4,12 (q,2H), 3,13 (s,3H) ppm.

### (B'-1)-4

### 1-Methyl-4-(2,2-difluorethyl)-1,2,4-triazolidin-3,5-dion

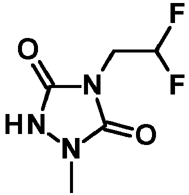

Zu einer auf -25 °C abgekühlten Lösung aus 10,0 g (123 mmol) 2,2-Difluor-ethanamin und 52 ml (373 mmol) Triethylamin in 100 ml Dichlormethan wurden 33,5 g (148 mmol) Bis(2,2,2-trifluorethyl)carbonat gegeben. Das resultierende Gemisch wurde über Nacht bei Raumtemperatur gerührt. Die flüchtigen Bestandteile wurden anschließend unter Vakuum entfernt. Der verbleibende Rückstand wurde mit 50 ml *N,N*-Diisopropylethylamin (Hünig-Base) und 14,6 g (124 mmol) Ethyl-1-methylhydrazincarboxylat versetzt und 48 Stunden unter Rückfluß erhitzt. Nach Abkühlen wurden alle flüchtigen Bestandteile unter Vakuum entfernt. Der Rückstand wurde mit einer methanolischen Natriummethylat-Lösung (hergestellt aus 7,00 g Natrium und 100 ml Methanol) versetzt und 4 Stunden unter Rückfluß erhitzt. Nach Abkühlen wurde der pH-Wert des Gemisches mit konzentrierter Salzsäure sauer eingestellt. Die Lösung wurde unter Vakuum vom Lösungsmittel befreit, der verbleibende Rückstand in Ethylessigester aufgenommen und bis zur Rückflusstemperatur erhitzt. Die Suspension wurde heiß filtriert und anschließend das Lösungsmittel unter Vakuum vom Filtrat entfernt. Der resultierende Rückstand wurde über Kieselgel chromatographiert und man erhielt 5,30 g (24 % der Theorie nach 2 Stufen) der Titelverbindung.
**¹H-NMR (400,0 MHz, d₆-DMSO**): δ = 8,62 (bs,1H), 6,03 (ttd,1H), 3,89 (ttd,2H) ppm, 3,22 (s,3H) ppm.

In Analogie zu einer den weiter oben beschriebenen Synthesen wurden die Verbindungen der Formeln (B'-1)-5 bis (B'-1)-10 hergestellt:

### (B'-1)-5

### 4-(2-Methoxyethyl)-1-methyl-1,2,4-triazolidin-3,5-dion

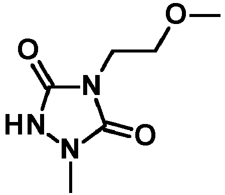

**¹H-NMR (400,0 MHz, d₆-DMSO**): δ = 10,42 (s,1H), 3,55-3,45 (m,4H), 3,21 (s,3H), 3,00 (s,3H) ppm.

### (B'-1)-6

### 4-(4-Chlorbenzyl)-1-methyl-1,2,4-triazolidin-3,5-dion

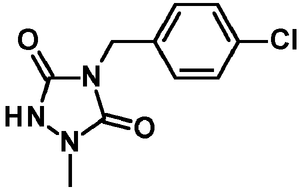

**¹H-NMR (400,0 MHz, d₆-DMSO**): δ = 10,61 (s,1H), 7,40 (d,2H), 7,29 (d,2H), 4,55 (s,2H), 3,04 (s,3H) ppm.

### (B'-1)-7

### 4-(3-Chlorbenzyl)-1-methyl-1,2,4-triazolidin-3,5-dion

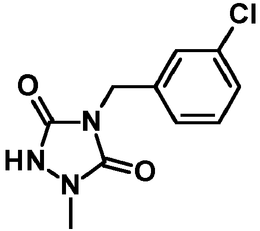

**¹H-NMR (400,0 MHz, d₆-DMSO**): δ = 10,63 (bs,1H), 7,40-7,20 (m,4 H), 4,56 (s,2H), 3,04 (s,3H) ppm.

### (B'-1)-8

### 4-(2-Chlorbenzyl)-1-methyl-1,2,4-triazolidin-3,5-dion

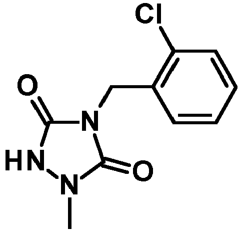

**¹H-NMR(400,0 MHz, d₆-DMSO**): δ = 10,68 (bs,1H), 7,50-7,44 (m,1H), 7,37-7,30 (m,2H), 7,20-7,14 (m,1H), 4,63 (s,2H), 3,06 (s,3H) ppm.

### (B'-1)-9

### 4-Benzyl-1-methyl-1,2,4-triazolidin-3,5-dion

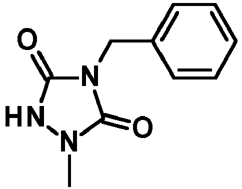

**¹H-NMR(400,0 MHz, d₆-DMSO**): δ = 10,55 (bs,1H), 7,40-7,20 (m,5H), 4,55 (s,2H), 3,04 (s,3H) ppm.

### (B'-1)-10

### 1-Methyl-4-[2-(methylsulfanyl)ethyl]-1,2,4-triazolidin-3,5-dion

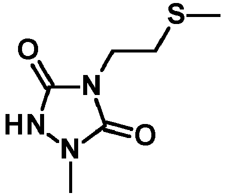

**¹H-NMR(400,0 MHz, d₆-DMSO**): δ = 8,53 (bs,1H), 3,70 (t,2H), 3,19 (t,3H), 2,78 (t,2H), 2,15 (s,3H) ppm.

### Synthese von Verbindungen der Formel (B'-2)

### (B'-2)-1

### 4-(2,2,2-Trifluorethyl)-1,2,4-triazin-3,5(2H,4H)-dion

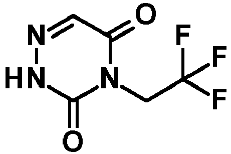

Zu einer Lösung von 5,00 g (32,2 mmol) 2-Acetyl-1,2,4-triazin-3,5(2*H*,4*H*)-dion in 30 ml *N,N-*Dimethylformamid (DMF) wurden portionsweise 1,42 g (35,5 mmol) Natriumhydrid 60 %ig in Mineralöl gegeben. Das Reaktionsgemisch wurde 30 Minuten bei Raumtemperatur gerührt und anschließend auf 0 °C abgekühlt. Danach wurden 7,85 g (33.8 mmol) Trifluoroethyltriflat in 5 ml DMF hinzugetropft und das Reaktionsgemisch wurde über Nacht bei Raumtemperatur gerührt. Anschließend wurde das Lösungsmittel unter Vakuum entfernt, der verbleibende Rückstand in Ethanol aufgenommen, mit 0,56 g (3,22 mmol) *para*-Toluolsulfonsäure versetzt und 2 Stunden unter Rückfluß erhitzt. Nach Entfernung des Lösungsmittels unter Vakuum wurde das Rohgemisch über Kieselgel chromatographiert, man erhielt 2,14 g (95 %ige Reinheit, 34 % der Theorie) der Titelverbindung.
**¹H-NMR (400,0 MHz, d₆-DMSO**): δ = 12,75 (s,1H), 7,45 (s,1H), 4,53 (q,2H) ppm.

### (B'-1)-2

### 4-(2,2,2-Trifluorethyl)-1,2,4-triazin-3,5(2H,4H)-dion

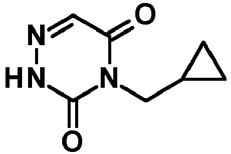

Analog zu der weiter oben beschriebenen Synthese wurden aus 5,00 g (32,2 mmol) 2-Acetyl-1,2,4-triazin-3,5(2*H*,4*H*)-dion 2,83 g (95 %ige Reinheit, 50% der Theorie) der Titelverbindung isoliert.
**¹H-NMR (400,0 MHz, d₆-DMSO**): δ = 12,53 (s,1H), 7,38 (s,1H), 3,65 (d,2H), 1,21-1,12 (m,1H), 0,50-0,30 (m,4H) ppm.

### Biologische Beispiele

### Meloidogyne incognita - Test

| | |
|---|---|
| Lösungsmittel: | 125,0 Gewichtsteile Aceton |

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser auf die gewünschte Konzentration.

Gefäße werden mit Sand, Wirkstofflösung, einer Ei-Larven-Suspension des südlichen Wurzelgallenälchens (*Meloidogyne incognita*) und Salatsamen gefüllt. Die Salatsamen keimen und die Pflänzchen entwickeln sich. An den Wurzeln entwickeln sich die Gallen.

Nach 14 Tagen wird die nematizide Wirkung anhand der Gallenbildung in % bestimmt. Dabei bedeutet 100 %, dass keine Gallen gefunden wurden; 0 % bedeutet, dass die Zahl der Gallen an den behandelten Pflanzen der unbehandelten Kontrolle entspricht.

Bei diesem Test zeigten z. B. die folgenden Verbindungen der Herstellungsbeispiele Wirkung von 90 % bei einer Aufwandmenge von 20 ppm: 1.

### Myzus persicae - Oraltest

| | |
|---|---|
| Lösungsmittel: | 100 Gewichtsteile Aceton |

Zur Herstellung einer zweckmäßigen Wirkstoffzubereitung löst man 1 Gewichtsteil Wirkstoff mit den angegebenen Gewichtsteilen Lösungsmittel und füllt mit Wasser bis zum Erreichen der gewünschten Konzentration auf.

50 µl der Wirkstoffzubereitung werden in Mikrotiterplatten überführt und mit 150 µl IPL41Insektenmedium (33% + 15% Zucker) auf eine Endvolumen von 200 µl aufgefüllt. Anschließend werden die Platten mit Parafilm verschlossen, durch den eine gemischte Population der Grünen Pfirsichblattlaus *(Myzus persicae),* die sich in einer zweiten Mikrotiterplatte befindet, hindurchstechen und die Lösung aufnehmen kann.

Nach 5 Tagen wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigten z. B. die folgenden Verbindungen der Herstellungsbeispiele Wirkung von 100 % bei einer Aufwandmenge von 4 ppm: 1, 2, 3, 4, 5, 7, 8, 9, 10, 11, 12, 14, 15, 17, 18, 20, 21, 22, 23, 24, 25, 26, 27.

Bei diesem Test zeigten z. B. die folgenden Verbindungen der Herstellungsbeispiele Wirkung von 90 % bei einer Aufwandmenge von 4 ppm: 6, 13, 16, 19, 28.

### Myzus persicae - Sprühtest

| | |
|---|---|
| Lösungsmittel: | 78 Gewichtsteile Aceton |
| | 1,5 Gewichtsteile Dimethylformamid |
| Emulgator: | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Chinakohlblattscheiben (*Brassica pekinensis*)*,* die von allen Stadien der Grünen Pfirsichblattlaus (*Myzus persicae*) befallen sind, werden mit einer Wirkstoffzubereitung der gewünschten Konzentration gespritzt.

Nach 5-6 Tagen wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigten z. B. die folgenden Verbindungen der Herstellungsbeispiele Wirkung von 100 % bei einer Aufwandmenge von 500 g/ha: 3, 6, 8, 18, 19, 21, 23, 25.

Bei diesem Test zeigten z. B. die folgenden Verbindungen der Herstellungsbeispiele Wirkung von 90 % bei einer Aufwandmenge von 500 g/ha: 1, 2, 4, 5, 7, 9, 11, 12, 14, 20, 26, 27.

### Phaedon cochleariae - Sprühtest

| | |
|---|---|
| Lösungsmittel: | 78,0 Gewichtsteile Aceton |
| | 1,5 Gewichtsteile Dimethylformamid |
| Emulgator: | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Chinakohlblattscheiben (*Brassica pekinensis*) werden mit einer Wirkstoffzubereitung der gewünschten Konzentration gespritzt und nach dem Abtrocknen mit Larven des Meerrettichblattkäfers *(Phaedon cochleariae)* besetzt.

Nach 7 Tagen wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigten z. B. die folgenden Verbindungen der Herstellungsbeispiele Wirkung von 83% bei einer Aufwandmenge von 500 g/ha: 4, 25.

## Patentansprüche

1. Verbindungen der Formel (I) 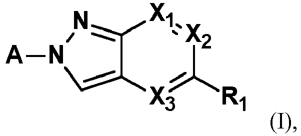 in welcher
A für einen A-Rest aus der Reihe (A-b) bis (A-f) 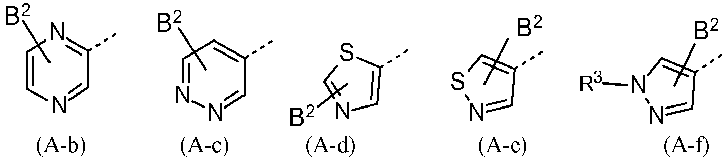 steht, worin die gestrichelte Linie die Bindung zum Kohlenstoffatom des Bicyclus der Formel (I) bedeutet und
B² für einen Rest aus der Reihe Wasserstoff, Halogen, Cyano, Nitro, Alkyl, Halogenalkyl, Alkoxy, Halogenalkoxy und jeweils gegebenenfalls substituiertes Cycloalkyl und Cycloalkenyl steht,
X₁ für Stickstoff (N) oder CH steht,
X₂ für Stickstoff oder CH steht,
X₃ für Stickstoff oder CH steht, wobei entweder keine oder nur eine der Variablen X₁, X₂ und X₃ für Stickstoff steht,
R₁ für einen B-Rest aus der Reihe 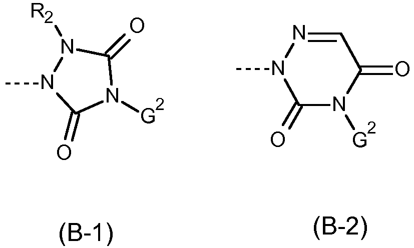 steht, worin die gestrichelte Linie die Bindung zum Kohlenstoffatom des Bicyclus der Formel (I) bedeutet,
R₂ für einen Rest aus der Reihe Wasserstoff, Alkyl, Halogenalkyl, Alkenyl, Alkinyl, Alkoxyalkyl, Alkyl-S-alkyl, Alkyl-S(O)-alkyl, Alkyl-S(O)₂-alkyl, Cycloalkyl, Halogencycloalkyl und Cycloalkenyl steht,
G² für einen Rest aus der Reihe Wasserstoff, Alkyl, Halogenalkyl, Alkenyl, Alkinyl, Alkoxyalkyl, Alkyl-S-alkyl, Alkyl-S(O)-alkyl, Alkyl-S(O)₂-alkyl, Cycloalkyl, Halogencycloalkyl, Cycloalkenyl, Halogencycloalkenyl, Cycloalkylalkyl, Cycloalkenylalkyl, Halogencycloalkenylalkyl und jeweils gegebenenfalls substituiertes Aryl, Arylalkyl, Pyridyl und Pyridylalkyl steht, und Verbindungen der Formel (I), in welcher
A für den Rest (A-a) 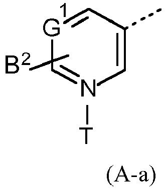 steht, worin die gestrichelte Linie die Bindung zum Kohlenstoffatom des Bicyclus der Formel (I) bedeutet und
G¹ für N oder C-B¹ steht,
B¹ für einen Rest aus der Reihe Wasserstoff, Halogen, Cyano, Nitro, Alkyl, Halogenalkyl, Alkoxy, Halogenalkoxy und jeweils gegebenenfalls substituiertes Cycloalkyl und Cycloalkenyl steht,
B² für einen Rest aus der Reihe Wasserstoff, Halogen, Cyano, Nitro, Alkyl, Halogenalkyl, Alkoxy, Halogenalkoxy und jeweils gegebenenfalls substituiertes Cycloalkyl und Cycloalkenyl steht,
T für Sauerstoff oder ein Elektronenpaar steht,
X₁ für Stickstoff oder CH steht,
X₂ für Stickstoff oder CH steht,
X₃ für Stickstoff oder CH steht, wobei entweder keine oder nur eine der Variablen X₁, X₂ und X₃ für Stickstoff steht,
R₁ für einen der folgenden B-Reste 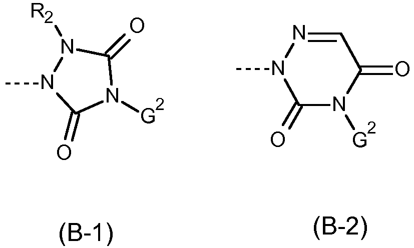 steht, worin die gestrichelte Linie die Bindung zum Kohlenstoffatom des Bicyclus der Formel (I) bedeutet, worin
R₂ für einen Rest aus der Reihe Wasserstoff, Alkyl, Halogenalkyl, Alkenyl, Alkinyl, Alkoxyalkyl, Alkyl-S-alkyl, Alkyl-S(O)-alkyl, Alkyl-S(O)₂-alkyl, Cycloalkyl, Halogencycloalkyl und Cycloalkenyl steht,
G² für einen Rest aus der Reihe Wasserstoff, Alkyl, Halogenalkyl, Alkenyl, Alkinyl, Alkoxyalkyl, Alkyl-S-alkyl, Alkyl-S(O)-alkyl, Alkyl-S(O)₂-alkyl, Cycloalkyl, Halogencycloalkyl, Cycloalkenyl, Halogencycloalkenyl, Cycloalkylalkyl, Cycloalkenylalkyl, Halogencycloalkenylalkyl und jeweils gegebenenfalls substituiertes Aryl, Arylalkyl, Pyridyl und Pyridylalkyl steht.

2. Verbindungen der Formel (I) gemäss Anspruch 1, in welchen
A für einen der folgenden A-Reste 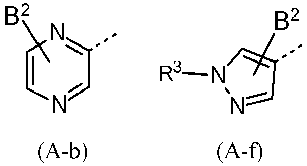 steht, worin die gestrichelte Linie die Bindung zum Kohlenstoffatom des Bicyclus der Formel (I) bedeutet, und
B² für einen Rest aus der Reihe Wasserstoff, Halogen, C₁-C₆-Alkyl und C₁-C₄-Halogenalkyl steht,
X₁ für CH steht,
X₂ für CH steht,
X₃ für CH steht,
R₁ für einen der folgenden B-Reste 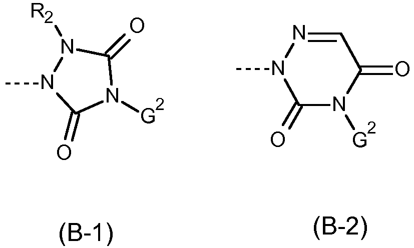 steht, worin die gestrichelte Linie die Bindung zum Kohlenstoffatom des Bicyclus der Formel (I) bedeutet, worin
R₂ für Wasserstoff oder C₁-C₆-Alkyl steht,
G² für einen Rest aus der Reihe Wasserstoff, C₁-C₆-Alkyl, Halogen-C₁-C₆-alkyl, C₃-C₆-Cycloalkyl, Halogen-C₃-C₆-cycloalkyl, C₃-C₆-Cycloalkenyl, Halogen-C₃-C₆-cycloalkenyl, Phenyl oder Benzyl (wobei Phenyl und Benzyl selbst wiederum substituiert sein können durch Halogen, Cyano, Nitro, C₁-C₆₋Alkyl, Halogen-C₁-C₆-alkyl und C₃-C₆-Cycloalkyl), Pyridyl oder Pyridylmethyl (wobei Pyridyl und Pyridylmethyl selbst wiederum substituiert sein können durch Halogen, Cyano, Nitro, C₁-C₆₋Alkyl, Halogen-C₁-C₆-alkyl und C₃-C₆-Cycloalkyl), steht, und
Verbindungen der Formel (I), in welchen
A für den folgenden A-Rest 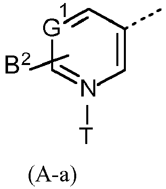 steht, worin die gestrichelte Linie die Bindung zum Kohlenstoffatom des Bicyclus der Formel (I) bedeutet, und
G¹ für N oder C-B¹ steht,
B¹ für einen Rest aus der Reihe Wasserstoff, Halogen, C₁-C₆-Alkyl und C₁-C₄-Halogenalkyl steht,
B² für einen Rest aus der Reihe Wasserstoff, Halogen, C₁-C₆-Alkyl und C₁-C₄-Halogenalkyl steht,
T für Sauerstoff oder ein Elektronenpaar steht,
X₁ für CH steht,
X₂ für CH steht,
X₃ für CH steht,
R₁ für einen der folgenden B-Reste 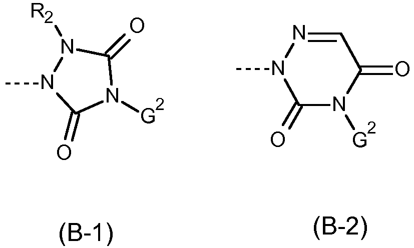 steht, worin die gestrichelte Linie die Bindung zum Kohlenstoffatom des Bicyclus der Formel (I) bedeutet, worin
R₂ für Wasserstoff oder C₁-C₆-Alkyl steht,
G² für einen Rest aus der Reihe Wasserstoff, C₁-C₆-Alkyl, Halogen-C₁-C₆-alkyl, C₃-C₆-Cycloalkyl, Halogen-C₃-C₆-cycloalkyl, C₃-C₆-Cycloalkenyl, Halogen-C₃-C₆-cycloalkenyl, Phenyl oder Benzyl (welche selbst wiederum substituiert sein können durch Halogen, Cyano, Nitro, C₁-C₆₋Alkyl, Halogen-C₁-C₆-alkyl und C₃-C₆-Cycloalkyl), Pyridyl oder Pyridylmethyl (welche selbst wiederum substituiert sein können durch Halogen, Cyano, Nitro, C₁-C₆₋Alkyl, Halogen-C₁-C₆-alkyl und C₃-C₆-Cycloalkyl), steht.

3. Verbindungen der Formel (I) gemäss Anspruch 2, worin
A für einen A-Rest aus der Reihe (A-b) und (A-f) 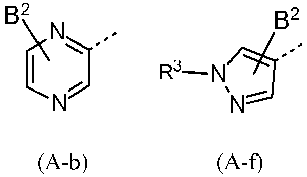 steht, worin die gestrichelte Linie die Bindung zum Kohlenstoffatom des Bicyclus der Formel (I) bedeutet,
B² für einen Rest aus der Reihe Wasserstoff, Halogen, C₁-C₆-Alkyl und C₁-C₄-Halogenalkyl steht,
X₁ für CH steht,
X₂ für CH steht,
X₃ für CH steht,
R₁ für einen B-Rest aus der Reihe 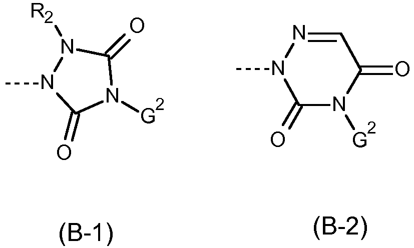 steht, worin die gestrichelte Linie die Bindung zum Kohlenstoffatom des Bicyclus der Formel (I) bedeutet, worin
R₂ für Wasserstoff oder C₁-C₆-Alkyl steht,
G² für einen Rest aus der Reihe Wasserstoff, C₁-C₆-Alkyl, Halogen-C₁-C₆-alkyl, C₃-C₆-Cycloalkyl, Halogen-C₃-C₆-cycloalkyl, C₃-C₆-Cycloalkenyl und Halogen-C₃-C₆-cycloalkenyl steht, und Verbindungen der Formel (I), worin
A für den A-Rest 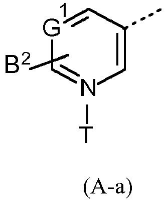 steht, worin die gestrichelte Linie die Bindung zum Kohlenstoffatom des Bicyclus der Formel (I) bedeutet,
G¹ für N oder C-B¹ steht,
B¹ für einen Rest aus der Reihe Wasserstoff, Halogen, C₁-C₆-Alkyl und C₁-C₄-Halogenalkyl steht,
B² für einen Rest aus der Reihe Wasserstoff, Halogen, C₁-C₆-Alkyl und C₁-C₄-Halogenalkyl steht,
T für Sauerstoff oder ein Elektronenpaar steht,
X₁ für CH steht,
X₂ für CH steht,
X₃ für CH steht,
R₁ für einen B-Rest aus der Reihe 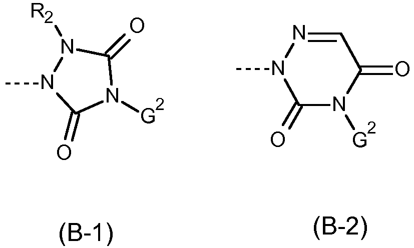 steht, worin die gestrichelte Linie die Bindung zum Kohlenstoffatom des Bicyclus der Formel (I) bedeutet, worin
R₂ für Wasserstoff oder C₁-C₆-Alkyl steht,
G² für einen Rest aus der Reihe Wasserstoff, C₁-C₆-Alkyl, Halogen-C₁-C₆-alkyl, C₃-C₆-Cycloalkyl, Halogen-C₃-C₆-cycloalkyl, C₃-C₆-Cycloalkenyl und Halogen-C₃-C₆-cycloalkenyl steht.

4. Verbindungen der Formel (I) gemäss Anspruch 1, in welchen
A für einen der folgenden A-Reste 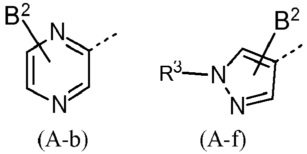 steht, worin die gestrichelte Linie die Bindung zum Kohlenstoffatom des Bicyclus der Formel (I) bedeutet,
B² für Wasserstoff steht,
X₁ für CH steht,
X₂ für CH steht,
X₃ für CH steht,
R₁ für einen B-Rest aus der Reihe 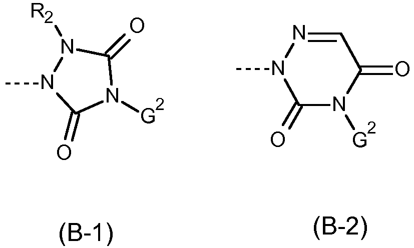 steht, worin die gestrichelte Linie die Bindung zum Kohlenstoffatom des Bicyclus der Formel (I) bedeutet, worin
R₂ für Wasserstoff oder C₁-C₆-Alkyl steht,
G² für einen Rest aus der Reihe Wasserstoff, C₁-C₆-Alkyl, Halogen-C₁-C₆-alkyl und C₃-C₆-Cycloalkyl steht, und
Verbindungen der Formel (I), in welchen
A für den A-Rest 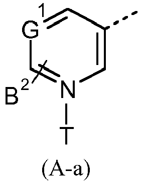 steht, worin die gestrichelte Linie die Bindung zum Kohlenstoffatom des Bicyclus der Formel (I) bedeutet,
G¹ für N oder C-B¹ steht,
B¹ für einen Rest aus der Reihe Wasserstoff und Fluor steht,
B² für Wasserstoff steht,
T für Sauerstoff oder ein Elektronenpaar steht,
X₁ für CH steht,
X₂ für CH steht,
X₃ für CH steht,
R₁ für einen B-Rest aus der Reihe 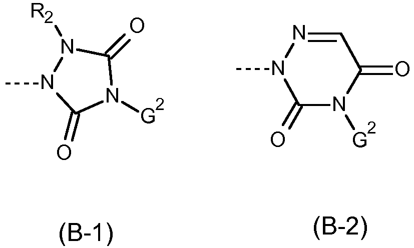 steht, worin die gestrichelte Linie die Bindung zum Kohlenstoffatom des Bicyclus der Formel (I) bedeutet, worin
R₂ für Wasserstoff oder C₁-C₆-Alkyl steht,
G² für einen Rest aus der Reihe Wasserstoff, C₁-C₆-Alkyl, Halogen-C₁-C₆-alkyl und C₃-C₆-Cycloalkyl steht.

5. Verbindungen der Formel (I) gemäss Anspruch 1, in welchen
A für 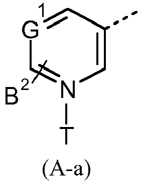 steht, worin die gestrichelte Linie die Bindung zum Kohlenstoffatom des Bicyclus der Formel (I) bedeutet,
G¹ für C-B¹ steht,
B¹ für Wasserstoff oder Fluor steht,
B² für Wasserstoff steht,
T für ein Elektronenpaar steht,
X₁ für CH steht,
X₂ für CH steht,
X₃ für CH steht,
R₁ für den Rest (B-1) 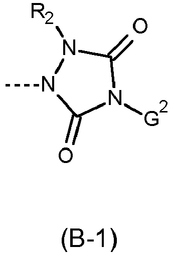 steht, worin die gestrichelte Linie die Bindung zum Kohlenstoffatom des Bicyclus der Formel (I) bedeutet, worin
R₂ für Wasserstoff oder C₁-C₆-Alkyl steht und
G² für einen Rest aus der Reihe Wasserstoff, C₁-C₆-Alkyl, Halogen-C₁-C₆-alkyl und C₃-C₆-Cycloalkyl steht.

6. Verbindungen der Formel (I) gemäss Anspruch 1, in welchen
A für den A-Rest 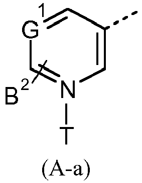 steht, worin die gestrichelte Linie die Bindung zum Kohlenstoffatom des Bicyclus der Formel (I) bedeutet,
G¹ für N oder C-B¹ steht,
B¹ für Wasserstoff oder Fluor steht,
B² für Wasserstoff steht,
T für ein Elektronenpaar steht,
X₁ für CH steht,
X₂ für CH steht,
X₃ für CH steht,
R₁ für den Rest (B-2) 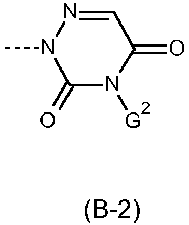 steht, worin die gestrichelte Linie die Bindung zum Kohlenstoffatom des Bicyclus der Formel (I) bedeutet, worin
G² für einen Rest aus der Reihe Wasserstoff, C₁-C₆-Alkyl, Halogen-C₁-C₆-alkyl und C₃-C₆-Cycloalkyl steht.

7. Verbindungen der Formel (I) gemäss Anspruch 1, in welchen
A für einen der folgenden A-Reste 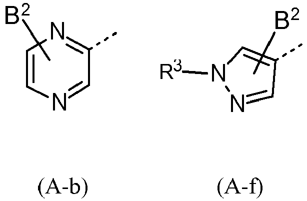 steht, worin die gestrichelte Linie die Bindung zum Kohlenstoffatom des Bicyclus der Formel (I) bedeutet, und
B² für einen Rest aus der Reihe Wasserstoff, Halogen, C₁-C₆-Alkyl und C₁-C₄-Halogenalkyl steht,
X₁ für CH steht,
X₂ für CH steht,
X₃ für CH steht,
R₁ für einen der folgenden B-Reste 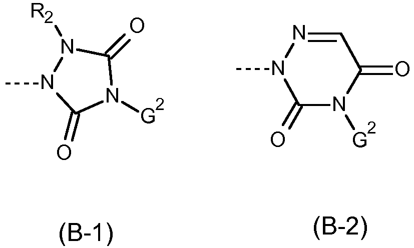 steht, worin die gestrichelte Linie die Bindung zum Kohlenstoffatom des Bicyclus der Formel (I) bedeutet, worin
R₂ für Wasserstoff oder C₁-C₆-Alkyl steht,
G² für einen Rest aus der Reihe Wasserstoff, C₁-C₆-Alkyl, Halogen-C₁-C₆-alkyl, C₃-C₆-Cycloalkyl, Halogen-C₃-C₆-cycloalkyl, C₃-C₆-Cycloalkenyl, Halogen-C₃-C₆-cycloalkenyl, Phenyl oder Benzyl (wobei Phenyl und Benzyl selbst wiederum substituiert sein können durch Halogen, Cyano, Nitro, C₁-C₆₋Alkyl, Halogen-C₁-C₆-alkyl und C₃-C₆-Cycloalkyl), Pyridyl oder Pyridylmethyl (wobei Pyridyl und Pyridylmethyl selbst wiederum substituiert sein können durch Halogen, Cyano, Nitro, C₁-C₆₋Alkyl, Halogen-C₁-C₆-alkyl und C₃-C₆-Cycloalkyl), steht, und
Verbindungen der Formel (I), in welchen
A für den folgenden A-Rest 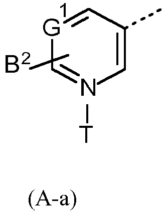 steht, worin die gestrichelte Linie die Bindung zum Kohlenstoffatom des Bicyclus der Formel (I) bedeutet, und
G¹ für N oder C-B¹ steht,
B¹ für einen Rest aus der Reihe Wasserstoff, Halogen, C₁-C₆-Alkyl und C₁-C₄-Halogenalkyl steht,
B² für einen Rest aus der Reihe Wasserstoff, Halogen, C₁-C₆-Alkyl und C₁-C₄-Halogenalkyl steht,
T für Sauerstoff oder ein Elektronenpaar steht,
X₁ für N oder CH steht,
X₂ für CH steht,
X₃ für CH steht,
R₁ für einen der folgenden B-Reste 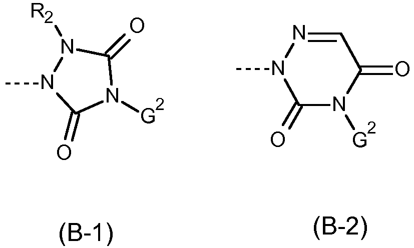 steht, worin die gestrichelte Linie die Bindung zum Kohlenstoffatom des Bicyclus der Formel (I) bedeutet, worin
R₂ für Wasserstoff, C₁-C₆-Alkyl oder Halogen-C₁-C₆-alkyl steht, und
G² für einen Rest aus der Reihe Wasserstoff, C₁-C₆-Alkyl, Halogen-C₁-C₆-alkyl, C₁-C₆-Alkoxy-C₁-C₆-alkyl, C₁-C₆-Alkyl-S-C₁-C₆-alkyl, C₁-C₆-Alkyl-S(O)-C₁-C₆-alkyl, C₁-C₆-Alkyl-S(O₂)-C₁-C₆-alkyl, C₃-C₆-Cycloalkyl, C₃-C₆-Cycloalkyl-C₁-C₆-alkyl, Halogen-C₃-C₆-cycloalkyl, C₃-C₆-Cycloalkenyl, Halogen-C₃-C₆-cycloalkenyl, Phenyl oder Benzyl (wobei Phenyl und Benzyl selbst wiederum substituiert sein können durch Halogen, Cyano, Nitro, C₁-C₆₋Alkyl, Halogen-C₁-C₆-alkyl und C₃-C₆-Cycloalkyl), Pyridyl oder Pyridylmethyl (wobei Pyridyl und Pyridylmethyl selbst wiederum substituiert sein können durch Halogen, Cyano, Nitro, C₁-C₆₋Alkyl, Halogen-C₁-C₆-alkyl und C₃-C₆-Cycloalkyl), steht.

8. Verbindungen der Formel (I) gemäss Anspruch 1, worin
A für einen A-Rest aus der Reihe (A-b) und (A-f) 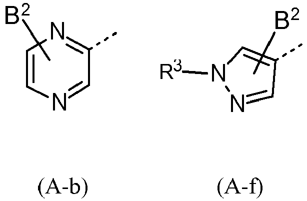 steht, worin die gestrichelte Linie die Bindung zum Kohlenstoffatom des Bicyclus der Formel (I) bedeutet,
B² für einen Rest aus der Reihe Wasserstoff, Halogen, C₁-C₆-Alkyl und C₁-C₄-Halogenalkyl steht,
X₁ für CH steht,
X₂ für CH steht,
X₃ für CH steht,
R₁ für einen B-Rest aus der Reihe 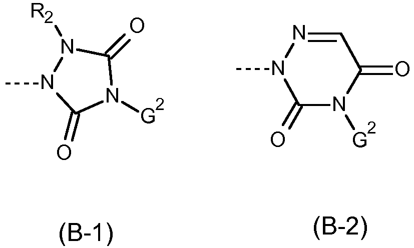 steht, worin die gestrichelte Linie die Bindung zum Kohlenstoffatom des Bicyclus der Formel (I) bedeutet, worin
R₂ für Wasserstoff oder C₁-C₆-Alkyl steht,
G² für einen Rest aus der Reihe Wasserstoff, C₁-C₆-Alkyl, Halogen-C₁-C₆-alkyl, C₃-C₆-Cycloalkyl, Halogen-C₃-C₆-cycloalkyl, C₃-C₆-Cycloalkenyl und Halogen-C₃-C₆-cycloalkenyl steht, und
Verbindungen der Formel (I), worin
A für den A-Rest 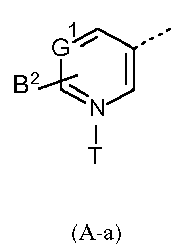 steht, worin die gestrichelte Linie die Bindung zum Kohlenstoffatom des Bicyclus der Formel (I) bedeutet,
G¹ für N oder C-B¹ steht,
B¹ für einen Rest aus der Reihe Wasserstoff, Halogen, C₁-C₆-Alkyl und C₁-C₄-Halogenalkyl steht,
B² für einen Rest aus der Reihe Wasserstoff, Halogen, C₁-C₆-Alkyl und C₁-C₄-Halogenalkyl steht,
T für Sauerstoff oder ein Elektronenpaar steht,
X₁ für N oder CH steht,
X₂ für CH steht,
X₃ für CH steht,
R₁ für einen B-Rest aus der Reihe 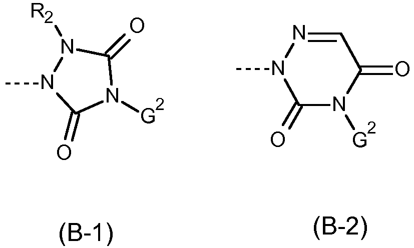 steht, worin die gestrichelte Linie die Bindung zum Kohlenstoffatom des Bicyclus der Formel (I) bedeutet, worin
R₂ für Wasserstoff, C₁-C₆-Alkyl oder Halogen-C₁-C₆-alkyl steht,
G² für einen Rest aus der Reihe Wasserstoff, C₁-C₆-Alkyl, Halogen-C₁-C₆-alkyl, C₁-C₆-Alkoxy-C₁-C₆-alkyl, C₁-C₆-Alkyl-S-C₁-C₆-alkyl, C₁-C₆-Alkyl-S(O)-C₁-C₆-alkyl, C₁-C₆-Alkyl-S(O₂)-C₁-C₆-alkyl, C₃-C₆-Cycloalkyl, C₃-C₆-Cycloalkyl-C₁-C₆-alkyl, Phenyl oder Benzyl (wobei Phenyl und Benzyl selbst wiederum substituiert sein können durch Halogen, Cyano, Nitro, C₁-C₆₋Alkyl, Halogen-C₁-C₆-alkyl und C₃-C₆-Cycloalkyl), Pyridylmethyl, welches durch Halogen substituiert sein kann, Halogen-C₃-C₆-cycloalkyl, C₃-C₆-Cycloalkenyl und Halogen-C₃-C₆-cycloalkenyl steht.

9. Verbindungen der Formel (I) gemäss Anspruch 1, in welchen
A für einen der folgenden A-Reste 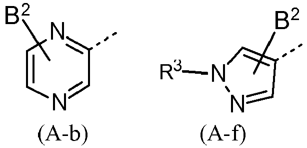 steht, worin die gestrichelte Linie die Bindung zum Kohlenstoffatom des Bicyclus der Formel (I) bedeutet,
B² für Wasserstoff steht,
X₁ für CH steht,
X₂ für CH steht,
X₃ für CH steht,
R₁ für einen B-Rest aus der Reihe 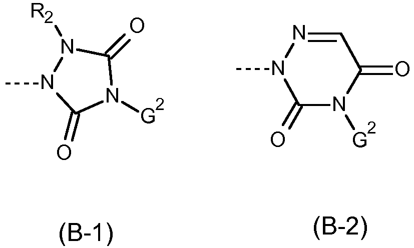 steht, worin die gestrichelte Linie die Bindung zum Kohlenstoffatom des Bicyclus der Formel (I) bedeutet, worin
R₂ für Wasserstoff oder C₁-C₆-Alkyl steht,
G² für einen Rest aus der Reihe Wasserstoff, C₁-C₆-Alkyl, Halogen-C₁-C₆-alkyl und C₃-C₆-Cycloalkyl steht, und
Verbindungen der Formel (I), in welchen
A für den A-Rest 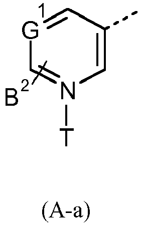 steht, worin die gestrichelte Linie die Bindung zum Kohlenstoffatom des Bicyclus der Formel (I) bedeutet,
G¹ für N oder C-B¹ steht,
B¹ für einen Rest aus der Reihe Wasserstoff und Fluor steht,
B² für Wasserstoff steht,
T für Sauerstoff oder ein Elektronenpaar steht,
X₁ für N oder CH steht,
X₂ für CH steht,
X₃ für CH steht,
R₁ für einen B-Rest aus der Reihe 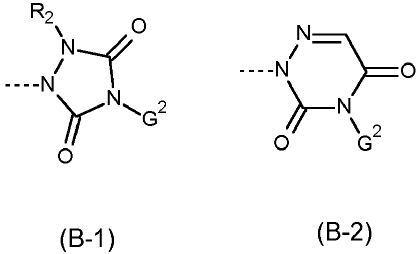 steht, worin die gestrichelte Linie die Bindung zum Kohlenstoffatom des Bicyclus der Formel (I) bedeutet, worin
R₂ für Wasserstoff, C₁-C₆-Alkyl oder Halogen-C₁-C₆-alkyl steht,
G² für einen Rest aus der Reihe C₁-C₄-Alkyl, Halogen-C₁-C₄-alkyl, C₁-C₄-Alkoxy-C₁-C₄-alkyl, C₁-C₄-Alkyl-S-C₁-C₄-alkyl, C₁-C₄-Alkyl-S(O)-C₁-C₄-alkyl, C₁-C₄-Alkyl-S(O₂)-C₁-C₄-alkyl,C₃-C₆-Cycloalkyl, C₃-C₆-Cycloalkyl-C₁-C₄-alkyl, Phenyl oder Benzyl (wobei Phenyl und Benzyl selbst wiederum substituiert sein können durch Halogen und Cyano), Pyridylmethyl, welches durch Halogen substituiert sein kann, steht.

10. Verbindungen der Formel (I) gemäss Anspruch 1, in welchen
A für 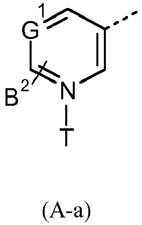 steht, worin die gestrichelte Linie die Bindung zum Kohlenstoffatom des Bicyclus der Formel (I) bedeutet,
G¹ für C-B¹ steht,
B¹ für Wasserstoff steht,
B² für Wasserstoff steht,
T für ein Elektronenpaar steht,
X₁ für N oder CH steht,
X₂ für CH steht,
X₃ für CH steht,
R₁ für den Rest (B-1) 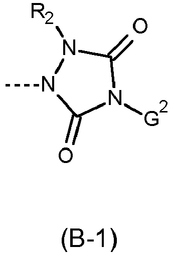 steht, worin die gestrichelte Linie die Bindung zum Kohlenstoffatom des Bicyclus der Formel (I) bedeutet, worin
R₂ für Wasserstoff, C₁-C₄-Alkyl oder Halogen-C₁-C₄-alkyl steht und
G² für einen Rest aus der Reihe C₁-C₄-Alkyl, Halogen-C₁-C₄-alkyl, C₁-C₄-Alkoxy-C₁-C₄-alkyl, C₁-C₄-Alkyl-S-C₁-C₄-alkyl, C₁-C₄-Alkyl-S(O)-C₁-C₄-alkyl, C₃-C₆-Cycloalkyl, C₃-C₆-Cycloalkyl-C₁-C₄-alkyl, Phenyl, Benzyl und Pyridylmethyl (wobei Phenyl, Benzyl und Pyridylmethyl selbst wiederum durch Halogen substituiert sein können) steht.

11. Verbindungen der Formel (I) gemäss Anspruch 1, in welchen
A für den A-Rest 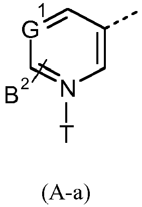 steht, worin die gestrichelte Linie die Bindung zum Kohlenstoffatom des Bicyclus der Formel (I) bedeutet,
G¹ für C-B¹ steht,
B¹ für Wasserstoff steht,
B² für Wasserstoff steht,
T für ein Elektronenpaar steht,
X₁ für N oder CH steht,
X₂ für CH steht,
X₃ für CH steht,
R₁ für den Rest (B-2) 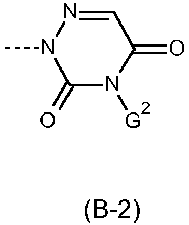 steht, worin die gestrichelte Linie die Bindung zum Kohlenstoffatom des Bicyclus der Formel (I) bedeutet, worin
G² für einen Rest aus der Reihe C₁-C₄-Alkyl, Halogen-C₁-C₄-alkyl, C₃-C₆-Cycloalkyl-C₁-C₄-alkyl, Phenyl oder Benzyl (wobei Phenyl und Benzyl selbst wiederum durch Halogen substituiert sein können) steht.

12. Verbindung gemäss Anspruch 1, **dadurch gekennzeichnet, dass** es sich um die Verbindung der Formel 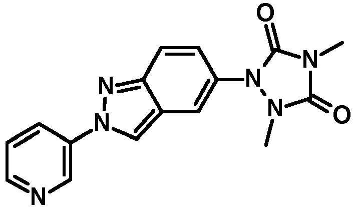 handelt.

13. Verbindung gemäss Anspruch 1, **dadurch gekennzeichnet, dass** es sich um die Verbindung der Formel 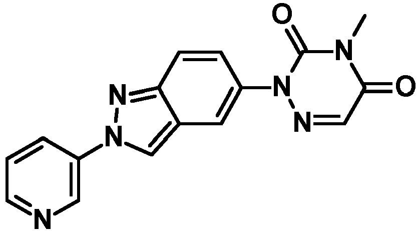 handelt.

14. Mittel, **gekennzeichnet durch** einen Gehalt von mindestens einer Verbindung der Formel (I) gemäß einem der Ansprüche 1 bis 13 und üblichen Streckmitteln und/oder oberflächenaktiven Substanzen.

15. Verwendung von Verbindungen der Formel (I) gemäß einem der Ansprüche 1 bis 13 oder von Mitteln gemäß Anspruch 14 zur Bekämpfung von Schädlingen.

## Claims

1. Compounds of the formula (I) 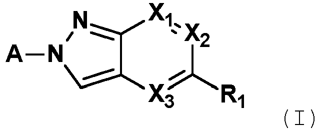 in which
A is an A radical from the group of (A-b) to (A-f) 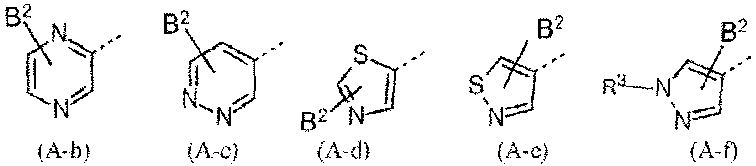 in which the broken line denotes the bond to the carbon atom of the bicyclic system of the formula (I) and
B² is a radical from the group of hydrogen, halogen, cyano, nitro, alkyl, haloalkyl, alkoxy, haloalkoxy and in each case optionally substituted cycloalkyl and cycloalkenyl,
X₁ is nitrogen (N) or CH,
X₂ is nitrogen or CH,
X₃ is nitrogen or CH, where either none or only one of the variables X₁, X₂ and X₃ is nitrogen,
R₁ is a B radical from the group of 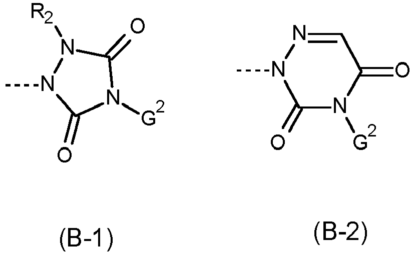 in which the broken line denotes the bond to the carbon atom of the bicyclic system of the formula (I),
R₂ is a radical from the group of hydrogen, alkyl, haloalkyl, alkenyl, alkynyl, alkoxyalkyl, alkyl-S-alkyl, alkyl-S(O)-alkyl, alkyl-S(O)₂-alkyl, cycloalkyl, halocycloalkyl and cycloalkenyl,
G² is a radical from the group of hydrogen, alkyl, haloalkyl, alkenyl, alkynyl, alkoxyalkyl, alkyl-S-alkyl, alkyl-S(O)-alkyl, alkyl-S(O)₂-alkyl, cycloalkyl, halocycloalkyl, cycloalkenyl, halocycloalkenyl, cycloalkylalkyl, cycloalkenylalkyl, halocycloalkenylalkyl and in each case optionally substituted aryl, arylalkyl, pyridyl and pyridylalkyl, and compounds of the formula (I) in which
A is the radical (A-a) 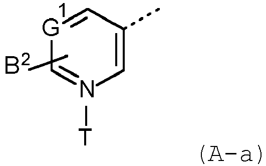 in which the broken line denotes the bond to the carbon atom of the bicyclic system of the formula (I) and
G¹ is N or C-B¹,
B¹ is a radical from the group of hydrogen, halogen, cyano, nitro, alkyl, haloalkyl, alkoxy, haloalkoxy and in each case optionally substituted cycloalkyl and cycloalkenyl,
B² is a radical from the group of hydrogen, halogen, cyano, nitro, alkyl, haloalkyl, alkoxy, haloalkoxy and in each case optionally substituted cycloalkyl and cycloalkenyl,
T is oxygen or an electron pair,
X₁ is nitrogen or CH,
X₂ is nitrogen or CH,
X₃ is nitrogen or CH, where either none or only one of the variables X₁, X₂ and X₃ is nitrogen,
R₁ is one of the following B radicals: 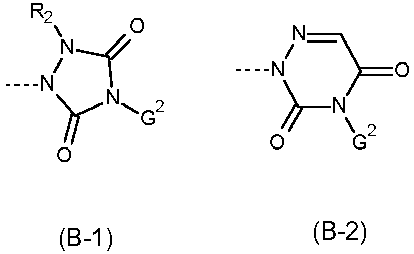 in which the broken line denotes the bond to the carbon atom of the bicyclic system of the formula (I) in which
R₂ is a radical from the group of hydrogen, alkyl, haloalkyl, alkenyl, alkynyl, alkoxyalkyl, alkyl-S-alkyl, alkyl-S(O)-alkyl, alkyl-S(O)₂-alkyl, cycloalkyl, halocycloalkyl and cycloalkenyl,
G² is a radical from the group of hydrogen, alkyl, haloalkyl, alkenyl, alkynyl, alkoxyalkyl, alkyl-S-alkyl, alkyl-S(O)-alkyl, alkyl-S(O)₂-alkyl, cycloalkyl, halocycloalkyl, cycloalkenyl, halocycloalkenyl, cycloalkylalkyl, cycloalkenylalkyl, halocycloalkenylalkyl and in each case optionally substituted aryl, arylalkyl, pyridyl and pyridylalkyl.

2. Compounds of the formula (I) according to Claim 1 in which
A is one of the following A radicals: 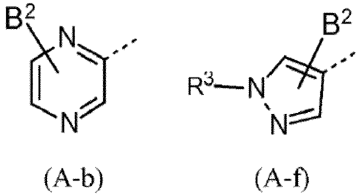 in which the broken line denotes the bond to the carbon atom of the bicyclic system of the formula (I), and
B² is a radical from the group of hydrogen, halogen, C₁-C₆-alkyl and C₁-C₄-haloalkyl,
X₁ is CH,
X₂ is CH,
X₃ is CH,
R₁ is one of the following B radicals: 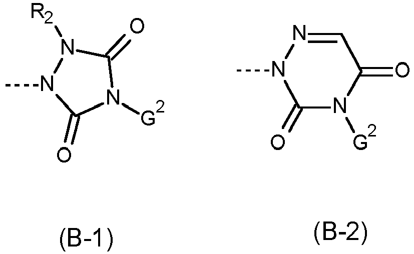 in which the broken line denotes the bond to the carbon atom of the bicyclic system of the formula (I) in which
R₂ is hydrogen or C₁-C₆-alkyl,
G² is a radical from the group of hydrogen, C₁-C₆-alkyl, halo-C₁-C₆-alkyl, C₃-C₆-cycloalkyl, halo-C₃-C₆-cycloalkyl, C₃-C₆-cycloalkenyl, halo-C₃-C₆-cycloalkenyl, phenyl or benzyl (where phenyl and benzyl themselves may in turn be substituted by halogen, cyano, nitro, C₁-C₆-alkyl, halo-C₁-C₆-alkyl and C₃-C₆-cycloalkyl), pyridyl or pyridylmethyl (where pyridyl and pyridylmethyl themselves may in turn be substituted by halogen, cyano, nitro, C₁-C₆-alkyl, halo-C₁-C₆-alkyl and C₃-C₆-cycloalkyl), and compounds of the formula (I) in which
A is the following A radical: 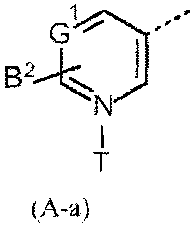 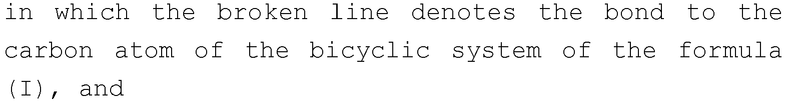
G¹ is N or C-B¹,
B¹ is a radical from the group of hydrogen, halogen, C₁-C₆-alkyl and C₁-C₄-haloalkyl,
B² is a radical from the group of hydrogen, halogen, C₁-C₆-alkyl and C₁-C₄-haloalkyl,
T is oxygen or an electron pair,
X₁ is CH,
X₂ is CH,
X₃ is CH,
R₁ is one of the following B radicals: 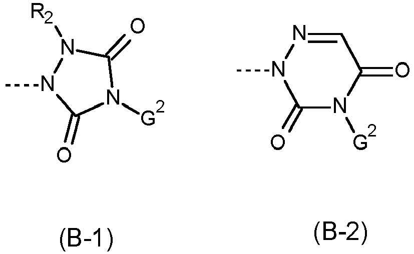 in which the broken line denotes the bond to the carbon atom of the bicyclic system of the formula (I) in which
R₂ is hydrogen or C₁-C₆-alkyl,
G² is a radical from the group of hydrogen, C₁-C₆-alkyl, halo-C₁-C₆-alkyl, C₃-C₆-cycloalkyl, halo-C₃-C₆-cycloalkyl, C₃-C₆-cycloalkenyl, halo-C₃-C₆-cycloalkenyl, phenyl or benzyl (which themselves may in turn be substituted by halogen, cyano, nitro, C₁-C₆-alkyl, halo-C₁-C₆-alkyl and C₃-C₆-cycloalkyl), pyridyl or pyridylmethyl (which themselves may in turn be substituted by halogen, cyano, nitro, C₁-C₆-alkyl, halo-C₁-C₆-alkyl and C₃-C₆-cycloalkyl).

3. Compounds of the formula (I) according to Claim 2 in which
A is an A radical from the group of (A-b) and (A-f) 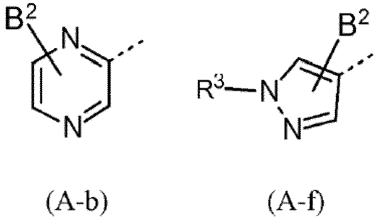 in which the broken line denotes the bond to the carbon atom of the bicyclic system of the formula (I),
B² is a radical from the group of hydrogen, halogen, C₁-C₆-alkyl and C₁-C₄-haloalkyl,
X₁ is CH,
X₂ is CH,
X₃ is CH,
R₁ is a B radical from the group of 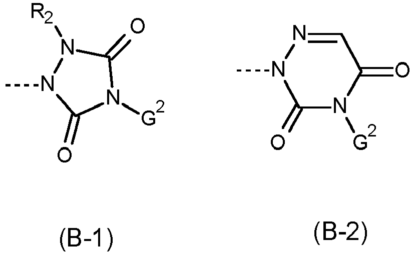 in which the broken line denotes the bond to the carbon atom of the bicyclic system of the formula (I) in which
R₂ is hydrogen or C₁-C₆-alkyl,
G² is a radical from the group of hydrogen, C₁-C₆-alkyl, halo-C₁-C₆-alkyl, C₃-C₆-cycloalkyl, halo-C₃-C₆-cycloalkyl, C₃-C₆-cycloalkenyl and halo-C₃-C₆-cycloalkenyl, and compounds of the formula (I) in which
A is the A radical 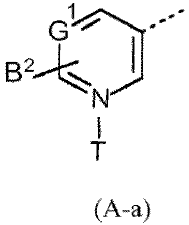 in which the broken line denotes the bond to the carbon atom of the bicyclic system of the formula (I),
G¹ is N or C-B¹,
B¹ is a radical from the group of hydrogen, halogen, C₁-C₆-alkyl and C₁-C₄-haloalkyl,
B² is a radical from the group of hydrogen, halogen, C₁-C₆-alkyl and C₁-C₄-haloalkyl,
T is oxygen or an electron pair,
X₁ is CH,
X₂ is CH,
X₃ is CH,
R₁ is a B radical from the group of 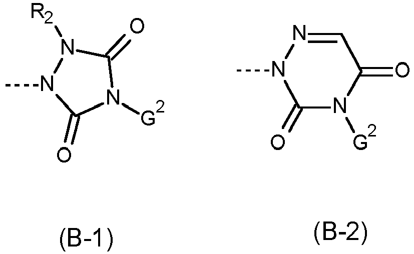 in which the broken line denotes the bond to the carbon atom of the bicyclic system of the formula (I), in which
R₂ is hydrogen or C₁-C₆-alkyl,
G² is a radical from the group of hydrogen, C₁-C₆-alkyl, halo-C₁-C₆-alkyl, C₃-C₆-cycloalkyl, halo-C₃-C₆-cycloalkyl, C₃-C₆-cycloalkenyl and halo-C₃-C₆-cycloalkenyl.

4. Compounds of the formula (I) according to Claim 1 in which
A is one of the following A radicals: 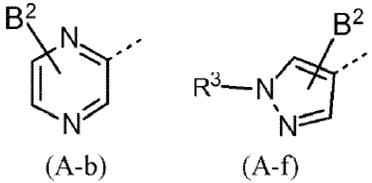 in which the broken line denotes the bond to the carbon atom of the bicyclic system of the formula (I),
B² is hydrogen,
X₁ is CH,
X₂ is CH,
X₃ is CH,
R₁ is a B radical from the group of 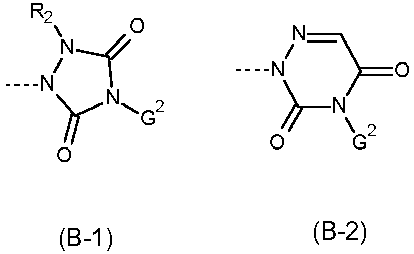 in which the broken line denotes the bond to the carbon atom of the bicyclic system of the formula (I) in which
R₂ is hydrogen or C₁-C₆-alkyl,
G² is a radical from the group of hydrogen, C₁-C₆-alkyl, halo-C₁-C₆-alkyl and C₃-C₆-cycloalkyl, and compounds of the formula (I) in which
A is the A radical 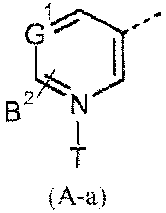 in which the broken line denotes the bond to the carbon atom of the bicyclic system of the formula (I),
G¹ is N or C-B¹,
B¹ is a radical from the group of hydrogen and fluorine,
B² is hydrogen,
T is oxygen or an electron pair,
X₁ is CH,
X₂ is CH,
X₃ is CH,
R₁ is a B radical from the group of 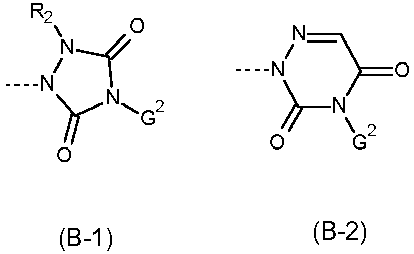 in which the broken line denotes the bond to the carbon atom of the bicyclic system of the formula (I) in which
R₂ is hydrogen or C₁-C₆-alkyl,
G² is a radical from the group of hydrogen, C₁-C₆-alkyl, halo-C₁-C₆-alkyl and C₃-C₆-cycloalkyl.

5. Compounds of the formula (I) according to Claim 1 in which
A is 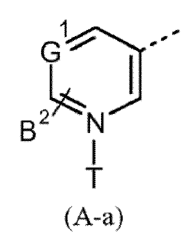 in which the broken line denotes the bond to the carbon atom of the bicyclic system of the formula (I),
G¹ is C-B¹,
B¹ is hydrogen or fluorine,
B² is hydrogen,
T is an electron pair,
X₁ is CH,
X₂ is CH,
X₃ is CH,
R₁ is the (B-1) radical 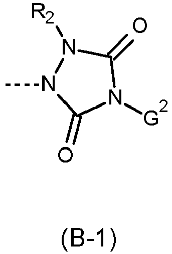 in which the broken line denotes the bond to the carbon atom of the bicyclic system of the formula (I) in which
R₂ is hydrogen or C₁-C₆-alkyl and
G² is a radical from the group of hydrogen, C₁-C₆-alkyl, halo-C₁-C₆-alkyl and C₃-C₆-cycloalkyl.

6. Compounds of the formula (I) according to Claim 1 in which
A is the A radical 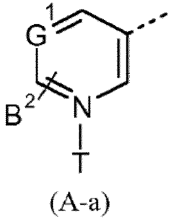 in which the broken line denotes the bond to the carbon atom of the bicyclic system of the formula (I),
G¹ is N or C-B¹,
B¹ is hydrogen or fluorine,
B² is hydrogen,
T is an electron pair,
X₁ is CH,
X₂ is CH,
X₃ is CH,
R₁ is the (B-2) radical 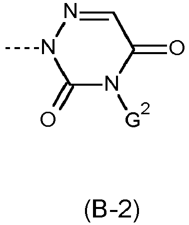 in which the broken line denotes the bond to the carbon atom of the bicyclic system of the formula (I) in which
G² is a radical from the group of hydrogen, C₁-C₆-alkyl, halo-C₁-C₆-alkyl and C₃-C₆-cycloalkyl.

7. Compounds of the formula (I) according to Claim 1 in which
A is one of the following A radicals: 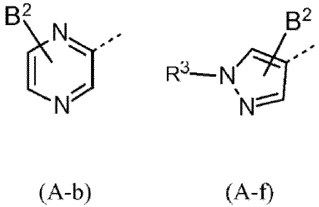 in which the broken line denotes the bond to the carbon atom of the bicyclic system of the formula (I), and
B² is a radical from the group of hydrogen, halogen, C₁-C₆-alkyl and C₁-C₄-haloalkyl,
X₁ is CH,
X₂ is CH,
X₃ is CH,
R₁ is one of the following B radicals: 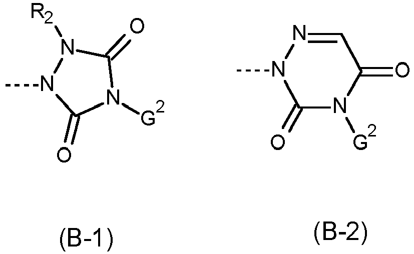 in which the broken line denotes the bond to the carbon atom of the bicyclic system of the formula (I) in which
R₂ is hydrogen or C₁-C₆-alkyl,
G² is a radical from the group of hydrogen, C₁-C₆-alkyl, halo-C₁-C₆-alkyl, C₃-C₆-cycloalkyl, halo-C₃-C₆-cycloalkyl, C₃-C₆-cycloalkenyl, halo-C₃-C₆-cycloalkenyl, phenyl or benzyl (where phenyl and benzyl themselves may in turn be substituted by halogen, cyano, nitro, C₁-C₆-alkyl, halo-C₁-C₆-alkyl and C₃-C₆-cycloalkyl), pyridyl or pyridylmethyl (where pyridyl and pyridylmethyl themselves may in turn be substituted by halogen, cyano, nitro, C₁-C₆-alkyl, halo-C₁-C₆-alkyl and C₃-C₆-cycloalkyl), and
compounds of the formula (I) in which
A is the following A radical: 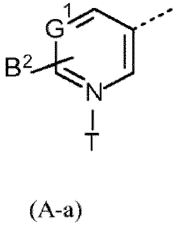 in which the broken line denotes the bond to the carbon atom of the bicyclic system of the formula (I), and
G¹ is N or C-B¹,
B¹ is a radical from the group of hydrogen, halogen, C₁-C₆-alkyl and C₁-C₄-haloalkyl,
B² is a radical from the group of hydrogen, halogen, C₁-C₆-alkyl and C₁-C₄-haloalkyl,
T is oxygen or an electron pair,
X₁ is N or CH,
X₂ is CH,
X₃ is CH,
R₁ is one of the following B radicals: 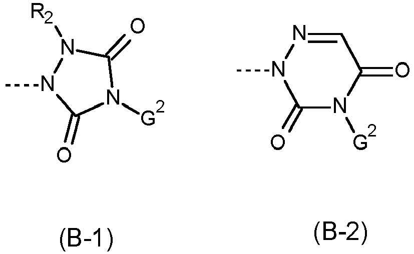 in which the broken line denotes the bond to the carbon atom of the bicyclic system of the formula (I) in which
R₂ is hydrogen, C₁-C₆-alkyl or halo-C₁-C₆-alkyl, and
G² is a radical from the group of hydrogen, C₁-C₆-alkyl, halo-C₁-C₆-alkyl, C₁-C₆-alkoxy-C₁-C₆-alkyl, C₁-C₆-alkyl-S-C₁-C₆-alkyl, C₁-C₆-alkyl-S(O)-C₁-C₆-alkyl, C₁-C₆-alkyl-S(O₂)-C₁-C₆-alkyl, C₃-C₆-cycloalkyl, C₃-C₆-cycloalkyl-C₁-C₆-alkyl, halo-C₃-C₆-cycloalkyl, C₃-C₆-cycloalkenyl, halo-C₃-C₆-cycloalkenyl, phenyl or benzyl (where phenyl and benzyl themselves may in turn be substituted by halogen, cyano, nitro, C₁-C₆-alkyl, halo-C₁-C₆-alkyl and C₃-C₆-cycloalkyl), pyridyl or pyridylmethyl (where pyridyl and pyridylmethyl themselves may in turn be substituted by halogen, cyano, nitro, C₁-C₆-alkyl, halo-C₁-C₆-alkyl and C₃-C₆-cycloalkyl).

8. Compounds of the formula (I) according to Claim 1 in which
A is an A radical from the group of (A-b) and (A-f) 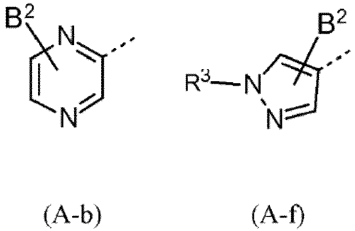 in which the broken line denotes the bond to the carbon atom of the bicyclic system of the formula (I),
B² is a radical from the group of hydrogen, halogen, C₁-C₆-alkyl and C₁-C₄-haloalkyl,
X₁ is CH,
X₂ is CH,
X₃ is CH,
R₁ is a B radical from the group of 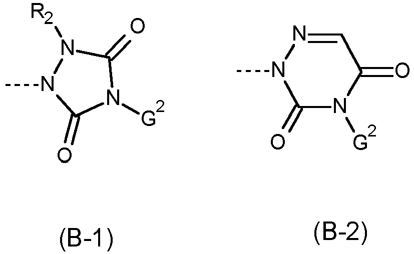 in which the broken line denotes the bond to the carbon atom of the bicyclic system of the formula (I) in which
R₂ is hydrogen or C₁-C₆-alkyl,
G² is a radical from the group of hydrogen, C₁-C₆-alkyl, halo-C₁-C₆-alkyl, C₃-C₆-cycloalkyl, halo-C₃-C₆-cycloalkyl, C₃-C₆-cycloalkenyl and halo-C₃-C₆-cycloalkenyl, and
compounds of the formula (I) in which
A is the A radical 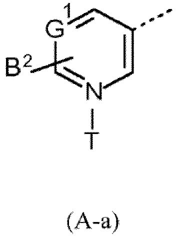 in which the broken line denotes the bond to the carbon atom of the bicyclic system of the formula (I),
G¹ is N or C-B¹,
B¹ is a radical from the group of hydrogen, halogen, C₁-C₆-alkyl and C₁-C₄-haloalkyl,
B² is a radical from the group of hydrogen, halogen, C₁-C₆-alkyl and C₁-C₄-haloalkyl,
T is oxygen or an electron pair,
X₁ is N or CH,
X₂ is CH,
X₃ is CH,
R₁ is a B radical from the group of 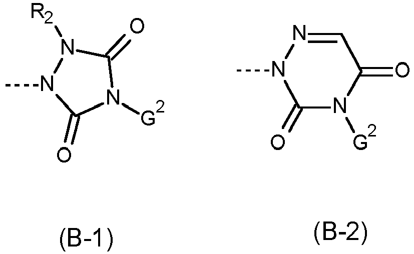 in which the broken line denotes the bond to the carbon atom of the bicyclic system of the formula (I) in which
R₂ is hydrogen, C₁-C₆-alkyl or halo-C₁-C₆-alkyl,
G² is a radical from the group of hydrogen, C₁-C₆-alkyl, halo-C₁-C₆-alkyl, C₁-C₆-alkoxy-C₁-C₆-alkyl, C₁-C₆-alkyl-S-C₁-C₆-alkyl, C₁-C₆-alkyl-S(O)-C₁-C₆-alkyl, C₁-C₆-alkyl-S(O₂)-C₁-C₆-alkyl, C₃-C₆-cycloalkyl, C₃-C₆-cycloalkyl-C₁-C₆-alkyl, phenyl or benzyl (where phenyl and benzyl themselves may in turn be substituted by halogen, cyano, nitro, C₁-C₆-alkyl, halo-C₁-C₆-alkyl and C₃-C₆-cycloalkyl), pyridylmethyl which may be substituted by halogen, and halo-C₃-C₆-cycloalkyl, C₃-C₆-cycloalkenyl and halo-C₃-C₆-cycloalkenyl.

9. Compounds of the formula (I) according to Claim 1 in which
A is one of the following A radicals: 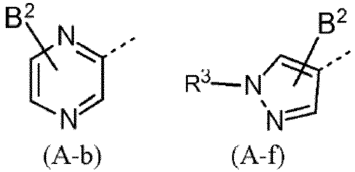 in which the broken line denotes the bond to the carbon atom of the bicyclic system of the formula (I),
B² is hydrogen,
X₁ is CH,
X₂ is CH,
X₃ is CH,
R₁ is a B radical from the group of 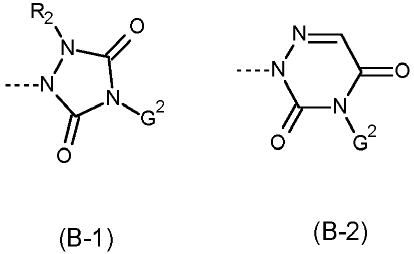 in which the broken line denotes the bond to the carbon atom of the bicyclic system of the formula (I) in which
R₂ is hydrogen or C₁-C₆-alkyl,
G² is a radical from the group of hydrogen, C₁-C₆-alkyl, halo-C₁-C₆-alkyl and C₃-C₆-cycloalkyl, and
compounds of the formula (I) in which
A is the A radical 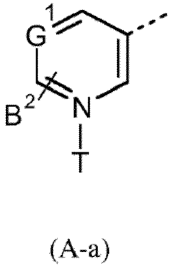 in which the broken line denotes the bond to the carbon atom of the bicyclic system of the formula (I),
G¹ is N or C-B¹,
B¹ is a radical from the group of hydrogen and fluorine,
B² is hydrogen,
T is oxygen or an electron pair,
X₁ is N or CH,
X₂ is CH,
X₃ is CH,
R₁ is a B radical from the group of 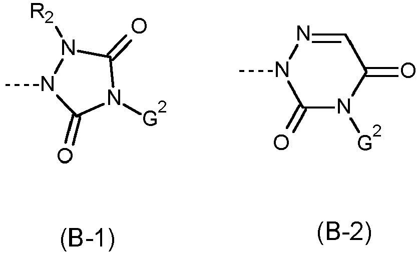 in which the broken line denotes the bond to the carbon atom of the bicyclic system of the formula (I) in which
R₂ is hydrogen, C₁-C₆-alkyl or halo-C₁-C₆-alkyl,
G² is a radical from the group of C₁-C₄-alkyl, halo-C₁-C₄-alkyl, C₁-C₄-alkoxy-C₁-C₄-alkyl, C₁-C₄-alkyl-S-C₁-C₄-alkyl, C₁-C₄-alkyl-S(O)-C₁-C₄-alkyl, C₁-C₄-alkyl-S (O₂)-C₁-C₄-alkyl, C₃-C₆-cycloalkyl, C₃-C₆-cycloalkyl-C₁-C₄-alkyl, phenyl or benzyl (where phenyl and benzyl themselves may in turn be substituted by halogen and cyano), pyridylmethyl which may be substituted by halogen.

10. Compounds of the formula (I) according to Claim 1 in which
A is 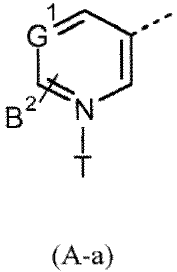 in which the broken line denotes the bond to the carbon atom of the bicyclic system of the formula (I),
G¹ is C-B¹,
B¹ is hydrogen,
B² is hydrogen,
T is an electron pair,
X₁ is N or CH,
X₂ is CH,
X₃ is CH,
R₁ is the (B-1) radical 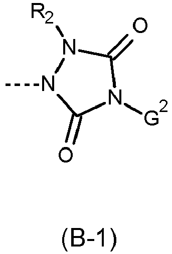 in which the broken line denotes the bond to the carbon atom of the bicyclic system of the formula (I) in which
R₂ is hydrogen, C₁-C₄-alkyl or halo-C₁-C₄-alkyl and
G² is a radical from the group of C₁-C₄-alkyl, halo-C₁-C₄-alkyl, C₁-C₄-alkoxy-C₁-C₄-alkyl, C₁-C₄-alkyl-S-C₁-C₄-alkyl, C₁-C₄-alkyl-S(O)-C₁-C₄-alkyl, C₃-C₆-cycloalkyl, C₃-C₆-cycloalkyl-C₁-C₄-alkyl, phenyl, benzyl and pyridylmethyl (where phenyl, benzyl and pyridylmethyl themselves may in turn be substituted by halogen).

11. Compounds of the formula (I) according to Claim 1 in which
A is the A radical 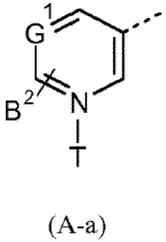 in which the broken line denotes the bond to the carbon atom of the bicyclic system of the formula (I),
G¹ is C-B¹,
B¹ is hydrogen,
B² is hydrogen,
T is an electron pair,
X₁ is N or CH,
X₂ is CH,
X₃ is CH,
R₁ is the (B-2) radical 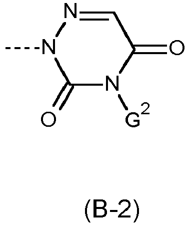 where the broken line denotes the bond to the carbon atom of the bicyclic system of the formula (I), where
G² is a radical from the group of C₁-C₄-alkyl, halo-C₁-C₄-alkyl, C₃-C₆-cycloalkyl-C₁-C₄-alkyl, phenyl or benzyl (where phenyl and benzyl themselves may in turn be substituted by halogen).

12. Compound according to Claim 1, **characterized in that** it is the compound of the formula

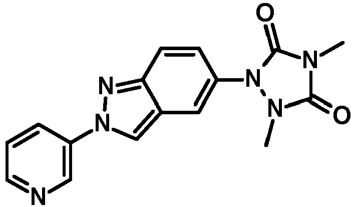

13. Compound according to Claim 1, **characterized in that** it is the compound of the formula

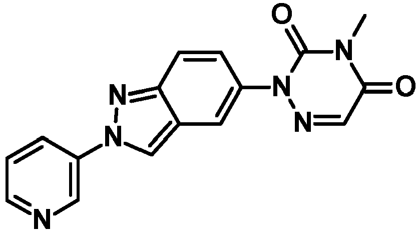

14. Compositions, **characterized by** a content of at least one compound of the formula (I) according to any of Claims 1 to 13 and customary extenders and/or surfactants.

15. Use of compounds of the formula (I) according to any of Claims 1 to 13 or of compositions according to Claim 14 for controlling pests.

## Revendications

1. Composés de formule (I) 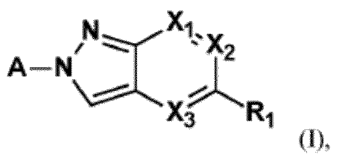 dans laquelle
A représente un radical A de la série (A-b) à (A-f) , 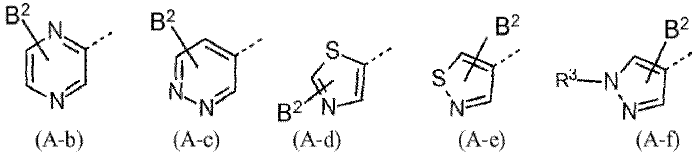 dans laquelle la ligne en pointillés signifie la liaison à l'atome de carbone du bicycle de formule (I) et
B² représente un radical de la série hydrogène, halogène, cyano, nitro, alkyle, halogénoalkyle, alcoxy, halogénoalcoxy et cycloalkyle et cycloalcényle à chaque fois éventuellement substitués,
X₁ représente azote (N) ou CH,
X₂ représente azote ou CH,
X₃ représente azote ou CH, soit aucune, soit une seule des variables X₁, X₂ et X₃ représentant azote,
R₁ représente un radical B de la série 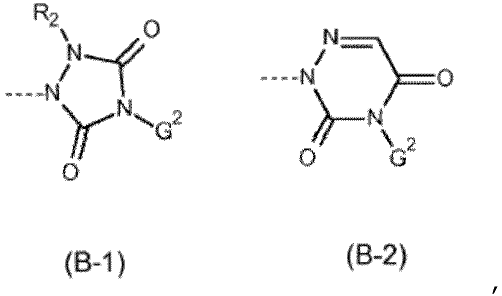 dans laquelle la ligne en pointillés signifie la liaison à l'atome de carbone du bicycle de formule (I),
R₂ représente un radical de la série hydrogène, alkyle, halogénoalkyle, alcényle, alcynyle, alcoxyalkyle, alkyl-S-alkyle, alkyl-S(O)-alkyle, alkyl-S(O)₂-alkyle, cycloalkyle, halogénocycloalkyle et cycloalcényle,
G₂ représente un radical de la série hydrogène, alkyle, halogénoalkyle, alcényle, alcynyle, alcoxyalkyle, alkyl-S-alkyle, alkyl-S(O)-alkyle, alkyl-S(O)₂-alkyle, cycloalkyle, halogénocycloalkyle, cycloalcényle, halogénocycloalcényle, cycloalkylalkyle, cycloalcénylalkyle, halogénocycloalcénylalkyle et aryle, arylalkyle, pyridyle et pyridylalkyle à chaque fois éventuellement substitués, et composés de formule (I), dans laquelle
A représente le radical (A-a) 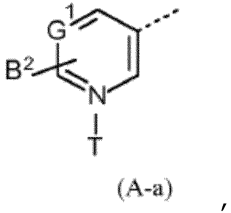 dans lequel la ligne en pointillés signifie la liaison à l'atome de carbone du bicycle de formule (I) et
G¹ représente N ou C-B¹,
B¹ représente un radical de la série hydrogène, halogène, cyano, nitro, alkyle, halogénoalkyle, alcoxy, halogénoalcoxy et cycloalkyle et cycloalcényle à chaque fois éventuellement substitués,
B² représente un radical de la série hydrogène, halogène, cyano, nitro, alkyle, halogénoalkyle, alcoxy, halogénoalcoxy et cycloalkyle et cycloalcényle à chaque fois éventuellement substitués,
T représente oxygène ou une paire d'électrons,
X₁ représente azote ou CH,
X₂ représente azote ou CH,
X₃ représente azote ou CH, soit aucune, soit une seule des variables X₁, X₂ et X₃ représentant azote,
R₁ représente un des radicaux B suivants 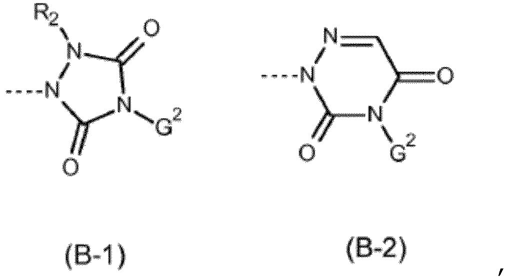 dans lesquels la ligne en pointillés signifie la liaison à l'atome de carbone du bicycle de formule (I), dans lesquels
R₂ représente un radical de la série hydrogène, alkyle, halogénoalkyle, alcényle, alcynyle, alcoxyalkyle, alkyl-S-alkyle, alkyl-S(O)-alkyle, alkyl-S(O)₂-alkyle, cycloalkyle, halogénocycloalkyle et cycloalcényle,
G₂ représente un radical de la série hydrogène, alkyle, halogénoalkyle, alcényle, alcynyle, alcoxyalkyle, alkyl-S-alkyle, alkyl-S(O)-alkyle, alkyl-S(O)₂-alkyle, cycloalkyle, halogénocycloalkyle, cycloalcényle, halogénocycloalcényle, cycloalkylalkyle, cycloalcénylalkyle, halogénocycloalcénylalkyle et aryle, arylalkyle, pyridyle et pyridylalkyle à chaque fois éventuellement substitués.

2. Composés de formule (I) selon la revendication 1, dans lesquels
A représente un des radicaux A suivants 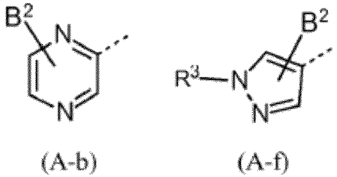 dans lesquels la ligne en pointillés signifie la liaison à l'atome de carbone du bicycle de formule (I), et
B² représente un radical de la série hydrogène, halogène, C₁-C₆-alkyle et C₁-C₄-halogénoalkyle,
X₁ représente CH,
X₂ représente CH,
X₃ représente CH,
R₁ représente un des radicaux B suivants 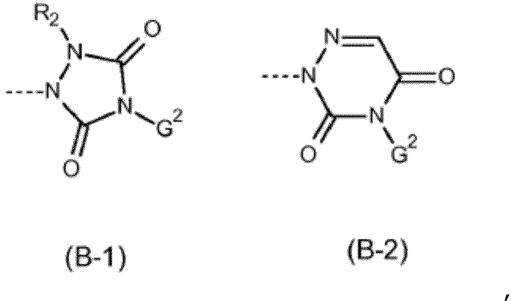 dans lesquels la ligne en pointillés signifie la liaison à l'atome de carbone du bicycle de formule (I), dans lesquels
R₂ représente hydrogène ou C₁-C₆-alkyle,
G² représente un radical de la série hydrogène, C₁-C₆-alkyle, halogéno-C₁-C₆-alkyle, C₃-C₆-cycloalkyle, halogéno-C₃-C₆-cycloalkyle, C₃-C₆-cycloalcényle, halogéno-C₃-C₆-cycloalcényle, phényle ou benzyle (phényle et benzyle eux-mêmes à leur tour pouvant être substitués par halogène, cyano, nitro, C₁-C₆-alkyle, halogéno-C₁-C₆-alkyle et C₃-C₆-cycloalkyle), pyridyle ou pyridylméthyle (pyridyle et pyridylméthyle eux-mêmes à leur tour pouvant être substitués par halogène, cyano, nitro, C₁-C₆-alkyle, halogéno-C₁-C₆-alkyle et C₃-C₆-cycloalkyle), et
composés de formule (I), dans lesquels
A représente le radical A suivant 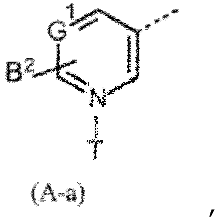 dans lequel la ligne en pointillés signifie la liaison à l'atome de carbone du bicycle de formule (I), et
G¹ représente N ou C-B¹,
B¹ représente un radical de la série hydrogène, halogène, C₁-C₆-alkyle et C₁-C₄-halogénoalkyle,
B² représente un radical de la série hydrogène, halogène, C₁-C₆-alkyle et C₁-C₄-halogénoalkyle,
T représente oxygène ou une paire d'électrons,
X₁ représente CH,
X₂ représente CH,
X₃ représente CH,
R₁ représente un des radicaux B suivants 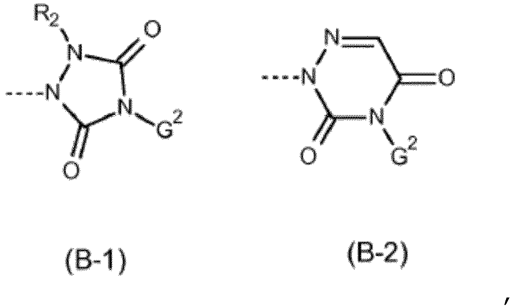 dans lesquels la ligne en pointillés signifie la liaison à l'atome de carbone du bicycle de formule (I), dans lesquels
R₂ représente hydrogène ou C₁-C₆-alkyle,
G² représente un radical de la série hydrogène, C₁-C₆-alkyle, halogéno-C₁-C₆-alkyle, C₃-C₆-cycloalkyle, halogéno-C₃-C₆-cycloalkyle, C₃-C₆-cycloalcényle, halogéno-C₃-C₆-cycloalcényle, phényle ou benzyle (phényle et benzyle eux-mêmes à leur tour pouvant être substitués par halogène, cyano, nitro, C₁-C₆-alkyle, halogéno-C₁-C₆-alkyle et C₃-C₆-cycloalkyle), pyridyle ou pyridylméthyle (pyridyle et pyridylméthyle eux-mêmes à leur tour pouvant être substitués par halogène, cyano, nitro, C₁-C₆-alkyle, halogéno-C₁-C₆-alkyle et C₃-C₆-cycloalkyle).

3. Composés de formule (I) selon la revendication 2, dans laquelle
A représente un radical A de la série (A-b) et (A-f) 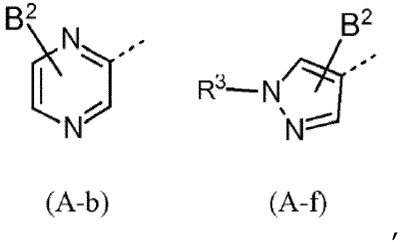 dans lesquels la ligne en pointillés signifie la liaison à l'atome de carbone du bicycle de formule (I), et
B² représente un radical de la série hydrogène, halogène, C₁-C₆-alkyle et C₁-C₄-halogénoalkyle,
X₁ représente CH,
X₂ représente CH,
X₃ représente CH,
R₁ représente un radical B de la série 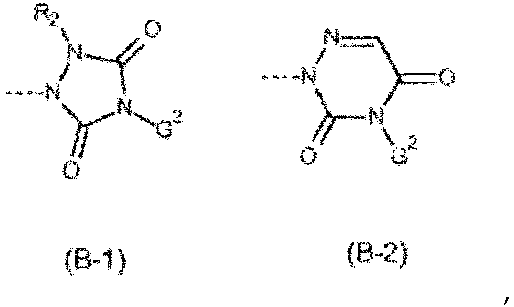 où la ligne en pointillés signifie la liaison à l'atome de carbone du bicycle de formule (I), où
R₂ représente hydrogène ou C₁-C₆-alkyle,
G² représente un radical de la série hydrogène, C₁-C₆-alkyle, halogéno-C₁-C₆-alkyle, C₃-C₆-cycloalkyle, halogéno-C₃-C₆-cycloalkyle, C₃-C₆-cycloalcényle et halogéno-C₃-C₆-cycloalcényle, et composés de formule (I), dans laquelle
A représente le radical A 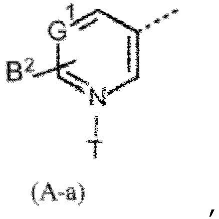 dans lequel la ligne en pointillés signifie la liaison à l'atome de carbone du bicycle de formule (I),
G¹ représente N ou C-B¹,
B¹ représente un radical de la série hydrogène, halogène, C₁-C₆-alkyle et C₁-C₄-halogénoalkyle,
B² représente un radical de la série hydrogène, halogène, C₁-C₆-alkyle et C₁-C₄-halogénoalkyle,
T représente oxygène ou une paire d'électrons,
X₁ représente CH,
X₂ représente CH,
X₃ représente CH,
R₁ représente un radical B de la série 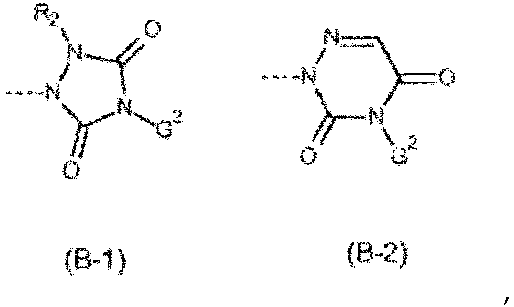 où la ligne en pointillés signifie la liaison à l'atome de carbone du bicycle de formule (I), où
R₂ représente hydrogène ou C₁-C₆-alkyle,
G² représente un radical de la série hydrogène, C₁-C₆-alkyle, halogéno-C₁-C₆-alkyle, C₃-C₆-cycloalkyle, halogéno-C₃-C₆-cycloalkyle, C₃-C₆-cycloalcényle et halogéno-C₃-C₆-cycloalcényle.

4. Composés de formule (I) selon la revendication 1, dans lesquels
A représente un des radicaux A suivants 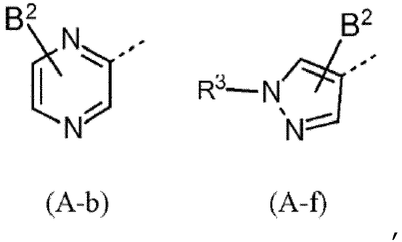 dans lesquels la ligne en pointillés signifie la liaison à l'atome de carbone du bicycle de formule (I),
B² représente hydrogène,
X₁ représente CH,
X₂ représente CH,
X₃ représente CH,
R₁ représente un radical B de la série 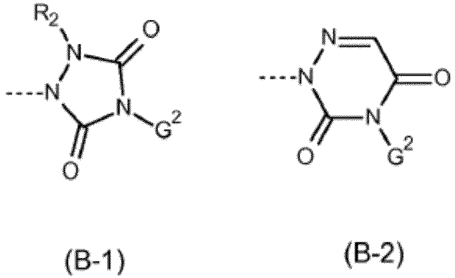 dans lesquels la ligne en pointillés signifie la liaison à l'atome de carbone du bicycle de formule (I), dans lesquels
R₂ représente hydrogène ou C₁-C₆-alkyle,
G² représente un radical de la série hydrogène, C₁-C₆-alkyle, halogéno-C₁-C₆-alkyle et C₃-C₆-cycloalkyle, et composés de formule (I), dans laquelle
A représente le radical A 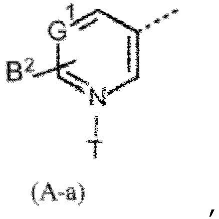 dans lequel la ligne en pointillés signifie la liaison à l'atome de carbone du bicycle de formule (I),
G¹ représente N ou C-B¹,
B¹ représente un radical de la série hydrogène et fluor,
B² représente hydrogène,
T représente oxygène ou une paire d'électrons,
X₁ représente CH,
X₂ représente CH,
X₃ représente CH,
R₁ représente un radical B de la série 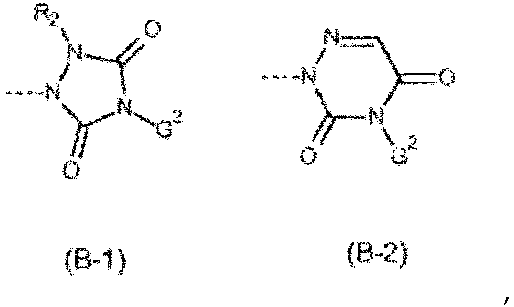 où la ligne en pointillés signifie la liaison à l'atome de carbone du bicycle de formule (I), où
R₂ représente hydrogène ou C₁-C₆-alkyle,
G² représente un radical de la série hydrogène, C₁-C₆-alkyle, halogéno-C₁-C₆-alkyle et C₃-C₆-cycloalkyle.

5. Composés de formule (I) selon la revendication 1, dans lesquels
A représente 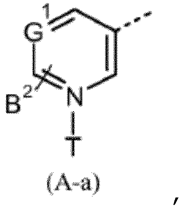 où la ligne en pointillés signifie la liaison à l'atome de carbone du bicycle de formule (I),
G¹ représente C-B¹,
B¹ représente hydrogène ou fluor,
B² représente hydrogène,
T représente une paire d'électrons,
X₁ représente CH,
X₂ représente CH,
X₃ représente CH,
R₁ représente le radical (B-1) 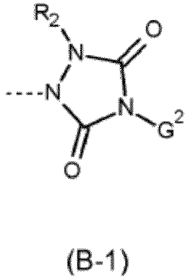 dans lequel la ligne en pointillés signifie la liaison à l'atome de carbone du bicycle de formule (I), dans lequel
R₂ représente hydrogène ou C₁-C₆-alkyle et
G² représente un radical de la série hydrogène, C₁-C₆-alkyle, halogéno-C₁-C₆-alkyle et C₃-C₆-cycloalkyle.

6. Composés de formule (I) selon la revendication 1, dans lesquels
A représente le radical A 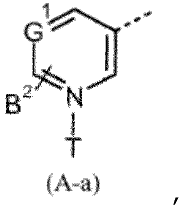 dans lequel la ligne en pointillés signifie la liaison à l'atome de carbone du bicycle de formule (I),
G¹ représente N ou C-B¹,
B¹ représente hydrogène ou fluor,
B² représente hydrogène,
T représente une paire d'électrons,
X₁ représente CH,
X₂ représente CH,
X₃ représente CH,
R₁ représente le radical (B-2) 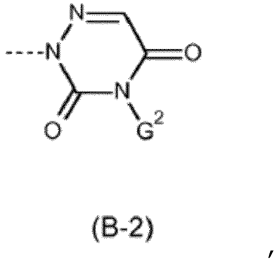 dans lequel la ligne en pointillés signifie la liaison à l'atome de carbone du bicycle de formule (I), dans lequel
G² représente un radical de la série hydrogène, C₁-C₆-alkyle, halogéno-C₁-C₆-alkyle et C₃-C₆-cycloalkyle.

7. Composés de formule (I) selon la revendication 1, dans lesquels
A représente un des radicaux A suivants 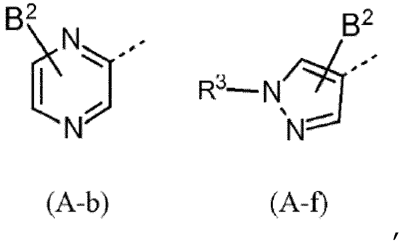 dans lesquels la ligne en pointillés signifie la liaison à l'atome de carbone du bicycle de formule (I), et
B² représente un radical de la série hydrogène, halogène, C₁-C₆-alkyle et C₁-C₄-halogénoalkyle,
X₁ représente CH,
X₂ représente CH,
X₃ représente CH,
R₁ représente un des radicaux B suivants 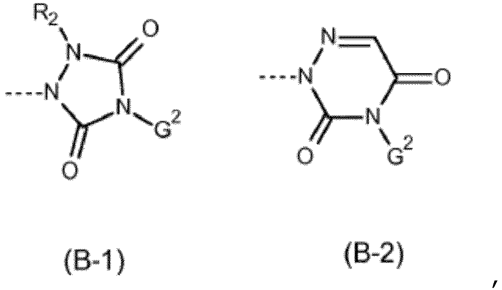 dans lesquels la ligne en pointillés signifie la liaison à l'atome de carbone du bicycle de formule (I), dans lesquels
R₂ représente hydrogène ou C₁-C₆-alkyle,
G² représente un radical de la série hydrogène, C₁-C₆-alkyle, halogéno-C₁-C₆-alkyle, C₃-C₆-cycloalkyle, halogéno-C₃-C₆-cycloalkyle, C₃-C₆-cycloalcényle, halogéno-C₃-C₆-cycloalcényle, phényle ou benzyle (phényle et benzyle eux-mêmes à leur tour pouvant être substitués par halogène, cyano, nitro, C₁-C₆-alkyle, halogéno-C₁-C₆-alkyle et C₃-C₆-cycloalkyle), pyridyle ou pyridylméthyle (pyridyle et pyridylméthyle eux-mêmes à leur tour pouvant être substitués par halogène, cyano, nitro, C₁-C₆-alkyle, halogéno-C₁-C₆-alkyle et C₃-C₆-cycloalkyle), et composés de formule (I), dans lesquels
A représente le radical A suivant 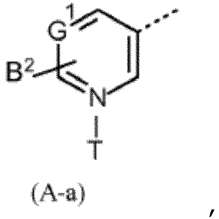 dans lequel la ligne en pointillés signifie la liaison à l'atome de carbone du bicycle de formule (I), et
G¹ représente N ou C-B¹,
B¹ représente un radical de la série hydrogène, halogène, C₁-C₆-alkyle et C₁-C₄-halogénoalkyle,
B² représente un radical de la série hydrogène, halogène, C₁-C₆-alkyle et C₁-C₄-halogénoalkyle,
T représente oxygène ou une paire d'électrons,
X₁ représente N ou CH,
X₂ représente CH,
X₃ représente CH,
R₁ représente un des radicaux B suivants 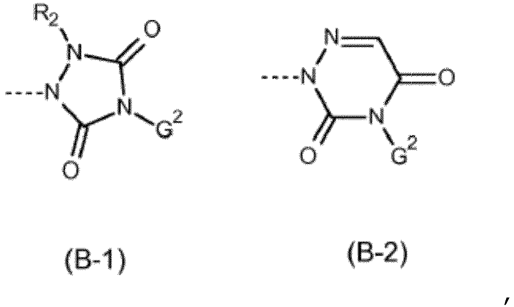 dans lesquels la ligne en pointillés signifie la liaison à l'atome de carbone du bicycle de formule (I), dans lesquels
R₂ représente hydrogène, C₁-C₆-alkyle ou halogéno-C₁-C₆-alkyle, et
G² représente un radical de la série hydrogène, C₁-C₆-alkyle, halogéno-C₁-C₆-alkyle, C₁-C₆-alcoxy-C₁-C₆-alkyle, C₁-C₆-alkyl-S-C₁-C₆-alkyle, C₁-C₆-alkyl-S(O)-C₁-C₆-alkyle, C₁-C₆-alkyl-S(O₂)-C₁-C₆-alkyle, C₃-C₆-cycloalkyle, C₃-C₆-cycloalkyl-C₁-C₆-alkyle, halogéno-C₃-C₆-cycloalkyle, C₃-C₆-cycloalcényle, halogéno-C₃-C₆-cycloalcényle, phényle ou benzyle (phényle et benzyle eux-mêmes à leur tour pouvant être substitués par halogène, cyano, nitro, C₁-C₆-alkyle, halogéno-C₁-C₆-alkyle et C₃-C₆-cycloalkyle), pyridyle ou pyridylméthyle (pyridyle et pyridylméthyle eux-mêmes à leur tour pouvant être substitués par halogène, cyano, nitro, C₁-C₆-alkyle, halogéno-C₁-C₆-alkyle et C₃-C₆-cycloalkyle).

8. Composés de formule (I) selon la revendication 1, dans lesquels
A représente un radical A de la série (A-b) et (A-f) 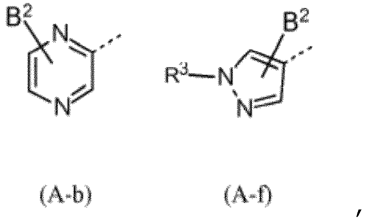 dans lesquels la ligne en pointillés signifie la liaison à l'atome de carbone du bicycle de formule (I),
B² représente un radical de la série hydrogène, halogène, C₁-C₆-alkyle et C₁-C₄-halogénoalkyle,
X₁ représente CH,
X₂ représente CH,
X₃ représente CH,
R₁ représente un radical B de la série 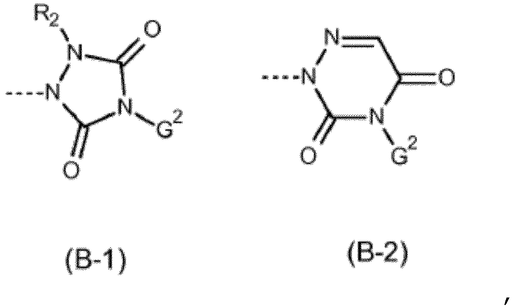 dans lesquels la ligne en pointillés signifie la liaison à l'atome de carbone du bicycle de formule (I), dans lesquels
R₂ représente hydrogène ou C₁-C₆-alkyle,
G² représente un radical de la série hydrogène, C₁-C₆-alkyle, halogéno-C₁-C₆-alkyle, C₃-C₆-cycloalkyle, halogéno-C₃-C₆-cycloalkyle, C₃-C₆-cycloalcényle et halogéno-C₃-C₆-cycloalcényle, et composés de formule (I), dans laquelle
A représente le radical A 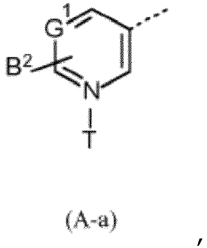 dans lequel la ligne en pointillés signifie la liaison à l'atome de carbone du bicycle de formule (I),
G¹ représente N ou C-B¹,
B¹ représente un radical de la série hydrogène, halogène, C₁-C₆-alkyle et C₁-C₄-halogénoalkyle,
B² représente un radical de la série hydrogène, halogène, C₁-C₆-alkyle et C₁-C₄-halogénoalkyle,
T représente oxygène ou une paire d'électrons,
X₁ représente N ou CH,
X₂ représente CH,
X₃ représente CH,
R₁ représente un radical B de la série 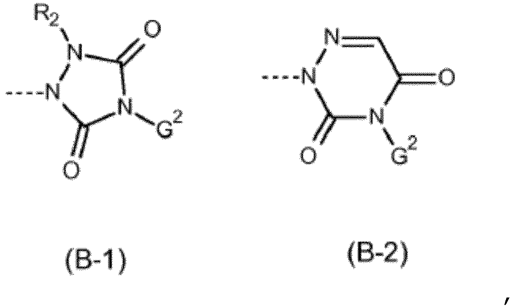 dans lesquels la ligne en pointillés signifie la liaison à l'atome de carbone du bicycle de formule (I), dans lesquels
R₂ représente hydrogène, C₁-C₆-alkyle ou halogéno-C₁-C₆-alkyle,
G² représente un radical de la série hydrogène, C₁-C₆-alkyle, halogéno-C₁-C₆-alkyle, C₁-C₆-alcoxy-C₁-C₆-alkyle, C₁-C₆-alkyl-S-C₁-C₆-alkyle, C₁-C₆-alkyl-S(O)-C₁-C₆-alkyle, C₁-C₆-alkyl-S(O₂)-C₁-C₆-alkyle, C₃-C₆-cycloalkyle, C₃-C₆-cycloalkyl-C₁-C₆-alkyle, phényle ou benzyle (phényle et benzyle eux-mêmes à leur tour pouvant être substitués par halogène, cyano, nitro, C₁-C₆-alkyle, halogéno-C₁-C₆-alkyle et C₃-C₆-cycloalkyle), pyridylméthyle, qui peut être substitué par halogène, halogéno-C₃-C₆-cycloalkyle, C₃-C₆-cycloalcényle et halogéno-C₃-C₆-cycloalcényle.

9. Composés de formule (I) selon la revendication 1, dans lesquels
A représente un des radicaux A suivants 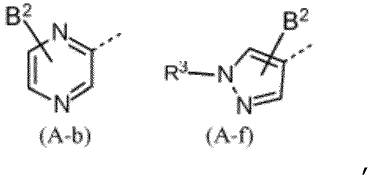 dans lesquels la ligne en pointillés signifie la liaison à l'atome de carbone du bicycle de formule (I),
B² représente hydrogène,
X₁ représente CH,
X₂ représente CH,
X₃ représente CH,
R₁ représente un radical B de la série 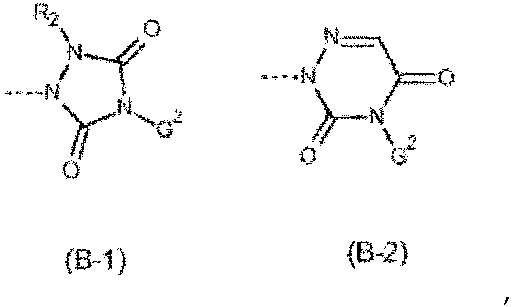 dans lesquels la ligne en pointillés signifie la liaison à l'atome de carbone du bicycle de formule (I), dans lesquels
R₂ représente hydrogène ou C₁-C₆-alkyle,
G² représente un radical de la série hydrogène, C₁-C₆-alkyle, halogéno-C₁-C₆-alkyle et C₃-C₆-cycloalkyle, et
composés de formule (I), dans lesquels
A représente le radical A 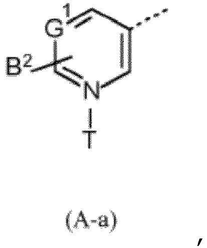 dans lequel la ligne en pointillés signifie la liaison à l'atome de carbone du bicycle de formule (I),
G¹ représente N ou C-B¹,
B¹ représente un radical de la série hydrogène et fluor,
B² représente hydrogène,
T représente oxygène ou une paire d'électrons,
X₁ représente N ou CH,
X₂ représente CH,
X₃ représente CH,
R₁ représente un radical B de la série 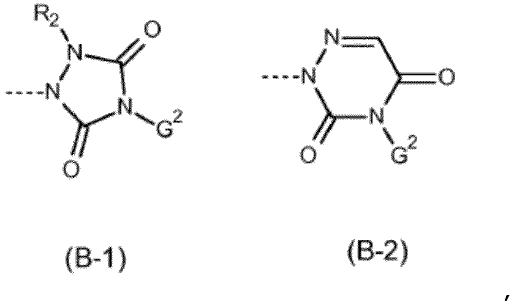 dans lesquels la ligne en pointillés signifie la liaison à l'atome de carbone du bicycle de formule (I), dans lesquels
R₂ représente hydrogène, C₁-C₆-alkyle ou halogéno-C₁-C₆-alkyle,
G² représente un radical de la série C₁-C₄-alkyle, halogéno-C₁-C₄-alkyle, C₁-C₄-alcoxy-C₁-C₄-alkyle, C₁-C₄-alkyl-S-C₁-C₄-alkyle, C₁-C₄-alkyl-S(O)-C₁-C₄-alkyle, C₁-C₄-alkyl-S(O₂)-C₁-C₄-alkyle, C₃-C₆-cycloalkyle, C₃-C₆-cycloalkyl-C₁-C₄-alkyle, phényle ou benzyle (phényle et benzyle eux-mêmes pouvant être à leur tour substitués par halogène et cyano), pyridylméthyle qui peut être substitué par halogène.

10. Composés de formule (I) selon la revendication 1, dans lesquels
A représente 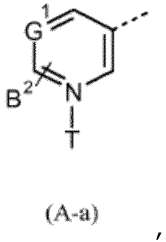 dans lequel la ligne en pointillés signifie la liaison à l'atome de carbone du bicycle de formule (I),
G¹ représente C-B¹,
B¹ représente hydrogène,
B² représente hydrogène,
T représente une paire d'électrons,
X₁ représente N ou CH,
X₂ représente CH,
X₃ représente CH,
R₁ représente le radical (B-1) 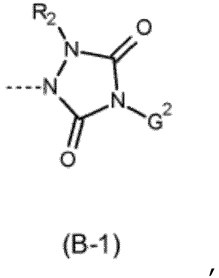 dans lequel la ligne en pointillés signifie la liaison à l'atome de carbone du bicycle de formule (I), dans lequel
R₂ représente hydrogène, C₁-C₄-alkyle ou halogéno-C₁-C₄-alkyle et
G² représente un radical de la série C₁-C₄-alkyle, halogéno-C₁-C₄-alkyle, C₁-C₄-alcoxy-C₁-C₄-alkyle, C₁-C₄-alkyl-S-C₁-C₄-alkyle, C₁-C₄-alkyl-S(O)-C₁-C₄-alkyle, C₃-C₆-cycloalkyle, C₃-C₆-cycloalkyl-C₁-C₄-alkyle, phényle, benzyle et pyridylméthyle (phényle, benzyle et pyridylméthyle pouvant être eux-mêmes à leur tour substitués par halogène).

11. Composés de formule (I) selon la revendication 1, dans lesquels
A représente le radical A 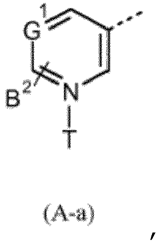 dans lequel la ligne en pointillés signifie la liaison à l'atome de carbone du bicycle de formule (I),
G¹ représente C-B¹,
B¹ représente hydrogène,
B² représente hydrogène,
T représente une paire d'électrons,
X₁ représente N ou CH,
X₂ représente CH,
X₃ représente CH,
R₁ représente le radical (B-2) 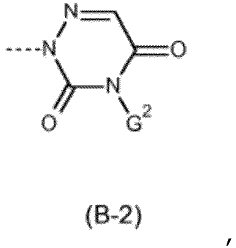 dans lequel la ligne en pointillés signifie la liaison à l'atome de carbone du bicycle de formule (I), dans lequel
G² représente un radical de la série C₁-C₄-alkyle, halogéno-C₁-C₄-alkyle, C₃-C₆-cycloalkyl-C₁-C₄-alkyle, phényle ou benzyle (phényle et benzyle pouvant eux-mêmes à leur tour être substitués par halogène).

12. Composé selon la revendication 1, **caractérisé en ce qu'**il est le composé de formule

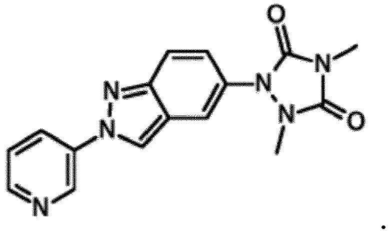

13. Composé selon la revendication 1, **caractérisé en ce qu'**il est le composé de formule

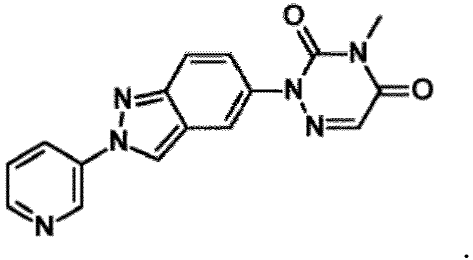

14. Produit, **caractérisé par** une teneur en au moins un composé de formule (I) selon l'une quelconque des revendications 1 à 13 et en diluants et/ou en substances tensioactives habituel(le)s.

15. Utilisation de composés de formule (I) selon l'une quelconque des revendications 1 à 13 ou de produits selon la revendication 14 pour la lutte contre des parasites.
